# EUROPEAN PATENT APPLICATION

(11) **EP 4 389 895 A1**
(43) Date of publication of application: **26.06.2024**
(21) Application number: 22895642.1
(22) Date of filing: 16.11.2022
(51) Int. Cl.: C12N 15/13, A01K 67/027, C07K 16/00, C07K 16/06, C12N 5/0735, C12N 5/10, C12N 15/06, C12N 15/09, C12N 15/10, C12N 15/85, C12N 15/90, C12P 21/08

(54) **MAMMALIAN ARTIFICIAL CHROMOSOMAL VECTOR HAVING HUMAN IMMUNOGLOBULIN HEAVY CHAIN LOCUS CONTAINING MODIFIED D REGION, AND CELL OR NON-HUMAN ANIMAL HOLDING SAID VECTOR**

(30) Priority: 16.11.2021 JP 2021186124
(71) Applicant: National University Corporation Tottori University, Tottori-shi, Tottori 680-8550 (JP)
(72) Inventor: KAZUKI Yasuhiro, Yonago-shi, Tottori 683-8503 (JP); TOMIZUKA Kazuma, Hachioji-shi, Tokyo 192-0392 (JP); UNO Narumi, Hachioji-shi, Tokyo 192-0392 (JP)
(74) Representative: D Young & Co LLP
(86) International application number: PCT/JP2022/042560
(87) International publication number: WO 2023/090361

(57) **Abstract**

Provided is an approach for achieving the expansion of a human antibody repertoire in a human antibody-producing non-human animal. Provided is a mammalian artificial chromosome vector comprising a human immunoglobulin heavy chain locus in which a D-region of a human immunoglobulin heavy chain is modified, and a human immunoglobulin light chain locus, a mammalian cell or a non-human animal comprising this mammalian artificial chromosome vector, and a method for substituting a D-region of a human immunoglobulin heavy chain with a modified sequence thereof in the production of this artificial chromosome vector.

## Description

### Technical Field

The present invention relates to a mammalian artificial chromosome vector comprising a human immunoglobulin heavy chain locus in which a D-region of a human immunoglobulin heavy chain is modified, and a human immunoglobulin light chain locus.

The present invention also relates to a mammalian cell or a non-human animal comprising the mammalian artificial chromosome vector.

The present invention further relates to a method for modifying the D-region in the production of the mammalian artificial chromosome vector.

### Background Art

A human antibody is not recognized as foreign substance in the human body and as such, is used as a therapeutic agent for tumor, autoimmune disease, or the like. Such an antibody may be produced by using a phage display technique or a human antibody-producing mouse. The human antibody-producing mouse is a trans-chromosomic (TC) mouse that retains a mouse artificial chromosome vector comprising full-length of human immunoglobulin heavy chain and light chain loci (Patent Literature 1, Patent Literature 2, and Non Patent Literature 1).

For example, when the human antibody is used for passive immunity against infectious disease caused by a virus such as human immunodeficiency virus (HIV), novel coronavirus (COVID-19), or influenza virus, a neutralizing antibody is well known to often lack efficacy for a reason such as the masking of a target antigen of the virus or rapid occurrence of a mutation of a target antigen of the virus. Control measures for infectious disease require a lot of time and cost because it is necessary to produce a new antibody capable of defending against the mutation. Furthermore, infectious disease is considered difficult to promptly suppress because it is necessary to take measures against the masking of a target antigen of the pathogenic virus, such as the development of an approach for activating the immune system or a therapeutic agent such as an antiviral agent.

Under these circumstances, the utility value of the human antibody-producing mouse is limited for a target antigen for which an antibody is unlikely to be induced in a human. Hence, there is a demand for the development of a technique that facilitates obtaining an antibody against the target antigen by expanding a human antibody repertoire. In addition, the construction of a platform for achieving a further expanded human antibody repertoire has been proposed (Non Patent Literature 3).

### Citation List

### Patent Literature

Patent Literature 1: JP Patent No. 4318736
Patent Literature 2: JP Patent No. 6868250

### Non Patent Literature

Non Patent Literature 1: K. Tomizuka et al., Proc. Natl. Acad. Sci. USA, 2000; 97(2): 922-927
Non Patent Literature 2: M. A. Alfaleh et al., Front. Immunol. 2020; 11: Article 1986, https://doi.org/10.3389/fimmu.2020.01986
Non Patent Literature 3: A. R. Rees, MABS 2020, 12(1), e1729683

### Summary of Invention

### Technical Problem

An object of the present invention is to provide an approach for achieving the expansion of a human antibody repertoire in a human antibody-producing non-human animal.

In this connection, Y. Di et al. (Immunology August 2021; 00: 1-14, doi:10.1111/imm.13407) have reported that a B-DH mouse in which a DH gene region on the mouse genome was recombinated with a bovine DH gene region was produced by using genome editing, then immunized with an antigen (OVA, BSA, EWL, and KLH), and examined for the production of an antibody comprising ultralong CDRH3, only to find that ultralong CDRH3 was merely observed at a low frequency.

### Solution to Problem

In order to achieve the object, the present inventors have paid attention to the finding that a bovine efficiently produces a broadly neutralizing antibody against HIV (M.J. Burke et al., Viruses 2020, 12, 473; doi:10.3390/v12040473), and conceived of an approach for expanding a human antibody repertoire and/or an approach by which a human antibody having antibody heavy chain CDR3 longer than usual appears.

Specifically, the present invention encompasses the following characteristics:
[1] A mammalian artificial chromosome vector comprising human immunoglobulin heavy chain and light chain loci, wherein a human-derived genomic sequence from D1-1 to D1-26 of a D-region of the human immunoglobulin heavy chain locus is substituted with a modified sequence of the D-region having a combination of the following (1) and (2) or (1) and (3), wherein the modified sequence of the D-region comprises:
   (1) sequences in which inter-open reading frame (ORF) human-derived genomic sequences from D1-1 to D1-26 of the D-region of the human immunoglobulin heavy chain locus or the inter-ORF human-derived genomic sequences except for the one between D3-9 and D3-10 are truncated to the length of 49 bp or more from the end of each VDJ recombination sequence of an inter-ORF region flanked by VDJ recombination sequences; and
   (2) ORF sequences from B1-1 to B9-4 of a D-region of a bovine-derived immunoglobulin heavy chain locus, ORF sequences from 1S5 to 1S35 of a D-region of a monkey-derived immunoglobulin heavy chain locus, or ORF sequences of a D-region of a sheep-, horse-, rabbit-, bird-, or shark-derived immunoglobulin heavy chain locus, instead of ORF sequences from D1-1 to D1-26 of the D-region of the human immunoglobulin heavy chain locus; or
   (3) 26 types of modified ORF sequences from D1-1 to D1-26 of the D-region of the human immunoglobulin heavy chain locus produced on the basis of a human antibody heavy chain CDR3 sequence of 18 or more amino acids, instead of ORF sequences from D1-1 to D1-26 of the D-region of the human immunoglobulin heavy chain locus.
[2] The mammalian artificial chromosome vector according to [1], wherein the inter-ORF human-derived genomic sequences of the D-region of the human animal immunoglobulin heavy chain locus in (1) comprise sequences truncated to the length of 100 to 500 bp.
[3] The mammalian artificial chromosome vector according to [1] or [2], wherein the ORF sequences from B1-1 to B9-4 of a D-region of a bovine-derived immunoglobulin heavy chain locus comprise the nucleotide sequences of SEQ ID NOs: 127 to 149 or nucleotide sequences having 90% or higher identity to the nucleotide sequences.
[4] The mammalian artificial chromosome vector according to [1] or [2], wherein the ORF sequences from 1S5 to 1S35 of a D-region of a monkey-derived immunoglobulin heavy chain locus comprise the nucleotide sequences of SEQ ID NOs: 150 to 175 or nucleotide sequences having 90% or higher identity to the nucleotide sequences.
[5] The mammalian artificial chromosome vector according to [1] or [2], wherein the 26 types of modified ORF sequences in (3) comprise the nucleotide sequences of SEQ ID NOs: 75 to 100 or nucleotide sequences having 90% or higher identity to the nucleotide sequences.
[6] The mammalian artificial chromosome vector according to any of [1] to [5], wherein the modified sequence of the D-region comprises the nucleotide sequence of SEQ ID NO: 2 or SEQ ID NO: 3 or a nucleotide sequence having 90% or higher identity to the nucleotide sequence.
[7] The mammalian artificial chromosome vector according to any of [1] to [6], wherein the D-region of the human immunoglobulin heavy chain locus further lacks D7-27.
[8] The mammalian artificial chromosome vector according to any of [1] to [7], wherein the vector is a rodent artificial chromosome vector.
[9] The mammalian artificial chromosome vector according to [8], wherein the rodent artificial chromosome vector is a mouse artificial chromosome vector.
[10] The mammalian artificial chromosome vector according to any of [1] to [9], wherein the light chain locus is a κ light chain locus and/or a λ light chain locus.
[11] A mammalian cell comprising a mammalian artificial chromosome vector according to any of [1] to [10].
[12] The mammalian cell according to [11], wherein the cell is a rodent cell or a human cell.
[13] The mammalian cell according to [11], wherein the cell is a pluripotent stem cell selected from the group consisting of a mammalian-derived iPS cell and ES cell.
[14] A non-human mammal comprising a mammalian artificial chromosome vector according to any of [1] to [10], wherein endogenous immunoglobulin heavy chain, κ light chain, and λ light chain genes or loci are disrupted.
[15] A non-human animal comprising human immunoglobulin heavy chain and light chain loci, wherein a human-derived genomic sequence from D1-1 to D1-26 of a D-region of the human immunoglobulin heavy chain locus is substituted with a modified sequence of the D-region having a combination of the following (1) and (2) or (1) and (3), wherein the modified sequence of the D-region comprises:
   (1) sequences in which inter-open reading frame (ORF) human-derived genomic sequences from D1-1 to D1-26 of the D-region of the human immunoglobulin heavy chain locus or the inter-ORF human-derived genomic sequences except for the one between D3-9 and D3-10 are truncated to the length of 49 bp or more from the end of each VDJ recombination sequence of an inter-ORF region flanked by VDJ recombination sequences; and
   (2) ORF sequences from B1-1 to B9-4 of a D-region of a bovine-derived immunoglobulin heavy chain locus, ORF sequences from 1S5 to 1S35 of a D-region of a monkey-derived immunoglobulin heavy chain locus, or ORF sequences of a D-region of a sheep-, horse-, rabbit-, bird-, or shark-derived immunoglobulin heavy chain locus, instead of ORF sequences from D1-1 to D1-26 of the D-region of the human immunoglobulin heavy chain locus; or
   (3) 26 types of modified ORF sequences from D1-1 to D1-26 of the D-region of the human immunoglobulin heavy chain locus produced on the basis of a human antibody heavy chain CDR3 sequence of 18 or more amino acids, instead of ORF sequences from D1-1 to D1-26 of the D-region of the human immunoglobulin heavy chain locus; and
   wherein endogenous immunoglobulin heavy chain, κ light chain, and λ light chain genes or loci are disrupted.
[16] The non-human animal according to [14] or [15], wherein the animal is a rodent.
[17] The non-human animal according to [16], wherein the rodent is a mouse or a rat.
[18] A method for producing an antibody, comprising immunizing a non-human animal according to any of [14] to [17] with a target antigen, and obtaining an antibody binding to the target antigen from blood of the non-human animal.
[19] A method for producing an antibody, comprising immunizing a non-human animal according to any of [14] to [17] with a target antigen, obtaining splenocytes, lymph node cells, or B cells from the non-human animal producing an antibody binding to the target antigen, fusing the splenocytes, the lymph node cells, or the B cells with myeloma cells to form hybridomas, and culturing the hybridomas to obtain a monoclonal antibody binding to the target antigen.
[20] A method for producing an antibody, comprising immunizing a non-human animal according to any of [14] to [17] with a target antigen, obtaining B cells from the non-human animal producing an antibody binding to the target antigen, obtaining nucleic acids encoding proteins composed of an antibody heavy chain and light chain or their variable regions from the B cells, and producing an antibody binding to the target antigen by a DNA recombination technique or a phage display technique using the nucleic acid.
[21] A method for substituting a D-region of a human immunoglobulin heavy chain with a modified sequence thereof in the production of a mammalian artificial chromosome vector according to any of [1] to [10], comprising:
   (1') providing a mammalian artificial chromosome vector comprising human immunoglobulin heavy chain and light chain loci;
   (2') deleting the D-region of the human immunoglobulin heavy chain from the mammalian artificial chromosome vector of the step (1');
   (3') inserting a cassette comprising a first site-directed recombinase recognition site, a promoter, and a second site-directed recombinase recognition site into a deletion site of the D-region in the vector obtained in the step (2');
   (4') inserting a cassette comprising the modified sequence of the D-region of the human immunoglobulin heavy chain, the first site-directed recombinase recognition site, a selection marker, and the second site-directed recombinase recognition site into the second site-directed recombinase recognition site of the cassette inserted in the step (3') using the second site-directed recombinase; and
   (5') obtaining the artificial chromosome vector in which the cassette comprising the first site-directed recombinase recognition site, the modified sequence of the D-region of the human immunoglobulin heavy chain, and the second site-directed recombinase recognition site is inserted into the deletion site of the D-region by site-directed recombination using the first site-directed recombinase.
[22] The method according to [21], wherein the D-region of the human immunoglobulin heavy chain is deleted by genome editing.
[23] A mammalian artificial chromosome vector comprising human immunoglobulin heavy chain and light chain loci, wherein a D-region is deleted from the human immunoglobulin heavy chain locus (the human immunoglobulin heavy chain locus lacks a D-region) and the human immunoglobulin heavy chain locus comprises a first site-directed recombinase recognition site, a promoter, and a second site-directed recombinase recognition site instead of the deleted D-region.
[24] The mammalian artificial chromosome vector according to [23], wherein the deleted D-region is a region from D1-1 to D1-26.
[25] The mammalian artificial chromosome vector according to [23], wherein the deleted D-region is a region from D1-1 to D7-27.

The description of the present application encompasses the contents described in Japanese Patent Application No. 2021-186124, on which the present application claims priority is based.

### Advantageous Effects of Invention

The present invention enables to expand an antibody repertoire in a human antibody-producing non-human animal (e.g., rodent) by substituting ORF (gene) sequences of a human-derived genomic sequence of a D-region of a human immunoglobulin heavy chain locus or a human minimalized (i.e., comprising the truncation of inter-open reading frame (ORF) regions) D-region sequence thereof with, for example, ORF sequences of a D-region derived from an animal such as a bovine or a monkey, or modified ORF sequences of the human D-region sequence, as well as that a human antibody comprising CDRH3 having a length of preferably 15 to 50 or more amino acids to appear.

### Brief Description of Drawings

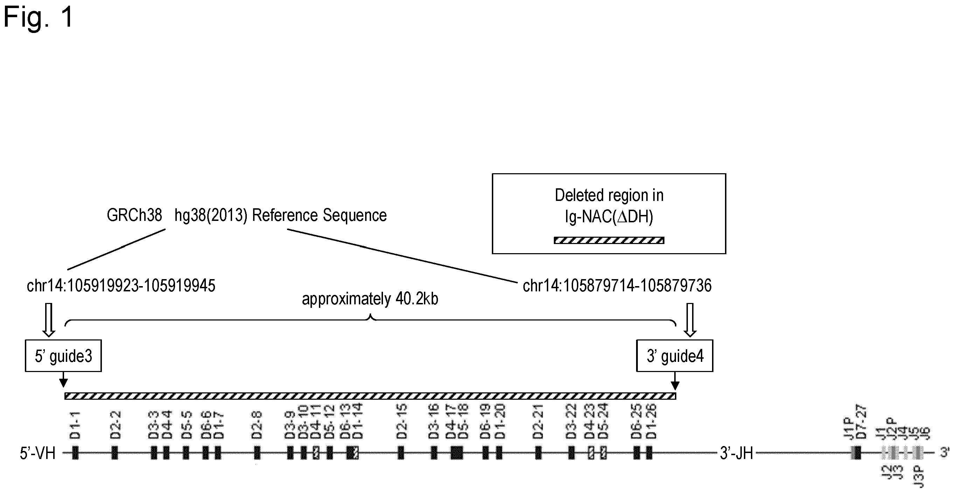

[Figure 1] This figure shows that a D-region sequence (region comprising D1-1 to D1-26) of a human immunoglobulin heavy chain locus is deleted by the combination of human gRNA Up3 and Down4. The human immunoglobulin heavy chain locus from which the heavy chain D-region has been deleted is referred to as Ig-NAC(ΔDH). This figure relates to Example 1.

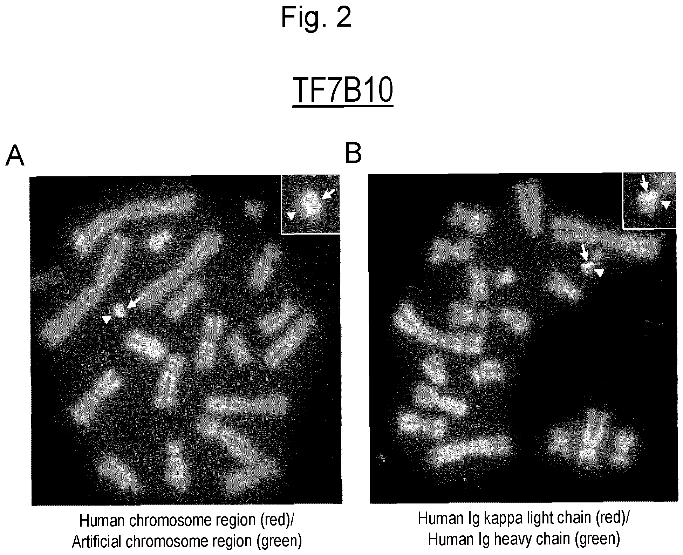

[Figure 2] Figure 2A shows a CHO cell (cell clone TF7-B10) retaining one copy of Ig-NAC(ΔDH) independently of host mouse chromosomes. In the Ig-NAC(ΔDH), the arrowhead depicts a human chromosome region (red), and the arrow depicts an artificial chromosome region (green). Figure 2B shows that the Ig-NAC(ΔDH) comprises a human immunoglobulin (Ig) heavy chain (arrow or green) and κ light chain (arrowhead or red). This figure shows results of Example 1.

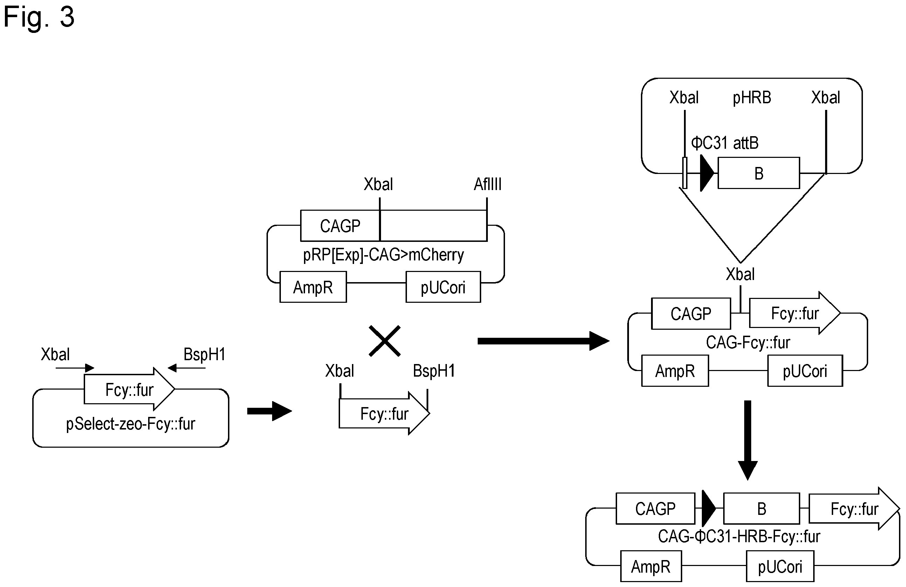

[Figure 3] This figure shows that Fcy::fur gene was inserted into a restriction enzyme AfiIII (NEB) and XbaI (NEB) cleavage site in a plasmid pRP[Exp]-CAG>mCherry (synthesized at VectorBuilder Inc.) having CAGP (CAG promoter; a conjugate of cytomegalovirus enhancer and chicken β-actin promoter) to obtain a plasmid CAG-Fcy::fur, then this plasmid was cleaved with a restriction enzyme XbaI, and a plasmid HRB-derived fragment ("B") cleaved at both ends with XbaI was inserted in between the CAGP and the Fcy::fur gene to obtain a plasmid CAG-ΦC31-HRB-Fcy::fur. This figure relates to Example 2.

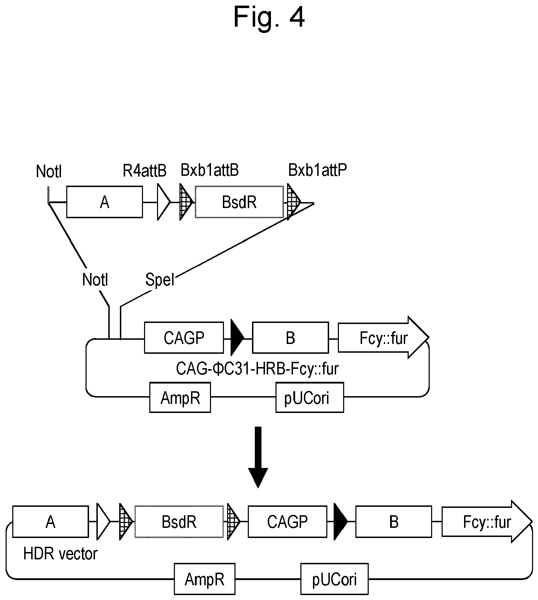

[Figure 4] This figure shows that the plasmid CAG-ΦC31-HRB-Fcy::fur was cleaved with restriction enzymes NotI (NEB) and SpeI (NEB), and a plasmid HRA-derived fragment ("A") was inserted upstream side of the CAGP to obtain an HDR vector. In the figure, BsdR represents blasticidin resistance gene, CAGP represents CAG promoter, R4 attB, Bxb1 attB, and Bxb1 attP each represent a recombinase recognition site, AmpR represents ampicillin resistance gene, and pUCori represents a replication origin of pUC, respectively. This figure relates to Example 2.

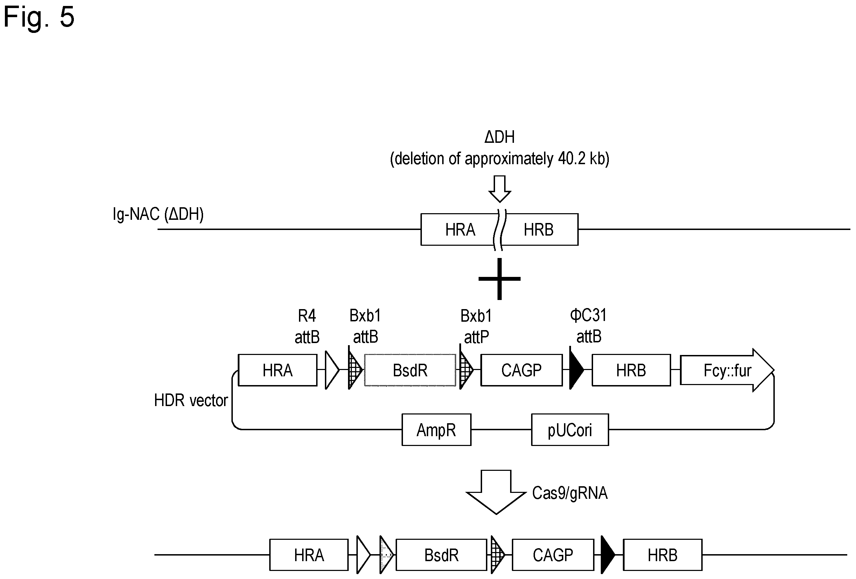

[Figure 5] This figure shows that the HDR vector was inserted near a deletion site of the DH region (ΔDH) in the Ig-NAC(ΔDH) in CHO cells by genome editing (Cas9/gRNA). This figure relates to Example 2.

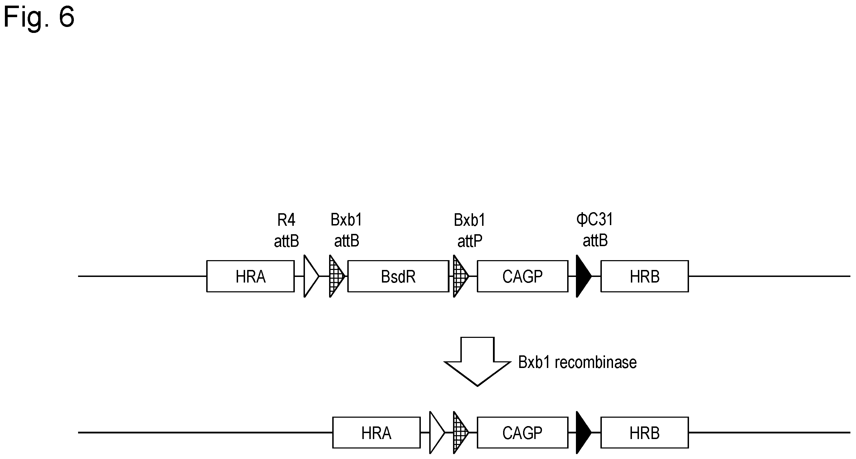

[Figure 6] This figure shows that the blasticidin resistance gene is removed from the HDR vector through site-directed recombination reaction by introducing Bxb1 recombinase to cells. This figure relates to Example 3.

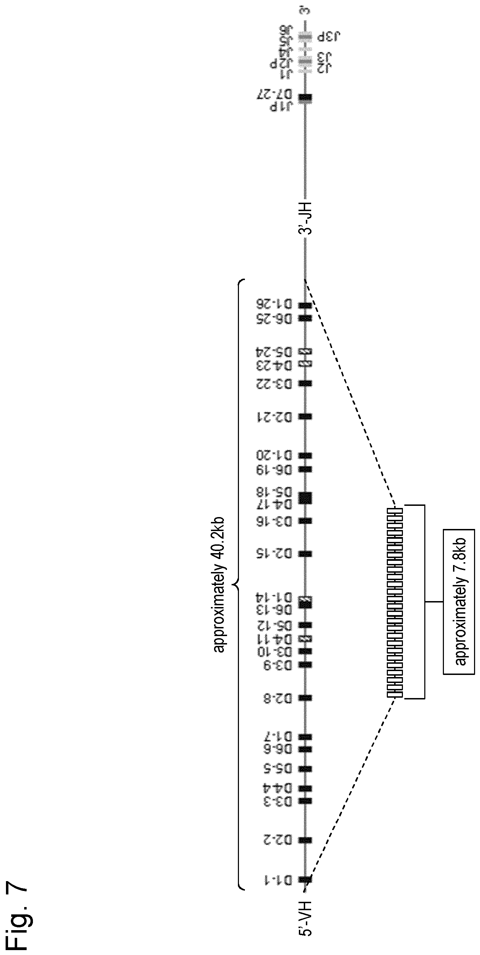

[Figure 7] This figure shows that each of inter-ORF genomic sequences from D1-1 to D1-26 of a D-region of a human immunoglobulin heavy chain gene is truncated to the size of approximately 200 bp to minimalize D-region. This figure relates to Example 6.

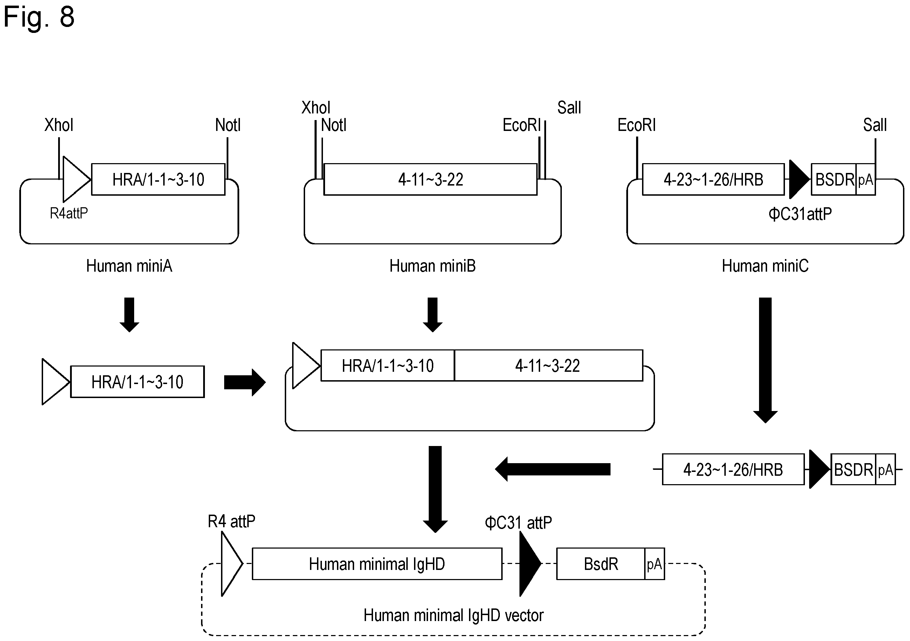

[Figure 8] This figure shows procedures of producing a human minimalized IgHD vector from human miniA, human miniB, and human miniC. In the figure, the human miniA bears R4 attP and from downstream of HRA to a D-fragment 3-10, the human miniB bears from D-fragments 3-10 to 3-22, and the human miniC bears from a D-fragment 3-22 to HRB, ΦC31 attP, and blasticidin resistance gene (BSDR), respectively. This figure relates to Example 6.

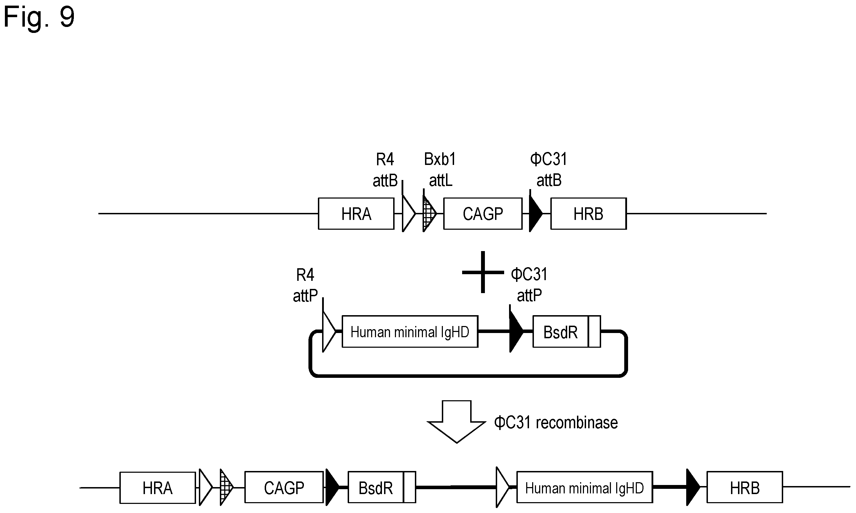

[Figure 9] This figure shows the production of an HDR vector in which human minimalized IgHD is inserted by site-directed recombination between ΦC31 attB of the HDR vector from which the blasticidin resistance gene has been removed as shown in Figure 6 and ΦC31 attP on the human minimalized IgHD vector. This figure relates to Example 6.

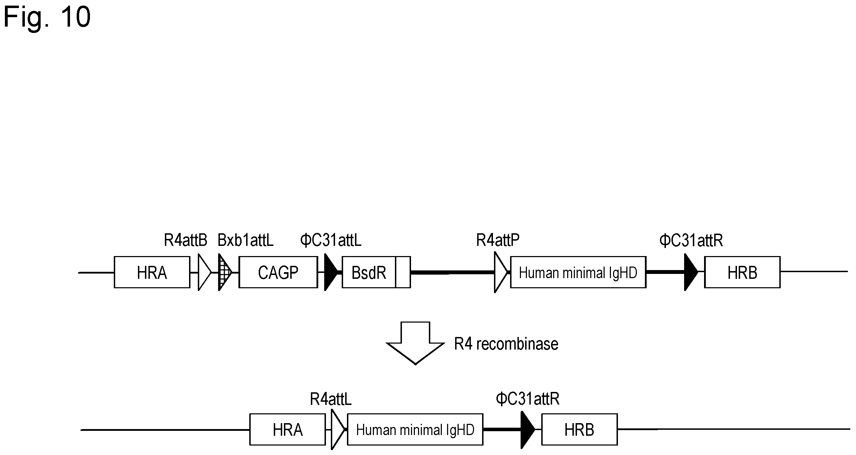

[Figure 10] This figure shows that unnecessary sequences such as the CAG promoter and the blasticidin resistance gene are removed from the produced HDR vector having the human minimalized IgHD insert in Figure 9 by introducing R4 recombinase to cells. This figure relates to Example 6.

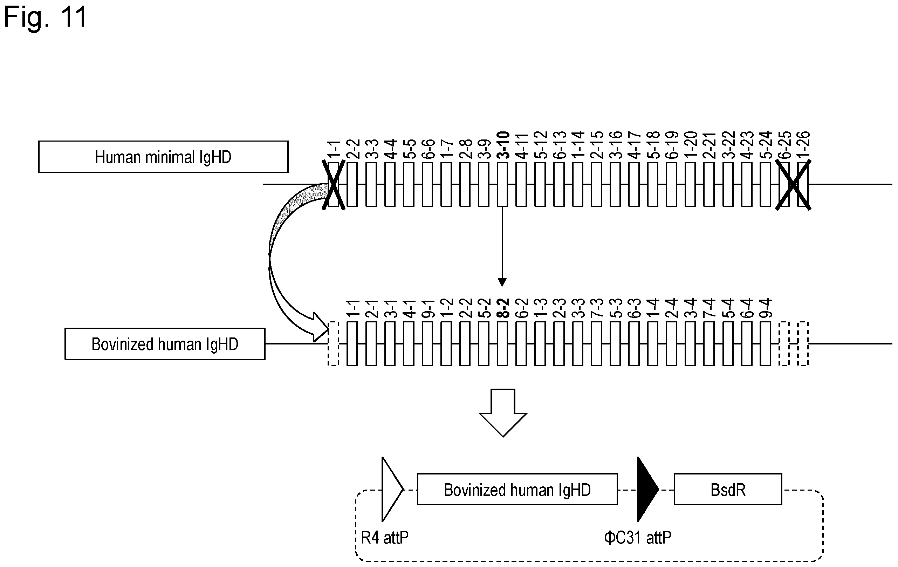

[Figure 11] This figure shows the production of a vector in which the human minimalized IgHD (in the figure, a total of 23 fragments from D2-2 to D5-24) in the vector comprising the human minimalized D-region of the immunoglobulin heavy chain locus (Figure 8) is substituted with bovinized human IgHD (in the figure, a total of 23 fragments from B1-1 to B9-4). In this context, unreplaced 3 human D-fragments (D1-1, D6-25, and D1-26) were deleted together with 100 bp upstream and downstream thereof because the number of bovine D-fragments (23) is fewer than that of human ones (26). This figure relates to Example 9.

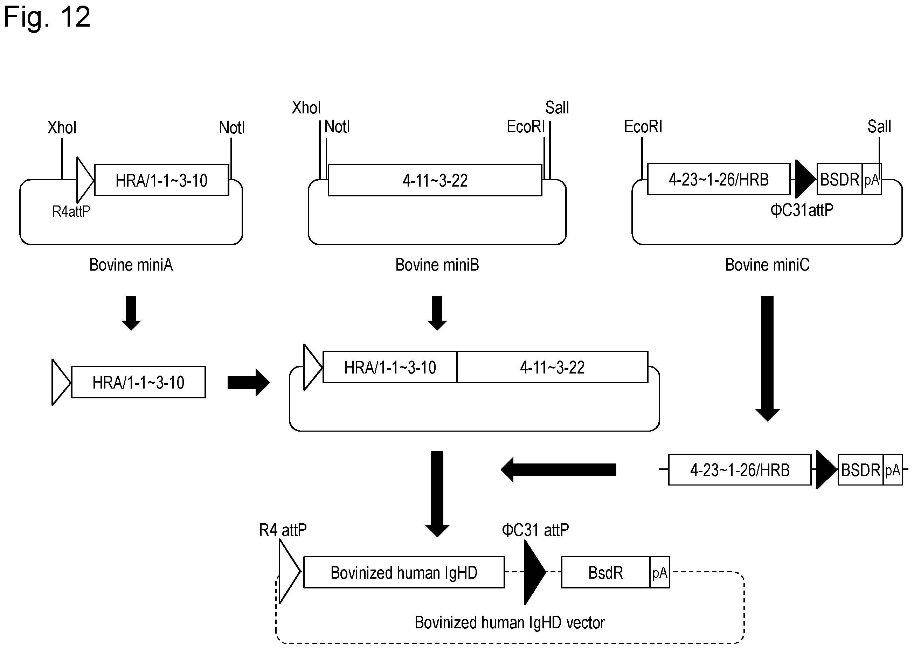

[Figure 12] This figure shows procedures of producing a bovinized human IgHD vector retaining bovine miniA, miniB, and miniC. In the figure, bovinized human IgHD was divided into 3 segments in accordance with Example 5, and these 3 segments of the plasmid DNA, bovine miniA, bovine miniB, and bovine miniC, were chemically synthesized (the synthesis was requested to Eurofins Genomics K.K.). This figure relates to Example 9.

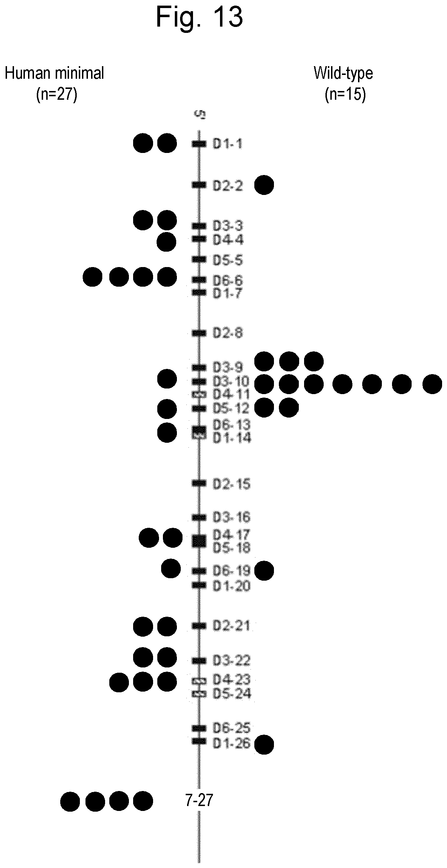

[Figure 13] This figure shows the frequency of use of a D-region in Ig µ-cDNA in splenocytes of a chimeric mouse retaining a mammalian artificial chromosome vector comprising a human immunoglobulin (Ig) heavy chain locus in which a D-region of a human heavy chain is substituted with a human minimalized D-region. The filled circle depicts the D-region used. The figure shows n = 27 (total number of filled circles) for the human minimalized type and on the other hand, n = 15 (total number of filled circles) for the wild type. This figure shows results of Example 14.

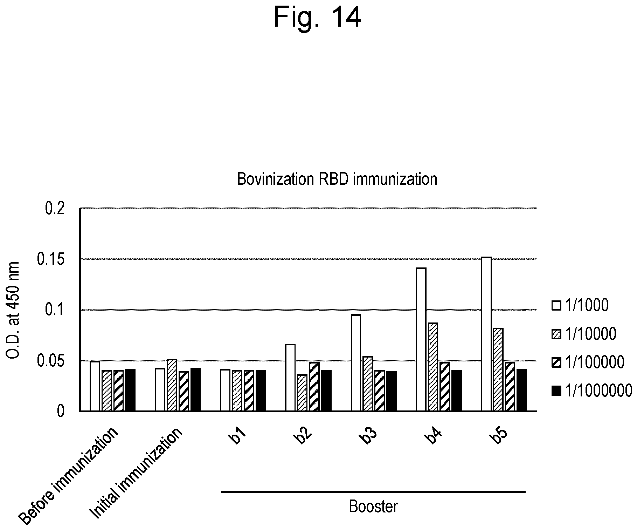

[Figure 14] This figure shows results of performing ELISA as to antiserum diluted at a ratio of 1/1,000 to 1/1,000,000 as shown using an antigen (receptor binding domain of SARS-CoV2 spike protein S1; RBD)-immobilized 96-well plate and an anti-human IgG Fc antibody in order to evaluate the titer of antigen-specific IgG in plasma in a TC mouse retaining modified Ig-NAC comprising a human immunoglobulin heavy chain locus modified with bovinized human IgHD. It was confirmed here that immune response occurred by continuous booster so that an antibody titer (ordinate; O.D. at 450 nm) against RBD was elevated. In the figure, b1 to b5 represent boosters 1 to 5. The figure shows results of Example 18.

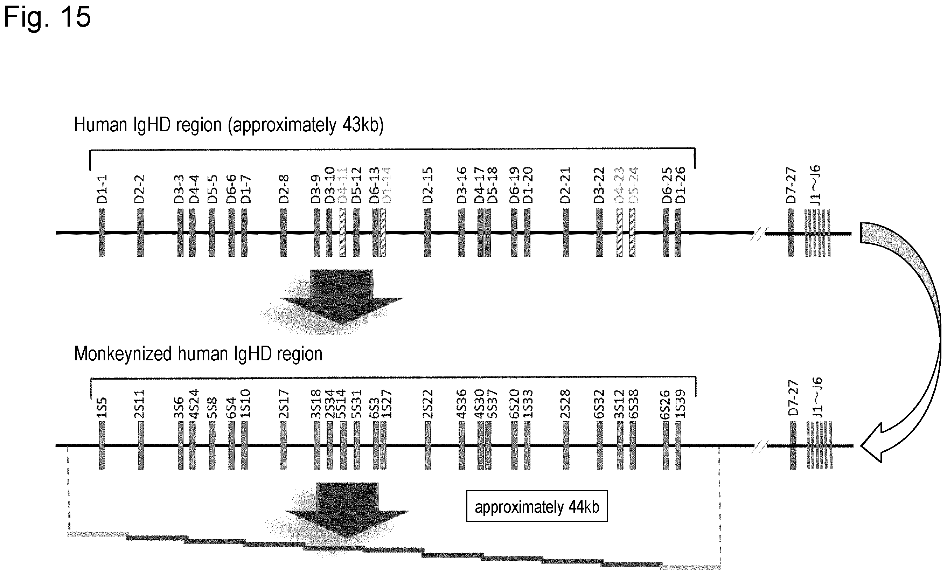

[Figure 15] This figure shows the structure of a monkeynized human IgHD region obtained by substituting a human minimalized IgHD region (approximately 43 kb) with cynomolgus macaque D-fragments (approximately 44 kb). This figure relates to Example 19.

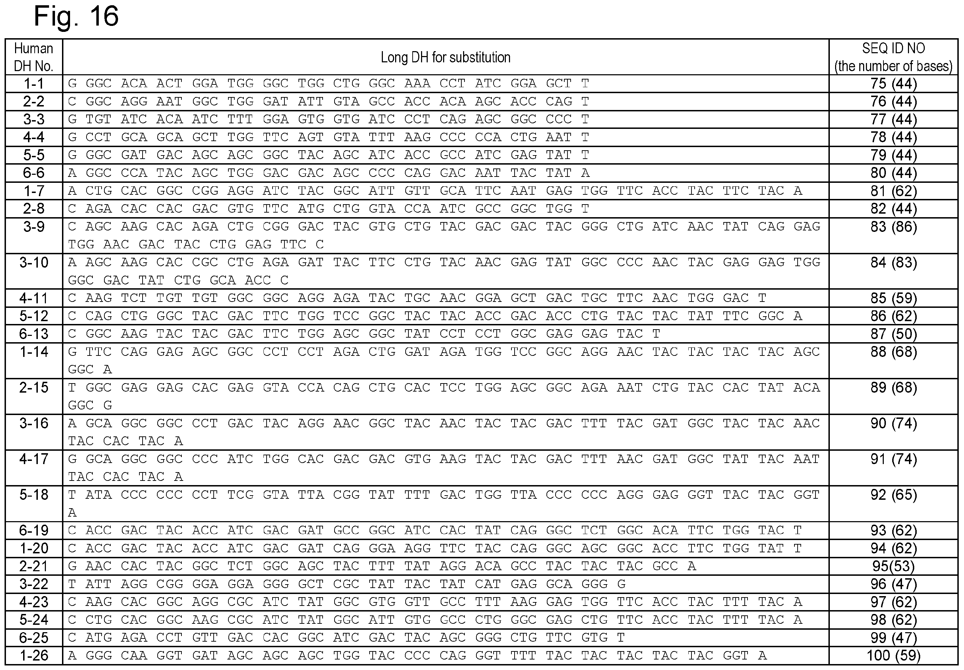

[Figure 16] This figure shows 26 modified long DH sequences for substituting D-fragments 1-1 to 1-26 of a human long minimalized IgHD region. This figure relates to Example 29.

### Description of Embodiments

The present invention will be described in more detail.

### 1. Mammalian artificial chromosome vector

In the first aspect, the present invention provides a mammalian artificial chromosome vector comprising human immunoglobulin heavy chain locus and light chain locus, wherein a human-derived genomic sequence from D1-1 to D1-26 of a D-region of the human immunoglobulin heavy chain locus is substituted with a modified sequence of the D-region having a combination of the following (1) and (2) or (1) and (3), wherein the modified sequence of the D-region comprises:
(1) sequences in which inter-ORF human-derived genomic sequences from D1-1 to D1-26 of the D-region of the human immunoglobulin heavy chain locus or the inter-ORF human-derived genomic sequences except for the one between D3-9 and D3-10 are truncated to the length of 49 bp or more from the end of each VDJ recombination sequence of an inter-ORF region flanked by VDJ recombination sequences; and
(2) ORF sequences from B1-1 to B9-4 of a D-region of a bovine-derived immunoglobulin heavy chain locus, ORF sequences from 1S5 to 1S35 of a D-region of a monkey-derived immunoglobulin heavy chain locus, or ORF sequences of a D-region of a sheep-, horse-, rabbit-, bird-, or shark-derived immunoglobulin heavy chain locus, instead of ORF sequences from D1-1 to D1-26 of the D-region of the human immunoglobulin heavy chain locus; or
(3) 26 types of modified ORF sequences from D1-1 to D1-26 of the D-region of the human immunoglobulin heavy chain locus produced on the basis of a human antibody heavy chain CDR3 sequence of 18 or more amino acids, instead of ORF sequences from D1-1 to D1-26 of the D-region of the human immunoglobulin heavy chain locus.

The term "D-region of a human immunoglobulin heavy chain locus" used herein refers to the whole region comprising all ORFs ("genes" (or regions encoding polypeptides) or also referred to as "D-fragments"), VDJ recombination sequences, and all inter-ORF (i.e., between the genes) regions.

The term "VDJ recombination sequences" used herein are sequences adjacent to upstream and downstream of V-, D-, and J-regions of human immunoglobulin heavy chain locus and light chain locus, and comprise preserved sequences of 7, 23, 9, and/or 12 bases located in a different manner depending on heavy chain, light chain κ, and light chain λ loci. For the D-region of the human immunoglobulin heavy chain locus, the recombination sequence comprises bases of 28 bp (i.e., 9 bp, 12 bp (spacer), and 7 bp).

The mammalian artificial chromosome vector is, without limitation, an artificial chromosome vector produced from a chromosome of a mammal such as a human or a rodent (e.g., a mouse, a rat, a hamster, and a guinea pig) and is more specifically an artificially produced chromosome vector that comprises the centromere and the telomere derived from the animal and comprises a human immunoglobulin heavy chain locus comprising a modified D-region of an antibody heavy chain, and optionally a human immunoglobulin light chain locus in long arm- and short arm (if present)-derived genomic sequences from which, for example, 99.5% to 100%, preferably 100%, of genes have been removed.

The term "telomere" used herein is natural telomere derived from the allogeneic or xenogeneic or artificial telomere. The allogeneic means an animal of the same species as that of a mammal from which a chromosome fragment of an artificial chromosome vector is derived. The xenogeneic means a mammal other than the animal (which includes a human). Also, the sequence of the artificial telomere refers to an artificially produced sequence having a telomere function, such as a (TTAGGG)n sequence (where "n" indicates the number of repetitions). The telomere sequence may be introduced into an artificial chromosome by a method involving telomere truncation (cleavage by artificial insertion of the telomere sequence), as disclosed in, for example, WO 00/10383. The telomere truncation may be used to truncate a chromosome in the production of the artificial chromosome of the present invention.

### [Modification of D-region of human immunoglobulin heavy chain locus]

Hereinafter, the modified sequence having a combination of (1) and (2) or (3) described above will be described.

The human immunoglobulin (also simply referred to as "human antibody") heavy chain locus comprised in the artificial chromosome vector of the present invention has modified only in genomic sequence of a D-region among a V-region, the D-region, a J-region, and a C-region. In this context, the modified human-derived genomic sequence of the D-region includes modified ORF (or gene) sequences of the human D-region or truncated (or "minimalized") inter-ORF regions. Further, ORF sequences in the human-derived genomic sequence of the human D-region or the minimalized sequence of the human D-region are substituted with ORF sequences of a D-region derived from another animal species or 26 types of modified ORF sequences from D1-1 to D1-26 of the D-region of the human immunoglobulin heavy chain locus produced on the basis of a human antibody heavy chain CDR3 sequence of 18 or more amino acids.

In general, a human antibody heavy chain variable region consists of framework (FR)1, CDRH1, FR2, CDRH2, FR3, CDRH3, FR4, and a part of constant region in order from the N terminus. FR1, CDRH1, FR2, CDRH2, and FR3 are encoded mainly by the nucleotide sequence of a V-region in a VDJ sequence formed by the recombination (or rearrangement) of the human immunoglobulin heavy chain locus. CDRH3 and FR4 are encoded mainly by the nucleotide sequence of a D-region and the nucleotide sequence of a J-region, respectively, in the VDJ sequence. In the present invention, the D-region is modified in order to produce a human antibody comprising antibody heavy chain CDR3 having a length of 18 or more amino acids.

On the other hand, a human antibody light chain variable region consists of FR1, CDRL1, FR2, CDRL2, FR3, CDRL3, FR4, and a part of constant region. FR1, CDRL1, FR2, CDRL2, FR3, and CDRL3 (partial) are encoded mainly by the nucleotide sequence of a V-region in a VJ sequence formed by the recombination (or rearrangement) of the human immunoglobulin light chain locus. CDRH3 (partial) and FR4 are encoded mainly by the nucleotide sequence of a J-region in the VJ sequence.

It is generally known that an antibody region to which a target antigen specifically binds is a CDRH3 domain of an antibody heavy chain hypervariable region. Therefore, in the present invention, the genomic sequence of the D-region of the human antibody heavy chain locus is modified so as to be able to expand a human antibody repertoire and preferably to obtain an antibody comprising CDRH3 having a larger amino acid length (e.g., 18 or more amino acids) than usual in a human antibody.

Hereinafter, the modified sequence of the human D-region will be described.

Examples of the modified sequence include a modified genomic sequence of the D-region comprising sequences in which genomic sequences of inter-ORF (or "inter-gene") regions in the D-region of the human immunoglobulin heavy chain locus are truncated to 49 bp or more from the end of each VDJ recombination sequence of an inter-ORF region flanked by VDJ recombination sequences, for example, truncated to the length of approximately 100 to approximately 500 bp, approximately 150 bp to approximately 300 bp, or approximately 200 bp to approximately 250 bp.

The D-region of the human immunoglobulin heavy chain locus (also referred to as an "IgHD" region) resides on human chromosome 14 (14q32.33) and comprises a nucleotide sequence of more than approximately 40 kb involving, for example, D1-1 (positioned at 23599-35048; accession No. X97051), D2-2 (positioned at 35049-37615; accession No. J00232), D3-3 (positioned at 37616-39425; accession No. X13972), D4-4 (positioned at 39426-40474; accession No. X13972), D5-5 (positioned at 40475-41880; accession No. X13972), D6-6 (positioned at 41881-43056; accession No. X13972), D1-7 (positioned at 43057-44649; accession No. X13972), D2-8 (positioned at 44650-47262; accession No. X13972), D3-9 (positioned at 47263-48619; accession No. X13972), D3-10 (positioned at 48620-49158; accession No. X13972), D4-11 (positioned at 49159-50078; accession No. X13972), D5-12 (positioned at 50079-51316; accession No. X13972), D6-13 (positioned at 51317-52324; accession No. X13972), D1-14 (positioned at 52325-53909; accession No. X13972), D2-15 (positioned at 53910-56408; accession No. J00234), D3-16 (positioned at 5640-58143; accession No. X97051), D4-17 (positioned at 58144-59187; accession No. X97051), D5-18 (positioned at 59188-60591; accession No. X97051), D6-19 (positioned at 60592-61769; accession No. X97051), IGHD1-20 (positioned at 61770; accession No. X97051), D2-21 (positioned at 61770-65916; accession No. X97051), D3-22 (positioned at 65917-67762; accession No. X93616), D4-23 (positioned at 67763-68826; accession No. X97051), D5-24 (positioned at 68827-70492; accession No. X97051), D6-25 (positioned at 70493-71926; accession No. X97051), and D1-26 (positioned at 719267-79765; accession No. X97051) in the order of 5' -> 3', and an additional sequence (D7-27) (M. Ruiz et al., Exp. Clin. Immunogenet. 16, 173-184 (1999)). Alternatively, the sequence of a human natural IgHD region is also disclosed in NG_001019.6 (nucleotide numbers (positions): 960181 to 1000622 (40442 bp)).

The ORF sequences from D1-1 to D1-26 of the D-region of the human immunoglobulin heavy chain (IgH) are, for example, the nucleotide sequences of SEQ ID NOs: 101 to 126 shown below. Examples of inter-ORF sizes after truncation are also shown between the sequences.
SEQ ID NO: 101 (IGHD1-1):
   ggtacaactggaacgac
   (inter-ORF size of D1-1 and D2-2 + VDJ recombination sequences (28 bp × 2): 256 bp)
SEQ ID NO: 102 (IGHD2-2):
   aggatattgtagtagtaccagctgctatgcc
   (inter-ORF size of D2-2 and D3-3 + VDJ recombination sequences (28 bp × 2): 256 bp)
SEQ ID NO: 103 (IGHD3-3):
   gtattacgatttttggagtggttattatacc
   (inter-ORF size of D3-3 and D4-4 + VDJ recombination sequences (28 bp × 2): 256 bp)
SEQ ID NO: 104 (IGHD4-4):
   tgactacagtaactac
   (inter-ORF size of D4-4 and D5-5 + VDJ recombination sequences (28 bp × 2): 256 bp)
SEQ ID NO: 105 (IGHD5-5):
   gtggatacagctatggttac
   (inter-ORF size of D5-5 and D6-6 + VDJ recombination sequences (28 bp × 2): 256 bp)
SEQ ID NO: 106 (IGHD6-6):
   gagtatagcagctcgtcc
   (inter-ORF size of D6-6 and D1-7 + VDJ recombination sequences (28 bp × 2): 256 bp)
SEQ ID NO: 107 (IGHD1-7):
   ggtataactggaactac
   (inter-ORF size of D1-7 and D2-8 + VDJ recombination sequences (28 bp × 2): 256 bp)
SEQ ID NO: 108 (IGHD2-8):
   aggatattgtactaatggtgtatgctatacc
   (inter-ORF size of D2-8 and D3-9 + VDJ recombination sequences (28 bp × 2): 255 bp)
SEQ ID NO: 109 (IGHD3-9):
   gtattacgatattttgactggttattataac
   (inter-ORF size of D3-9 and D3-10 + VDJ recombination sequences (28 bp × 2): 153 bp)
SEQ ID NO: 110 (IGHD3-10):
   gtattactatggttcggggagttattataac
   (inter-ORF size of D3-10 and D4-11 + VDJ recombination sequences (28 bp × 2): 316 bp)
SEQ ID NO: 111 (IGHD4-11):
   tgactacagtaactac
   (inter-ORF size of D4-11 and D5-12 + VDJ recombination sequences (28 bp × 2): 256 bp)
SEQ ID NO: 112 (IGHD5-12):
   gtggatatagtggctacgattac
   (inter-ORF size of D5-12 and D6-13 + VDJ recombination sequences (28 bp × 2): 256 bp)
SEQ ID NO: 113 (IGHD6-13):
   gggtatagcagcagctggtac
   (inter-ORF size of D6-13 and D1-14 + VDJ recombination sequences (28 bp × 2): 259 bp)
SEQ ID NO: 114 (IGHD1-14):
   ggtata accggaaccac
   (inter-ORF size of D1-14 and D2-15 + VDJ recombination sequences (28 bp × 2): 256 bp)
SEQ ID NO: 115 (IGHD2-15):
   aggatattgtagtggtggtagctgctactcc
   (inter-ORF size of D2-15 and D3-16 + VDJ recombination sequences (28 bp × 2): 256 bp)
SEQ ID NO: 116 (IGHD3-16):
   gtattatgattacgtttgggggagttatgcttatacc
   (inter-ORF size of D3-16 and D4-17 + VDJ recombination sequences (28 bp × 2): 256 bp)
SEQ ID NO: 117 (IGHD4-17):
   tgactacggtgactac
   (inter-ORF size of D4-17 and D5-18 + VDJ recombination sequences (28 bp × 2): 256 bp)
SEQ ID NO: 118 (IGHD5-18):
   gtggatacagctatggttac
   (inter-ORF size of D5-18 and D6-19 + VDJ recombination sequences (28 bp × 2): 256 bp)
SEQ ID NO: 119 (IGHD6-19):
   gggtatagcagtggctggtac
   (inter-ORF size of D6-19 and D1-20 + VDJ recombination sequences (28 bp × 2): 256 bp)
SEQ ID NO: 120 (IGHD1-20):
   ggtataactggaacgac
   (inter-ORF size of D1-20 and D2-21 + VDJ recombination sequences (28 bp × 2): 256 bp)
SEQ ID NO: 121 (IGHD2-21):
   agcatattgtggtggtgattgctattcc
   (inter-ORF size of D2-21 and D3-22 + VDJ recombination sequences (28 bp × 2): 256 bp)
SEQ ID NO: 122 (IGHD3-22):
   gtattactatgatagtagtggttattactac
   (inter-ORF size of D3-22 and D4-23 + VDJ recombination sequences (28 bp × 2): 316 bp)
SEQ ID NO: 123 (IGHD4-23):
   tgactacggtggtaactcc
   (inter-ORF size of D4-23 and D5-24 + VDJ recombination sequences (28 bp × 2): 256 bp)
SEQ ID NO: 124 (IGHD5-24):
   gtagagatggctacaattac
   (inter-ORF size of D5-24 and D6-25 + VDJ recombination sequences (28 bp × 2): 256 bp)
SEQ ID NO: 125 (IGHD6-25):
   gggtatagcagcggctac
   (inter-ORF size of D6-25 and D1-26 + VDJ recombination sequences (28 bp × 2): 256 bp)
SEQ ID NO: 126 (IGHD1-26):
   ggtatagtgggagctactac

The modified sequence comprises modified ORFs of the human IgHD region and comprises truncated (or minimalized) human-derived genomic sequences of inter-ORF regions (i.e., inter-gene regions) so as to each have a length of 49 bp or more, for example, approximately 100 bp to approximately 500 bp, approximately 150 bp to approximately 300 bp, or approximately 200 bp to approximately 250 bp from the end of each VDJ recombination sequence of an inter-ORF region flanked by VDJ recombination sequences. The length of the modified human IgHD region after minimalization is preferably approximately 10 kb or smaller such that this region is insertable into a (usual) plasmid vector. For example, truncation to approximately 200 bp results in a minimalized length of approximately 8 kb.

A method for the truncation (or minimalization) described above is not particularly limited. For example, the sequence of each inter-ORF region may be deleted equally from the central part thereof such that 49 bp or more remains from the end of a VDJ recombination sequence adjacent to each ORF in the inter-ORF region. The minimalization to obtain the human minimalized D-region of interest may be performed, for example, by dividing D1-1 to D1-26 of the human D-region into several (e.g., 3 to 5) segments, preparing their respective minimalized segments, and linking the obtained minimalized segments in order from the 5' side (Figure 8). The nucleotide sequence of a D-region comprising ORF sequences, VDJ recombination sequences (28 bp × 2), and minimalized inter-ORF sequences from D1-1 to D1-26 of the human minimalized D-region has, for example, the nucleotide sequence of SEQ ID NO: 1 shown below; a nucleotide sequence having 90% or higher, 95% or higher, 97% or higher, 98% or higher, or 99% or higher identity to the sequence; or a nucleotide sequence which comprises the deletion, substitution, or addition of one or several nucleotides (or bases) in said nucleotide sequence.

### SEQ ID NO: 1:

### [Chem. 1]

1 gtcgcctcta cgggaaatcc gggactcagc agtaaccctc aggaagcagg gcccaggcgc
61 cgtttaatag gaggcttcct cacaatgaaa cttttagaaa gccttgacta caatgatgac
121 cttggtgtgg ctgtgaacac tgtcagctcc cacagctgct gcagcaaaaa atgtccatag
181 acagggtggg ggcccggggt cgtctgctgt cctgctcagc ccacagcacg catggaggat
241 ctgaggtgcc acacctgacg cccaggccag aacatgcctc cctccagggt gacctgccat
301 gtcctgcatt gctggaggga caggggcagc ctatgaggat ctggggccag gagatgaatc
361 ctattaaccc agaggaaaac taacaggacc caagcaccct ccccgttgaa gctgacctgc
421 ccagaggggc ctgggcccac cccacacacc ggggcggaat gtgtacaggc cccggtctct
481 gtgggtgttc cgctaactgg ggctcccagt gctcacccca caactaaagc gagccccagc
541 ctccagagcc cccgaaggag atgccgccca caagcccagc ccccatccag gaggccccag
601 agctcagggc gccggggcag attctgaaca gccccgagtc acggtgggta caactggaac
661 gaccaccgtg agaaaaactg tgtccaaaac tctctcctgg cccctgctgg aggccgcgcc
721 agagagggga gcagccgccc cgaacctagg tcctgctcag ctcacacgac ccccagcagc
781 cagagcacag tccctgagtt tcagacaaaa accccctgga aatcatagta tcagcaggag
841 aactagccag agacagcaag aggggactca gtgactcccg cggggacagg aggattttgt
901 gggggctcgt gtcactgtga ggatattgta gtagtaccag ctgctatgcc cacagtgaca
961 cagccccatt cccaaagccc tgctgtaaac gcttccactt ctggagctga ggggctgggg
1021 ggagcgtctg ggaagtaggg cctaggggtg gccatcaatg cccaaaacgc accagactca
1081 gtagacctga gagcgctggg cccacagcct cccctcggtg ccctgctacc tcctcaggtc
1141 agccctggac atcccgggtt tccccaggcc tggcggtagg tttggggtga ggtctgtgtc
1201 actgtggtat tacgattttt ggagtggtta ttatacccac agtgtcacag agtccatcaa
1261 aaacccatcc ctgggaacct tctgccacag ccctccctgt ggggcaccgc tgcgtgccat
1321 gttaggattt tgactgagga cacagcacca tgggtatggt ggctagaaag gtctgagatc
1381 cccaggacgc agcaccactg tcaatggggg ccccagacgc ctggaccagg gcctgcgtgg
1441 gaaaggcctc tgggcacact caggggcttt ttgtgaaggg tcctcctact gtgtgactac
1501 agtaactacc acagtgatga acccagcagc aaaaactgac cggactccca aggtttatgc
1561 acacttctcc gctcagagct ctccaggatc agaagagccg ggcccaaggg tttctgccca
1621 gaccctcggc ctctaggccc catgtcccca gtcctggggg cccctggcac agctgtctgg
1681 accctctcta ttccctggga agctcctcct gacagccccg cctccagttc caggtgtggt
1741 tattgtcagg gggtgtcaga ctgtggtgga tacagctatg gttaccacag tggtgctgcc
1801 catagcagca accaggccaa gtagacaggc ccctgctgtg cagccccagg cctccagctc
1861 acctgcttct cctggggctc tcaaggctgc tgttttctgc actctcccct ctgggcaggc
1921 cccccagaac cagggatgag gacgccccgt caaggccaga aaagaccaag ttgtgctgag
1981 cccagcaagg gaaggtcccc aaacaaacca ggaagtttct gaaggtgtct gtgtcacagt
2041 ggagtatagc agctcgtccc acagtgacac tcgccaggcc agaaacccca tcccaagtca
2101 gcggaatgca gagagagcag ggaggacatg tttaggatct gaggccgcac ctgacaccca
2161 ggccagcaga cgtctcctgt ccatggccca cacctaaaac gagccacagc ctcagagccc
2221 ctgaaggaga ccccgcccac aagcccagcc cccacccagg aggccccaga gcacagggcg
2281 ccccgtcgga ttctgaacag ccccgagtca cagtgggtat aactggaact accactgtga
2341 gaaaagcttc gtccaaaacg gtctcctggc cacagtcgga ggccccgcca gagaggggag

2401 cagccacccc aaacccatgt tctgccggct cccatgaccc cgtgcacctg gagccccaca
2461 cccagagttt cagacaaaaa ccccctggaa tgcacagtct cagcaggaga gccagccaga
2521 gccagcaaga tggggctcag tgacacccgc agggacagga ggattttgtg ggggctcgtg
2581 tcactgtgag gatattgtac taatggtgta tgctataccc acagtgacac agccccattc
2641 ccaaagccct actgcaaacg cattccactt ctggggctga ggggctgggg gagcgtctgg
2701 gaaatagggc tcaggggtgt ccatcaatgc ccaaaacgca ccagaccagc agacctgaga
2761 gcgctgggcc cacagcctcc cctcagagcc ctgctgcctc ctccggtcag ccctggacat
2821 cccaggtttc cccaggcctg ccggtaggtt tagaatgagg tctgtgtcac tgtggtatta
2881 cgatattttg actggttatt ataaccacag tgtcacagag tccatcaaaa acccatgcct
2941 ggaagcttcc cgccacagcc ctccccatgg ggccctgctg cctcctcagg tcagccccgg
3001 acatcccggg tttccccagg ctgggcggta ggtttggggt gaggtctgtg tcactgtggt
3061 attactatgg ttcggggagt tattataacc acagtgtcac agagtccatc aaaaacccat
3121 ccctgggagc ctcccgccac agccctccct gcaggggacc ggtacgtgcc atgttaggat
3181 tttgatcgag gagacagcac catgggtatg gtggctaatg catgcggccg ccctgtgacc
3241 caaacccgca gggcagcagg cacgatggac aggcccgcac agcggcctga gatccccagg
3301 acgcagcacc gctgtcaata ggggccccaa atgcctggac cagggcctgc gtgggaaagg
3361 tctctggcca cactcgggct tttgtgaagg ggccctcctg ctgtgtgact acagtaacta
3421 ccatagtgat gaacccagtg gcaaaaactg gctggaaacc caggggctgt gtgcacgcct
3481 cagcttggag ctctccagga gcacaagagc cgggcccaag gatttgtgcc cagaccctca
3541 gcctctaggc cccacgtccc cagtcctggg ggcccctggc tcagctgtct ggaccctccc
3601 tgttccctgg gaagctcctc ctgacagccc cgcctccagt tccaggtgtg gttattgtca
3661 ggcgatgtca gactgtggtg gatatagtgg ctacgattac cacagtggtg ccgcccatag
3721 cagcaaccag gccaagtaga caggcccctg ctgcgcagcc ccaggcatcc acttcacctg
3781 cttctcctgg ggctctcaag gctgctgtct gtcctctggc cctctgtggc aggcccccca
3841 gaaccagggt tgagggacgc cccgtcaaag ccagacaaaa ccaaggggtg ttgagcccag
3901 caagggaagg cccccaaaca gaccaggagg tttctgaagg tgtctgtgtc acagtggggt
3961 atagcagcag ctggtaccac agtgacactc acccagccag aaaccccatt ccaagtcagc
4021 ggaagcagag agagcaggga ggacacgttt aggatctgag actgcacctg acacccaggc
4081 cagcagacgt ctcccctcca gggcacccca cacctaaaat gagccacagc ctccggagcc
4141 cccgcaggag accccgccca caagcccagc ccccacccag gaggccccag agctcagggc
4201 gccccgtcgg attccgaaca gccccgagtc acagcgggta taaccggaac caccactgtc
4261 agaatagcta cgtcaaaaac tgtccagtgg ccactgccgg aggccccgcc agagagggca
4321 gcagccactc tgatcccatg tcctgccggc tcccatgacc cccagcacgc ggagccccac
4381 accctgagtt tcagacaaca acccgctgga atgcacagtc tcagcaggag aacagaccaa
4441 agccagcaaa agggacctcg gtgacaccag tagggacagg aggattttgt gggggctcgt
4501 gtcactgtga ggatattgta gtggtggtag ctgctactcc cacagtgaca cagacccatt
4561 cccaaagccc tactgcaaac acacccactc ctggggctga ggggctgggg gagcgtctgg
4621 gaagtagggt ccaggggtgt ctatcaatgt ccaaaatgca ccagactgca gtagacctga
4681 gagcgctggg cccacagcct cccctcggtg ccctgctgcc tcctcaggtc agccctggac
4741 atcccgggtt tccccaggcc agatggtagg tttgaagtga ggtctgtgtc actgtggtat
4801 tatgattacg tttgggggag ttatcgttat acccacagca tcacacggtc catcagaaac
4861 ccatgccaca gccctccccg caggggaccg ccgcgtgcca tgttacgatt ttgatcgagg
4921 acacagcgcc atgggtatgg tggctaccac agcagtgcag cgaaaggcct gagatcccca
4981 ggacgcagca ccactgtcaa tgggggcccc agacgcctgg accagggcct gcgtgggaaa

5041 ggccgctggg cacactcagg ggctttttgt gaaggcccct cctactgtgt gactacggtg
5101 actaccacag tgatgaaact agcagcaaaa actggccgga cacccaggga ccatgcacac
5161 ttctcagctt ggagctctcc aggaccagaa gagtcaggtc tgagggtttg tagccagacc
5221 ctcggcctct aggccccacg tccccagtcc tgggggcccc tggcacagct gtctggaccc
5281 tccctcttcc ctgggaagct cctcctgaca gccccgcctc cagttccagg tgtggttatt
5341 gtcagggggt gtcagactgt ggtggataca gctatggtta ccacagtggt gctgcccata
5401 gcagcaacca ggccaagtag acaggcccct gctgtgcagc cccaggcctc cagctcacct
5461 gcttctcctg gggctctcaa ggtcactgtt gtctgtactc tgccctctgg gcaggccccc
5521 caaaaccagg gttgagggat gatccgtcaa ggcagacaag accaaggggc actgacccca
5581 gcaagggaag gctcccaaac agacgaggag gtttctgaag ctgtctgtat cacagtgggg
5641 tatagcagtg gctggtacca cagtgacact cgccaggcca gaaaccccgt cccaagtcag
5701 cggaagcaga gagagcaggg aggacacgtt taggatctga ggccgcacct gacacccagg
5761 gcagcagacg tctcccctcc agggcataaa gcgagcccca gcctccagag ccccctaagc
5821 attccccgcc cagcagccca gcccctgccc ccacccagga ggccccagag ctcagggcgc
5881 ctggtcggat tctgaacagc cccgagtcac agtgggtata actggaacga ccaccgtgag
5941 aaaaactgtg tccaaaactg actcctggca gcagtcggag gccccgccag agaggggagc
6001 agccggcctg aacccatgtc ctgccggttc ccatgacccc cagcacccag agccccacgt
6061 tcagacaaca acccgctgga atgcacagtc tcagcaggag agccaggcca gagccagcaa
6121 gaggagactc ggtgacacca gtctcctgta gggacaggag gattttgtgg gggttcgtgt
6181 cactgtgagc atattgtggt ggtgactgct attcccacag tgacacaacc ccattcctaa
6241 agccctactg caaacgcacc cactcctggg actgaggggc tgggggagca tctgggaagt
6301 atggcctagg ggtgtccatc aatgcccaaa atgcaccaga ctccagtaga cctgagagcg
6361 ctgggcccac agcctcccct cggggccctg ctgcctcctc aggtcagccc tggacatccc
6421 gggtttcccc aggcctggcg gtaggtttga agtgaggtct gtgtcactgt ggtattacta
6481 tgatagtagt ggttattact accacagtgt cacagagtcc atcaaaaact catgcctggg
6541 agcctcccac cacagccctc cctgcggggg accgctgcat gccgtgttag gattttgatc
6601 gaggacacgg cgccatgggt atggtggcta gaattcacgc gtagggtgag tcagacccac
6661 ctgccctcaa tggcaggcgg ggaagattca gaaaggcctg agatccccag gacgcagcac
6721 cactgtcaat gggggcccca gacgcctgga ccagggcctg tgtgggaaag gcctctggcc
6781 acactcaggg gctttttgtg aagggccctc ctgctgtgtg actacggtgg taactcccac
6841 agtgatgaaa ccagcagcaa aaactgaccg gactcgcagg gtttatgcac acttctcggc
6901 tcggagctct ccaggagcac aagagccagg cccgagggtt tgtgcccaga ccctcggcct
6961 ctaggcccag gctctctagc actgggggcc cctggcacag ctgtctggac cctccctgtt
7021 ccctgggaag ctcctcctga cagccccgcc tccagttcca ggtgtggtta ttgtcagggg
7081 gtgccaggcc gtggtagaga tggctacaat taccacagtg gtgccgccca tagcagcaac
7141 caggccaagt agacagaccc ctgccacgca gccccaggcc tccagctcac ctgcttctcc
7201 tggggctctc aaggctgctg tctgccctct ggccctctgt gggcccccca gaaccagtgt
7261 tgagggacac ccctgtccag ggcagccaag tccaagaggc gcgctgagcc cagcaaggga
7321 aggcccccaa acaaaccagg aggtttctga agctgtctgt gtcacagtcg ggtatagcag
7381 cggctaccac aatgacactg ggcaggacag aaaccccatc ccaagtcagc cgaaggcaga
7441 gagagcaggc aggacacatt taggatctga ggccacacct gacactcaag ccaacagatg
7501 tctcccctcc agggccacac ctaaggtgag ccacagccgc cagagcctcc acaggagacc
7561 ccacgcagca gcccagcccc tacccaggag gccccagagc tcagggcgcc tgggtggatt
7621 ctgaacagcc ccgagtcacg gtgggtatag tgggagctac taccactgtg agaaaagcta

7681 tgtccaaaac tgtctcccgg ccactgctgg aggcccagcc agagaaggga ccagccgccc
7741 gaacatacga ccttcccagc cctcatgacc cccagcactt ggagctccac agtgtcccca
7801 ttggatggtg aggacggggg ccggggccat ctgcacctcc caacatcacc cccaggcagc
7861 acaggcacaa accccaaatc cagagccgac accaggaaca cagacacccc aataccctgg
7921 gggaccctgg ccctggtgac ttcccactgg gatccacccc cgtgtccacc tggatcaaag
7981 accccaccgc tgtctctgtc cctcactcag ggcctgctga ggggcgggtg ctttggagca
8041 gactcaggtt taggggccac cattgtgggg cccaacctcg accaggacac agatttttct
8101 ttcctgccct ggggcaacac agactttggg gtctgtgcag ggaggacctt ctggaaagtc
8161 accaagcaca gagccctgac tgaggtggtc tcaggaagac cccccaggag gggtttgtgc
8221 cccttcctct catgtggacc ccatgccccc caagataggg gcatcatgca gggcaggtcc
8281 tccatgcagc caccactagg caactccctg gcgccggtcc ccactgcgcc tccatcccgg
8341 ctctggggat gcagccacca tggccacacc aggcagcccg ggtccagcaa ccctgcagtg
8401 cccaagccct tggcaggatt cccagaggct ggagcccacc cctcctcatc cccccacacc
8461 tgcacacaca cacctacccc ctgcccagtc cccctccagg agggttggag ccgcccatag
8521 ggtgggcgct ccaggtctca ctcactcgct tcccttcctg ggcaaaggag cctcgtgccc
8581 cggtcccccc tgacggcgct gggcacaggt gtgggtactg ggccccaggg ctcctccagc
8641 cccagctgcc ctgctctccc tgggaggcct gggcaccacc agaccaccag tccagggcac
8701 agccccaggg agccgcccac tgccagctca caggaagaag ataagcttca gaccctcagg
8761 gccgggagct gccttcctgc caccccttcc tgccccagac ctccatgccc tcccccaacc
8821 acttacacac aagccaggga gctgtttcca cacagttcaa ccccaaacca ggacggcctg
8881 gcactcgggt cactgccatt tctgtctgca ttcgctccca gcgcccctgt gttccctccc
8941 tcctccctcc ttcctttctt cctgcattgg gttc

The term "several" used herein refers to an integer of 2 to 10.

The sequence of the human minimalized D-region such as SEQ ID NO: 1 may be used to substitute ORFs of the human IgHD region with xenogeneic ORFs.

With the minimalization of the human D-region according to the present invention, for example, the frequency of use of human minimalized D-fragments in chimeric mouse spleen Ig µ-cDNA is elevated to n = 27 (n = 15 for wild type) (Figure 13). This indicates that the truncation of inter-ORF regions in the D-region of the human immunoglobulin heavy chain has no influence on an antibody repertoire.

Another modified sequence comprises a genomic sequence of a D-region of an immunoglobulin heavy chain locus derived from a non-human animal capable of producing an antibody whose antibody heavy chain CDR3 has an amino acid length of 18 or more, or a modified sequence thereof.

Such a non-human animal is, for example, an animal such as a bovine, a monkey (e.g., a cynomolgus macaque), sheep, a horse, a rabbit, a bird, or a shark (e.g., a bull shark, a sandbar shark, and a nurse shark). At least these animal species can be confirmed as species having longer D-fragments than the longest human D-fragment (37 bp) by retrieval from, for example, the international ImMunoGeneTics information system (IMGT). Hereinafter, a bovine and a monkey will be specifically described, but as for other animal species, the ORF sequences from D1-1 to D1-26 of the D-region of the human immunoglobulin heavy chain locus may also be substituted in order to comprise ORF sequences of a D-region of an immunoglobulin heavy chain locus derived from any of the animal species by the same manner.

The D-region of the bovine immunoglobulin heavy chain locus (21q24) comprises, for example, a nucleotide sequence involving B1-1, B2-1, B3-1, B4-1, B9-1, B1-2, B2-2, B5-2, B8-2, B6-2, B1-3, B2-3, B3-3, B7-3, B5-3, B6-3, B1-4, B2-4, B3-4, B7-4, B5-4, B6-4, and B9-4 of IGHD in the order of 5' -> 3'.

The ORF sequences from D1-1 to D1-26 of the D-region of the human immunoglobulin heavy chain locus may be substituted in order to comprise ORF sequences from B1-1 to B9-4 of the D-region of the bovine-derived immunoglobulin heavy chain locus. The nucleotide sequence of the bovinized human minimalized D-region thus obtained has, for example, the nucleotide sequence of SEQ ID NO: 2 shown below, a nucleotide sequence having 90% or higher, 95% or higher, 97% or higher, 98% or higher, or 99% or higher identity to the sequence, or a nucleotide sequence comprising the deletion, substitution, or addition of one or several nucleotides (or bases) in said nucleotide sequence.

### SEQ ID NO: 2:

### [Chem. 2]

1 gtcgcctcta cgggaaatcc gggactcagc agtaaccctc aggaagcagg gcccaggcgc
61 cgtttaatag gaggcttcct cacaatgaaa cttttagaaa gccttgacta caatgatgac
121 cttggtgtgg ctgtgaacac tgtcagctcc cacagctgct gcagcaaaaa atgtccatag
181 acagggtggg ggcccggggt cgtctgctgt cctgctcagc ccacagcacg catggaggat
241 ctgaggtgcc acacctgacg cccaggccag aacatgcctc cctccagggt gacctgccat
301 gtcctgcatt gctggaggga caggggcagc ctatgaggat ctggggccag gagatgaatc
361 ctattaaccc agaggaaaac taacaggacc caagcaccct ccccgttgaa gctgacctgc
421 ccagaggggc ctgggcccac cccacacacc ggggcggaat gtgtacaggc cccggtctct
481 gtgggtgttc cgctaactgg ggctcccagt gctcacccca caactaaagc gagccccagc
541 ctccagagcc cccgaaggag atgccgccca caagcccagc ccccatccag gaggccccag
601 agctcaggcc ctgagtttca gacaaaaacc ccctggaaat catagtatca gcaggagaac
661 tagccagaga cagcaagagg ggactcagtg actcccgcgg ggacaggagg atttgtgggg
721 ggctcgtgtc actgtgagaa taccgtgatg atggttactg ctacacccac agtgacacag
781 ccccattccc aaagccctgc tgtaaacgct tccacttctg gagctgaggg gctgggggga
841 gcgtctggga agtagggcct aggggtggcc atcaatgccc aaaacgcacc agactcagta
901 gacctgagag cgctgggccc acagcctccc ctcggtgccc tgctacctcc tcaggtcagc
961 cctggacatc ccgggtttcc ccaggcctgg cggtaggttt ggggtgaggt ctgtgtcact
1021 gtgttactat agtgaccacc acagtgtcac agagtccatc aaaaacccat ccctgggaac
1081 cttctgccac agccctccct gtggggcacc gctgcgtgcc atgttaggat tttgactgag
1141 gacacagcac catgggtatg gtggctagaa aggtctgaga tccccaggac gcagcaccac
1201 tgtcaatggg ggccccagac gcctggacca gggcctgcgt gggaaaggcc tctgggcaca
1261 ctcaggggct ttttgtgaag ggtcctccta ctgtggtatt gtggtagcta ttgtggtagt
1321 tattatggta ccacagtgat gaacccagca gcaaaaactg accggactcc caaggtttat
1381 gcacacttct ccgctcagag ctctccagga tcagaagagc cgggcccaag ggtttctgcc
1441 cagaccctcg gcctctaggc cccatgtccc cagtcctggg ggcccctggc acagctgtct
1501 ggaccctctc tattccctgg gaagctcctc ctgacagccc cgcctccagt tccaggtgtg
1561 gttattgtca gggggtgtca gactgtggta gttatagtgg ttatggttat ggttatagtt
1621 atggttatac cacagtggtg ctgcccatag cagcaaccag gccaagtaga caggcccctg
1681 ctgtgcagcc ccaggcctcc agctcacctg cttctcctgg ggctctcaag gctgctgttt
1741 tctgcactct cccctctggg caggcccccc agaaccaggg atgaggacgc cccgtcaagg
1801 ccagaaaaga ccaagttgtg ctgagcccag caaggaaggg tccccaaaca aaccaggaag
1861 tttctgaagg tgtctgtgtc acagtggaac tcggtggggc cacagtgaca ctcgccaggc
1921 cagaaacccc atcccaagtc agcggaatgc agagagagca gggaggacat gtttaggatc
1981 tgaggccgca cctgacaccc aggccagcag acgtctcctg tccatggccc acacctaaaa
2041 cgagccacag cctcagagcc cctgaaggag accccgccca caagcccagc ccccacccag
2101 gaggccccag agcacagggc gccccgtcgg attctgaaca gccccgagtc acagtgagaa
2161 tatcgtgatg atggttactg ctacacccac tgtgagaaaa gcttcgtcca aaacggtctc
2221 ctggccacag tcggaggccc cgccagagag gggagcagcc accccaaacc catgttctgc
2281 cggctcccat gaccccgtgc acctggagcc ccacacccag agtttcagac aaaaaccccc
2341 tggaatgcac agtctcagca ggagagccag ccagagccag caagatgggg ctcagtgaca
2401 cccgcaggga caggaggatt ttgtgggggc tcgtgtcact gtgttactat agtgaccacc
2461 acagtgacac agccccattc ccaaagccct actgcaaacg cattccactt ctggggctga

2521 ggggctgggg gagcgtctgg gaaatagggc tcaggggtgt ccatcaatgc ccaaaacgca
2581 ccagaccagc agacctgaga gcgctgggcc cacagcctcc cctcagagcc ctgctgcctc
2641 ctccggtcag ccctggacat cccaggtttc cccaggcctg ccggtaggtt tagaatgagg
2701 tctgtgtcac tgtgatgata cgataggtgt ggttgtagtt attgtagtgt tgctaccaca
2761 gtgtcacaga gtccatcaaa aacccatgcc tggaagcttc ccgccacagc cctccccatg
2821 gggccctgct gcctcctcag gtcagccccg gacatcccgg gtttccccag gctgggcggt
2881 aggtttgggg tgaggtctgt gtcactgtgg tagttgtcct gatggttata gttatggtta
2941 tggttgtggt tatggttatg gttgtagtgg ttatgattgt tatggttatg gtggttatgg
3001 tggttatggt ggttatggtt atagtagtta tagttatagt tatacttacg aatataccac
3061 agtgtcacag agtccatcaa aaacccatcc ctgggagcct cccgccacag ccctccctgc
3121 aggggaccgg tacgtgccat gttaggattt tgatcgagga gacagcacca tgggtatggt
3181 ggctaatgca tgcggccgcc ctgtgaccca aacccgcagg gcagcaggca cgatggacag
3241 gcccgcacag cggcctgaga tccccaggac gcagcaccgc tgtcaatagg ggccccaaat
3301 gcctggacca gggcctgcgt gggaaaggtc tctggccaca ctcgggcttt ttgtgaaggg
3361 ccctcccaca gtggtagttg ttatagtggt tatggttatg gttgtggtta tggttatggt
3421 tatgattata ccatagtgat gaacccagtg gcaaaaactg gctggaaacc caggggctgt
3481 gtgcacgcct cagcttggag ctctccagga gcacaagagc cgggcccaag gatttgtgcc
3541 cagaccctca gcctctaggc cccacgtccc cagtcctggg ggcccctggc tcagctgtct
3601 ggaccctccc tgttccctgg gaagctcctc ctgacagccc cgcctccagt tccaggtgtg
3661 gttattgtca ggcgatgtca gactgtgaga ctatcgtgat gatggttact gctacaccca
3721 cagtgactca ggccctgaca taaagtctga cccgcacaca ggtgtggagc tggccaatgc
3781 atccccaggg gcactgggct cccaagcaca gtggtgccgc ccatagcagc aaccaggcca
3841 agtagacagg cccctgctgc gcagccccag gcatccactt cacctgcttc tcctggggct
3901 ctcaaggctg ctgtctgtcc tctggccctc tgtggcaggc cccccagaac cagggttgag
3961 ggacgccccg tcaaagccag acaaaaccaa ggggtgttga gcccagcaag ggaaggcccc
4021 caaacagacc aggaggtttc tgaaggtgtc tgtgtcacag tgttactata gtgaccacca
4081 cagtgacact cacccagcca gaaaccccat tccaagtcag cggaagcaga gagagcaggg
4141 aggacacgtt taggatctga gactgcacct gacacccagg ccagcagacg tctcccctcc
4201 agggcacccc acacctaaaa tgagccacag cctccggagc ccccgcagga gaccccgccc
4261 acaagcccag cccccaccca ggaggcccca gagctcaggg cgccccgtcg gattccgaac
4321 agccccgagt cacagtggta ttgtggtagc tattgtggta gttattatgg taccactgtc
4381 agaatagcta cgtcaaaaac tgtccagtgg ccactgccgg aggccccgcc agagagggca
4441 gcagccactc tgatcccatg tcctgccggc tcccatgacc cccagcacgc ggagccccac
4501 accctgagtt tcagacaaca acccgctgga atgcacagtc tcagcaggag aacagaccaa
4561 agccagcaaa agggacctcg gtgacaccag tagggacagg aggattttgt gggggctcgt
4621 gtcactgtgg tagttatggt ggttatggtt atggtggtta tggttgttat ggttatggtt
4681 atggttatgg ttataccaca gtgacacaga cccattccca aagccctact gcaaacacac
4741 ccactcctgg ggctgagggg ctgggggagc gtctgggaag tagggtccag gggtgtctat
4801 caatgtccaa aatgcaccag actgcagtag acctgagagc gctgggccca cagcctcccc
4861 tcggtgccct gctgcctcct caggtcagcc ctggacatcc cgggtttccc caggccagat
4921 ggtaggtttg aagtgaggtc tgtgtcactg tgatgatacg ataggtgtgg ttttagttat
4981 tgtagtgttg ctaccacagc atcacacggt ccatcagaaa cccatgccac agccctcccc
5041 gcaggggacc gccgcgtgcc atgttacgat tttgatcgag gacacagcgc catgggtatg
5101 gtggctacca cagcagtgca gcgaaaggcc tgagatcccc aggacgcagc accactgtca

5161 atgggggccc cagacgcctg gaccagggcc tgcgtgggaa aggccgctgg gcacactcag
5221 ggggcttttg tgaaggcccc tcctactgtg gtagttgtta tagtggttat ggttatggtt
5281 atggttgtgg ttatggttat ggttatacca cagtgatgaa actagcagca aaaactggcc
5341 ggacacccag ggaccatgca cacttctcag cttggagctc tccaggacca gaagagtcag
5401 gtctgagggt ttgtagccag accctcggcc tctaggcccc acgtccccag tcctgggggc
5461 ccctggcaca gctgtctgga ccctccctct tccctgggaa gctcctcctg acagccccgc
5521 ctccagttcc aggtgtggtt attgtcaggg ggtgtcagac tgtgagaata tcgtgatgat
5581 ggttactgct acacccacag tggtgctgcc catagcagca accaggccaa gtagacaggc
5641 ccctgctgtg cagccccagg cctccagctc acctgcttct cctggggctc tcaaggtcac
5701 tgttgtctgt actctgccct ctgggcaggc cccccaaaac cagggttgag ggatgatccg
5761 tcaaggcaga caagaccaag ggcactgacc cccagcaagg gaaggctccc aaacagacga
5821 ggaggtttct gaagctgtct gtatcacagt gttactatag tgaccaccac agtgacactc
5881 gccaggccag aaaccccgtc ccaagtcagc ggaagcagag agagcaggga ggacacgttt
5941 aggatctgag gccgcacctg acacccaggg cagcagacgt ctcccctcca gggcataaag
6001 cgagccccag cctccagagc cccctaagca ttccccgccc agcagcccag cccctgcccc
6061 cacccaggag gccccagagc tcagggcgcc tggtcggatt ctgaacagcc ccgagtcaca
6121 gtggtattgt ggtagctatt gtggtagtta ttatggtacc accgtgagaa aaactgtgtc
6181 caaaactgac tcctggcagc agtcggaggc cccgccagag aggggagcag ccggcctgaa
6241 cccatgtcct gccggttccc atgaccccca gcacccagag ccccacgttc agacaacaac
6301 ccgctggaat gcacagtctc agcaggagag ccaggccaga gccagcaaga ggagactcgg
6361 tgacaccagt ctcctgtagg gacaggagga ttttgtgggg gttcgtgtca ctgtggtagt
6421 tatggtggtt atggttatgg tggttatggt tgttatggtt atggttatgg ttatggttat
6481 ggttatacca cagtgacaca accccattcc taaagcccta ctgcaaacgc acccactcct
6541 gggactgagg ggctggggga gcatctggga agtatggcct aggggtgtcc atcaatgccc
6601 aaaatgcacc agactccagt agacctgaga gcgctgggcc cacagcctcc cctcggggcc
6661 ctgctgcctc ctcaggtcag ccctggacat cccgggtttc cccaggcctg gcggtaggtt
6721 tgaagtgagg tctgtgtcac tgtgatgata cgataggtgt ggttttagtt attgtagtgt
6781 tgctaccaca gtgtcacaga gtccatcaaa aactcatgcc tgggagcctc ccaccacagc
6841 cctccctgcg ggggaccgct gcatgccgtg ttaggatttt gatcgaggac acggcgccat
6901 gggtatggtg gctagaattc acgcgtaggg tgagtcagac ccacctgccc tcaatggcag
6961 gcggggaaga ttcagaaagg cctgagatcc ccaggacgca gcaccactgt caatgggggc
7021 cccagacgcc tggaccaggg cctgtgtggg aaaggcctct ggccacactc aggggctttt
7081 tgtgaagggc cctcccacag tggtagttgt tatagtggtt atggttatgg ttatggttgt
7141 ggttatggtt atggttatac cacagtgatg aaaccagcag caaaaactga ccggactcgc
7201 agggtttatg cacacttctc ggctcggagc tctccaggag cacaagagcc aggcccgagg
7261 gtttgtgccc agaccctcgg cctctaggcc caggctctct agcactgggg gcccctggca
7321 cagctgtctg gaccctccct gttccctggg aagctcctcc tgacagcccc gcctccagtt
7381 ccaggtgtgg ttattgtcag ggggtgccac acagtggaac tcggtggggc cacagtggtg
7441 ccgcccatag cagcaaccag gccaagtaga cagacccctg ccacgcagcc ccaggcctcc
7501 agctcacctg cttctcctgg ggctctcaag gctgctgtct gccctctggc cctctgtggt
7561 ctcccggcca ctgctggagg cccagccaga gaagggacca gccgcccgaa catacgacct
7621 tcccagccct catgaccccc agcacttgga gctccacagt gtccccattg gatggtgagg
7681 acgggggccg gggccatctg cacctcccaa catcaccccc aggcagcaca ggcacaaacc
7741 ccaaatccag agccgacacc aggaacacag acaccccaat accctggggg accctggccc

7801 tggtgacttc ccactgggat ccacccccgt gtccacctgg atcaaagacc ccaccgctgt
7861 ctctgtccct cactcagggc ctgctgaggg gcgggtgctt tggagcagac tcaggtttag
7921 gggccaccat tgtggggccc aacctcgacc aggacacaga tttttctttc ctgccctggg
7981 gcaacacaga ctttggggtc tgtgcaggga ggaccttctg gaaagtcacc aagcacagag
8041 ccctgactga ggtggtctca ggaagacccc caggaggggg tttgtgcccc ttcctctcat
8101 gtggacccca tgccccccaa gataggggca tcatgcaggg caggtcctcc atgcagccac
8161 cactaggcaa ctccctggcg ccggtcccca ctgcgcctcc atcccggctc tggggatgca
8221 gccaccatgg ccacaccagg cagcccgggt ccagcaaccc tgcagtgccc aagcccttgg
8281 caggattccc agaggctgga gcccacccct cctcatcccc ccacacctgc acacacacac
8341 ctaccccctg cccagtcccc ctccaggagg gttggagccg cccatagggt gggcgctcca
8401 ggtctcactc actcgcttcc cttcctgggc aaaggagcct cgtgccccgg tcccccctga
8461 cggcgctggg cacaggtgtg ggtactgggc cccagggctc ctccagcccc agctgccctg
8521 ctctccctgg gaggcctggg caccaccaga ccaccagtcc agggcacagc cccagggagc
8581 cgcccactgc cagctcacag gaagaagata agcttcagac cctcagggcc gggagctgcc
8641 ttcctgccac cccttcctgc cccagacctc catgccctcc cccaaccact tacacacaag
8701 ccagggagct gtttccacac agttcaaccc caaaccagga cggcctggca ctcgggtcac
8761 tgccatttct gtctgcattc gctcccagcg cccctgtgtt ccctccctcc tccctccttc
8821 ctttcttcct gcattgggtt c

The ORF sequences from B1-1 to B9-4 of the bovine IgHD region are, for example, the following nucleotide sequences of SEQ ID NOs: 127 to 149.
SEQ ID NO: 127 (B1-1):
   agaataccgtgatgatggttactgctacacc
SEQ ID NO: 128 (B1-2):
   agaatatcgtgatgatggttactgctacacc
SEQ ID NO: 129 (B 1-3): 
agactatcgtgatgatggttactgctacacccacagtgactcaggccctgacataaagtctgacccgcacacaggtgtggag
ctggccaatgcatccccaggggcactgggctcccaag
SEQ ID NO: 130 (B1-4):
   agaatatcgtgatgatggttactgctacacc
SEQ ID NO: 131 (B2-1):
   ttactatagtgaccac
SEQ ID NO: 132 (B2-2):
   ttactatagtgaccac
SEQ ID NO: 133 (B2-3):
   ttactatagtgaccac
SEQ ID NO: 134 (B2-4):
   ttactatagtgaccac
SEQ ID NO: 135 (B3-1):
   gtattgtggtagctattgtggtagttattatggtac
SEQ ID NO: 136 (B3-3):
   gtattgtggtagctattgtggtagttattatggtac
SEQ ID NO: 137 (B3-4):
   gtattgtggtagctattgtggtagttattatggtac
SEQ ID NO: 138 (B4-1):
   gtagttatagtggttatggttatggttatagttatggttatac
SEQ ID NO: 139 (B5-2):
   atgatacgataggtgtggttgtagttattgtagtgttgctac
SEQ ID NO: 140 (B5-3):
   atgatacgata ggtgtggttttagttattgta gtgttgcta c
SEQ ID NO: 141 (B5-4):
   atgatacgata ggtgtggttttagttattgta gtgttgcta c
SEQ ID NO: 142 (B6-2):
   gtagttgttatagtggttatggttatggttgtggttatggttatggttatgattatac
SEQ ID NO: 143 (B6-3):
   gtagttgttatagtggttatggttatggttatggttgtggttatggttatggttatac
SEQ ID NO: 144 (B6-4):
   gtagttgttatagtggttatggttatggttatggttgtggttatggttatggttatac
SEQ ID NO: 145 (B7-3):
   gtagttatggtggttatggttatggtggttatggttgttatggttatggttatggttatggttatac
SEQ ID NO: 146 (B7-4):
   gtagttatggtggttatggttatggtggttatggttgttatggttatggttatggttatggttatggttatac
SEQ ID NO: 147 (B8-2): 
gtagttgtcctgatggttatagttatggttatggttgtggttatggttatggttgtagtggttatgattgttatggttatggtggttatgg
tggttatggtggttatggttatagtagttatagttatagttatacttacgaatatac
SEQ ID NO: 148 (B9-1):
   gaactcggtggggc
SEQ ID NO: 149 (B9-4):
   gaactcggtggggc

The D-region of the cynomolgus macaque immunoglobulin heavy chain locus (Chr7q; G.-Y. Yu et al., Immunogenetics 2016; 68: 417-428) comprises a nucleotide sequence of approximately 44 kb involving 1S5, 2S11, 3S6, 4S24, 5S8, 6S4, 1S10, 2S17, 3S18, 2S34, 5S14, 5S31, 6S3, 1S27, 2S22, 4S36, 4S30, 5S37, 6S20, 1S33, 2S28, 6S32, 3S12, 6S38, 6S26, and 1S39 of IGHD in the order of 5' -> 3' (Figure 15).

The ORF sequences from D1-1 to D1-26 of the D-region of the human immunoglobulin heavy chain locus may be substituted in order to comprise ORF sequences from 1S5 to 1S35 of the D-region of the monkey immunoglobulin heavy chain locus.

The ORF sequences from 1S5 to 1S35 of the cynomolgus macaque D-region are, for example, the following nucleotide sequences of SEQ ID NOs: 150 to 175.
SEQ ID NO: 150 (1S5):
   ggtataactggaactac
SEQ ID NO: 151 (2S11):
   agaatattgtagtagtacttactgctcctcc
SEQ ID NO: 152 (3S6):
   gtattacgaggatgattacggttactattacacccacagcgt
SEQ ID NO: 153 (4S24):
   tgactacggtagcagctac
SEQ ID NO: 154 (5S8):
   gtggatacagctacagttac
SEQ ID NO: 155 (6S4):
   gggtatagcagcggctggtac
SEQ ID NO: 156 (1S10):
   ggtatagctggaacgac
SEQ ID NO: 157 (2S17):
   agaatactgtactggtagtggttgctatgcc
SEQ ID NO: 158 (3S18):
   gtactggggtgattattatgac
SEQ ID NO: 159 (2S34):
   agcatattgtagtggtggtgtctgctacacc
SEQ ID NO: 160 (5S14):
   gtggatacagctacagttaccacagttttgccacc
SEQ ID NO: 161 (5S31):
   gtggata ta gcta cggttac
SEQ ID NO: 162 (6S9):
   gggtatagcagctggtcc
SEQ ID NO: 163 (1S27):
   ggtataactggaatgac
SEQ ID NO: 164 (2S22):
   agaatattgtagtggtatttactgctatgcc
SEQ ID NO: 165 (4S36):
   tgaatacagtaactac
SEQ ID NO: 166 (4S30):
   tgactacggtaactac
SEQ ID NO: 167 (5S37):
   ggggatacagtgggtacagttac
SEQ ID NO: 168 (6S20):
   gggtatagcggcagctggaac
SEQ ID NO: 169 (1S33):
   ggaacacctggaacgac
SEQ ID NO: 170 (2S28):
   agcacactgtagtgatagtggctgctcctcc
SEQ ID NO: 171 (6S32):
   gggtatagcggtggctggtcc
SEQ ID NO: 172 (3S12):
   gtattactatagtggtagttattactaccacagtgt
SEQ ID NO: 173 (6S38):
   gggtatagcagcagcta
SEQ ID NO: 174 (6S26):
   gggtatagcagcggctggtcc
SEQ ID NO: 175 (1S39):
   ggtatagtgggaactacaac

An alternative modified sequence comprises 26 types of modified ORF sequences from D1-1 to D1-26 of the D-region of the human immunoglobulin heavy chain locus produced on the basis of a human antibody heavy chain CDR3 sequence of 18 or more amino acids, instead of ORFs from D1-1 to D1-26 of the D-region of the human immunoglobulin heavy chain locus.

The presence of a long CDRH3 sequence in a human antibody, albeit at low frequency, is known (L. Yu and Y. Guan, Frontiers in Immunology, 2014, 24, doi: 10.3389/fimmu.2014.00250).

Such modified ORFs of the D-region comprise, for example, the nucleotide sequences represented by SEQ ID NOs: 75 to 100 as shown in Figure 16, nucleotide sequences having 90% or higher, 95% or higher, 97% or higher, 98% or higher, or 99% or higher identity to the nucleotide sequences, or nucleotide sequences comprising the deletion, substitution, or addition of one or several nucleotides (or bases) in said nucleotide sequences. ORFs from D1-1 to D1-26 of the human IgHD region may be substituted with corresponding 26 types of modified ORF sequences described above. The nucleotide sequence of the human minimalized D-region thus substituted comprises, for example, the nucleotide sequence of SEQ ID NO: 3, a nucleotide sequence having 90% or higher, 95% or higher, 97% or higher, 98% or higher, or 99% or higher identity to the nucleotide sequence, or a nucleotide sequence comprising the deletion, substitution, or addition of one or several nucleotides (or bases) of said nucleotide sequence by.

### SEQ ID NO: 3:

### [Chem. 3]

1 gtcgcctcta cgggaaatcc gggactcagc agtaaccctc aggaagcagg gcccaggcgc
61 cgtttaatag gaggcttcct cacaatgaaa cttttagaaa gccttgacta caatgatgac
121 cttggtgtgg ctgtgaacac tgtcagctcc cacagctgct gcagcaaaaa atgtccatag
181 acagggtggg ggcccggggt cgtctgctgt cctgctcagc ccacagcacg catggaggat
241 ctgaggtgcc acacctgacg cccaggccag aacatgcctc cctccagggt gacctgccat
301 gtcctgcatt gctggaggga caggggcagc ctatgaggat ctggggccag gagatgaatc
361 ctattaaccc agaggaaaac taacaggacc caagcaccct ccccgttgaa gctgacctgc
421 ccagaggggc ctgggcccac cccacacacc ggggcggaat gtgtacaggc cccggtctct
481 gtgggtgttc cgctaactgg ggctcccagt gctcacccca caactaaagc gagccccagc
541 ctccagagcc cccgaaggag atgccgccca caagcccagc ccccatccag gaggccccag
601 agctcagggc gccggggcag attctgaaca gccccgagtc acggtggggc acaactggat
661 ggggctggct gggcaaacct atcggagctt caccgtgaga aaaactgtgt ccaaaactct
721 ctcctggccc ctgctggagg ccgcgccaga gaggggagca gccgccccga acctaggtcc
781 tgctcagctc acacgacccc cagcagccag agcacagtcc ctgagtttca gacaaaaacc
841 ccctggaaat catagtatca gcaggagaac tagccagaga cagcaagagg ggactcagtg
901 actcccgcgg ggacaggagg attttgtggg ggctcgtgtc actgtgcggc aggaatggct
961 gggatattgt agccaccaca agcacccagt cacagtgaca cagccccatt cccaaagccc
1021 tgctgtaaac gcttccactt ctggagctga ggggctgggg ggagcgtctg ggaagtaggg
1081 cctaggggtg gccatcaatg cccaaaacgc accagactca gtagacctga gagcgctggg
1141 cccacagcct cccctcggtg ccctgctacc tcctcaggtc agccctggac atcccgggtt
1201 tccccaggcc tggcggtagg tttggggtga ggtctgtgtc actgtggtgt atcacaatct
1261 ttggagtggt gatccctcag agcggcccct cacagtgtca cagagtccat caaaaaccca
1321 tccctgggaa ccttctgcca cagccctccc tgtggggcac cgctgcgtgc catgttagga
1381 ttttgactga ggacacagca ccatgggtat ggtggctaga aaggtctgag atccccagga
1441 cgcagcacca ctgtcaatgg gggccccaga cgcctggacc agggcctgcg tgggaaaggc
1501 ctctgggcac actcaggggc tttttgtgaa gggtcctcct actgtggcct gcagcagctt
1561 ggttcagtgt atttaagccc ccactcaatt cacagtgatg aacccagcag caaaaactga
1621 ccggactccc aaggtttatg cacacttctc cgctcagagc tctccaggat cagaagagcc
1681 gggcccaagg gtttctgccc agaccctcgg cctctaggcc ccatgtcccc agtcctgggg
1741 gcccctggca cagctgtctg gaccctctct attccctggg aagctcctcc tgacagcccc
1801 gcctccagtt ccaggtgtgg ttattgtcag ggggtgtcag actgtggggc gatgacagca
1861 gcggctacag catcaccgcc atcgagtatt cacagtggtg ctgcccatag cagcaaccag
1921 gccaagtaga caggcccctg ctgtgcagcc ccaggcctcc agctcacctg cttctcctgg
1981 ggctctcaag gctgctgttt tctgcactct cccctctggg caggcccccc agaaccaggg
2041 atgaggacgc cccgtcaagg ccagaaaaga ccaagttgtg ctgagcccag caaggaaggg
2101 tccccaaaca aaccaggaag tttctgaagg tgtctgtgtc acagtgaggc ccatacagct
2161 gggacgacag cccccaggac aattactata cacagtgaca ctcgccaggc cagaaacccc
2221 atcccaagtc agcggaatgc agagagagca gggaggacat gtttaggatc tgaggccgca
2281 cctgacaccc aggccagcag acgtctcctg tccatggccc acacctaaaa cgagccacag
2341 cctcagagcc cctgaaggag accccgccca caagcccagc ccccacccag gaggccccag
2401 agcacagggc gccccgtcgg attctgaaca gccccgagtc acagtgactg cacggccgga

2461 ggatctacgg cattgttgca ttcaatgagt ggttcaccta cttctacaca ctgtgagaaa
2521 agcttcgtcc aaaacggtct cctggccaca gtcggaggcc ccgccagaga ggggagcagc
2581 caccccaaac ccatgttctg ccggctccca tgaccccgtg cacctggagc cccacaccca
2641 gagtttcaga caaaaacccc ctggaatgca cagtctcagc aggagagcca gccagagcca
2701 gcaagatggg gctcagtgac acccgcaggg acaggaggat tttgtggggg ctcgtgtcac
2761 tgtgcagaca ccacgacgtg ttcatgctgg taccaatcgc cggctggtca cagtgacaca
2821 gccccattcc caaagcccta ctgcaaacgc attccacttc tggggctgag gggctggggg
2881 agcgtctggg aaatagggct caggggtgtc catcaatgcc caaaacgcac cagaccagca
2941 gacctgagag cgctgggccc acagcctccc ctcagagccc tgctgcctcc tccggtcagc
3001 cctggacatc ccaggtttcc ccaggcctgc cggtaggttt agaatgaggt ctgtgtcact
3061 gtgcagcaag cacagactgc gggactacgt gctgtacgac gactacgggc tgatcaacta
3121 tcaggagtgg aacgactacc tggagttccc acagtgtcac agagtccatc aaaaacccat
3181 gcctggaagc ttcccgccac agccctcccc atggggccct gctgcctcct caggtcagcc
3241 ccggacatcc cgggtttccc caggctgggc ggtaggtttg gggtgaggtc tgtgtcactg
3301 tgaagcaagc accgcctgag agattacttc ctgtacaacg agtatggccc caactacgag
3361 gagtggggcg actatctggc aaccccacag tgtcacagag tccatcaaaa acccatccct
3421 gggagcctcc cgccacagcc ctccctgcag gggaccggta cgtgccatgt taggattttg
3481 atcgaggaga cagcaccatg ggtatggtgg ctaatgcatg cggccgccct gtgacccaaa
3541 cccgcagggc agcaggcacg atggacaggc ccgcacagcg gcctgagatc cccaggacgc
3601 agcaccgctg tcaatagggg ccccaaatgc ctggaccagg gcctgcgtgg gaaaggtctc
3661 tggccacact cgggcttttt gtgaagggcc ctcctgctgt gcaagtcttg ttgtggcggc
3721 aggagatact gcaacggagc tgactgcttc aactgggact catagtgatg aacccagtgg
3781 caaaaactgg ctggaaaccc aggggctgtg tgcacgcctc agcttggagc tctccaggag
3841 cacaagagcc gggcccaagg atttgtgccc agaccctcag cctctaggcc ccacgtcccc
3901 agtcctgggg gcccctggct cagctgtctg gaccctccct gttccctggg aagctcctcc
3961 tgacagcccc gcctccagtt ccaggtgtgg ttattgtcag gcgatgtcag actgtgccag
4021 ctgggctacg acttctggtc cggctactac accgacaccc tgtactacta tttcggcaca
4081 cagtggtgcc gcccatagca gcaaccaggc caagtagaca ggcccctgct gcgcagcccc
4141 aggcatccac ttcacctgct tctcctgggg ctctcaaggc tgctgtctgt cctctggccc
4201 tctgtggcag gccccccaga accagggttg agggacgccc cgtcaaagcc agacaaaacc
4261 aaggggtgtt gagcccagca agggaaggcc cccaaacaga ccaggaggtt tctgaaggtg
4321 tctgtgtcac agtgcggcaa gtactacgac ttctggagcg gctatcctcc tggcgaggag
4381 tactcacagt gacactcacc cagccagaaa ccccattcca agtcagcgga agcagagaga
4441 gcagggagga cacgtttagg atctgagact gcacctgaca cccaggccag cagacgtctc
4501 ccctccaggg caccccacac ctaaaatgag ccacagcctc cggagccccc gcaggagacc
4561 ccgcccacaa gcccagcccc cacccaggag gccccagagc tcagggcgcc ccgtcggatt
4621 ccgaacagcc ccgagtcaca gcggttccag gagagcggcc ctcctagact ggatagatgg
4681 tccggcagga actactacta ctacagcggc acactgtcag aatagctacg tcaaaaactg
4741 tccagtggcc actgccggag gccccgccag agagggcagc agccactctg atcccatgtc
4801 ctgccggctc ccatgacccc cagcacgcgg agccccacac cctgagtttc agacaacaac
4861 ccgctggaat gcacagtctc agcaggagaa cagaccaaag ccagcaaaag ggacctcggt
4921 gacaccagta gggacaggag gattttgtgg gggctcgtgt cactgtgtgg cgaggagcac
4981 gaggtaccac agctgcactc ctggagcggc agaaatctgt accactatac aggcgcacag
5041 tgacacagac ccattcccaa agccctactg caaacacacc cactcctggg gctgaggggc

5101 tgggggagcg tctgggaagt agggtccagg ggtgtctatc aatgtccaaa atgcaccaga
5161 ctgcagtaga cctgagagcg ctgggcccac agcctcccct cggtgccctg ctgcctcctc
5221 aggtcagccc tggacatccc gggtttcccc aggccagatg gtaggtttga agtgaggtct
5281 gtgtcactgt gagcaggcgg ccctgactac aggaacggct acaactacta cgacttttac
5341 gatggctact acaactacca ctacacacag catcacacgg tccatcagaa acccatgcca
5401 cagccctccc cgcaggggac cgccgcgtgc catgttacga ttttgatcga ggacacagcg
5461 ccatgggtat ggtggctacc acagcagtgc agcgaaaggc ctgagatccc caggacgcag
5521 caccactgtc aatgggggcc ccagacgcct ggaccagggc ctgcgtggga aggccgctgg
5581 ggcacactca ggggcttttt gtgaaggccc ctcctactgt gggcaggcgg ccccatctgg
5641 cacgacgacg tgaagtacta cgactttaac gatggctatt acaattacca ctacacacag
5701 tgatgaaact agcagcaaaa actggccgga cacccaggga ccatgcacac ttctcagctt
5761 ggagctctcc aggaccagaa gagtcaggtc tgagggtttg tagccagacc ctcggcctct
5821 aggccccacg tccccagtcc tgggggcccc tggcacagct gtctggaccc tccctcttcc
5881 ctgggaagct cctcctgaca gccccgcctc cagttccagg tgtggttatt gtcagggggt
5941 gtcagactgt gtataccccc cccttcggta ttacggtatt ttgactggtt accccccagg
6001 gagggttact acggtacaca gtggtgctgc ccatagcagc aaccaggcca agtagacagg
6061 cccctgctgt gcagccccag gcctccagct cacctgcttc tcctggggct ctcaaggtca
6121 ctgttgtctg tactctgccc tctgggcagg ccccccaaaa ccagggttga gggatgatcc
6181 gtcaaggcag acaagaccaa ggggcactga ccccagcaag ggaaggctcc caaacagacg
6241 aggaggtttc tgaagctgtc tgtatcacag tgcaccgact acaccatcga cgatgccggc
6301 atccactatc agggctctgg cacattctgg tactcacagt gacactcgcc aggccagaaa
6361 ccccgtccca agtcagcgga agcagagaga gcagggagga cacgtttagg atctgaggcc
6421 gcacctgaca cccagggcag cagacgtctc ccctccaggg cataaagcga gccccagcct
6481 ccagagcccc ctaagcattc cccgcccagc agcccagccc ctgcccccac ccaggaggcc
6541 ccagagctca gggcgcctgg tcggattctg aacagccccg agtcacagtg caccgactac
6601 accatcgacg atcagggaag gttctaccag ggcagcggca ccttctggta ttcaccgtga
6661 gaaaaactgt gtccaaaact gactcctggc agcagtcgga ggccccgcca gagaggggag
6721 cagccggcct gaacccatgt cctgccggtt cccatgaccc ccagcaccca gagccccacg
6781 ttcagacaac aacccgctgg aatgcacagt ctcagcagga gagccaggcc agagccagca
6841 agaggagact cggtgacacc agtctcctgt agggacagga ggattttgtg ggggttcgtg
6901 tcactgtgga accactacgg ctctggcagc tacttttata ggacagccta ctactacgcc
6961 acacagtgac acaaccccat tcctaaagcc ctactgcaaa cgcacccact cctgggactg
7021 aggggctggg ggagcatctg ggaagtatgg cctaggggtg tccatcaatg cccaaaatgc
7081 accagactcc agtagacctg agagcgctgg gcccacagcc tcccctcggg gccctgctgc
7141 ctcctcaggt cagccctgga catcccgggt ttccccaggc ctggcggtag gtttgaagtg
7201 aggtctgtgt cactgtgtat taggcgggga ggaggggctc gctattacta tcatgaggca
7261 ggggcacagt gtcacagagt ccatcaaaaa ctcatgcctg ggagcctccc accacagccc
7321 tccctgcggg ggaccgctgc atgccgtgtt aggattttga tcgaggacac ggcgccatgg
7381 gtatggtggc tagaattcac gcgtagggtg agtcagaccc acctgccctc aatggcaggc
7441 ggggaagatt cagaaaggcc tgagatcccc aggacgcagc accactgtca atgggggccc
7501 cagacgcctg gaccagggcc tgtgtgggaa aggcctctgg ccacactcag gggctttttg
7561 tgaagggccc tcctgctgtg caagcacggc aggcgcatct atggcgtggt tgcctttaag
7621 gagtggttca cctactttta cacacagtga tgaaaccagc agcaaaaact gaccggactc
7681 gcagggttta tgcacacttc tcggctcgga gctctccagg agcacaagag ccaggcccga

7741 gggtttgtgc ccagaccctc ggcctctagg cccaggctct ctagcactgg gggcccctgg
7801 cacagctgtc tggaccctcc ctgttccctg ggaagctcct cctgacagcc ccgcctccag
7861 ttccaggtgt ggttattgtc agggggtgcc aggccgtgcc tgcacggcaa gcgcatctat
7921 ggcattgtgg ccctgggcga gctgttcacc tacttttaca cacagtggtg ccgcccatag
7981 cagcaaccag gccaagtaga cagacccctg ccacgcagcc ccaggcctcc agctcacctg
8041 cttctcctgg ggctctcaag gctgctgtct gccctctggc cctctgtggg ccccccagaa
8101 ccagtgttga gggacacccc tgtccagggc agccaagtcc aagaggcgcg ctgagcccag
8161 caagggaagg cccccaaaca aaccaggagg tttctgaagc tgtctgtgtc acagtccatg
8221 agacctgttg accacggcat cgactacagc gggctgttcg tgtcacaatg acactgggca
8281 ggacagaaac cccatcccaa gtcagccgaa ggcagagaga gcaggcagga cacatttagg
8341 atctgaggcc acacctgaca ctcaagccaa cagatgtctc ccctccaggg ccacacctaa
8401 ggtgagccac agccgccaga gcctccacag gagaccccac gcagcagccc agcccctacc
8461 caggaggccc cagagctcag ggcgcctggg tggattctga acagccccga gtcacggtga
8521 gggcaaggtg atagcagcag ctggtacccc cagggttttt actactacta ctacggtaca
8581 ctgtgagaaa agctatgtcc aaaactgtct cccggccact gctggaggcc cagccagaga
8641 agggaccagc cgcccgaaca tacgaccttc ccagccctca tgacccccag cacttggagc
8701 tccacagtgt ccccattgga tggtgaggac gggggccggg gccatctgca cctcccaaca
8761 tcacccccag gcagcacagg cacaaacccc aaatccagag ccgacaccag gaacacagac
8821 accccaatac cctgggggac cctggccctg gtgacttccc actgggatcc acccccgtgt
8881 ccacctggat caaagacccc accgctgtct ctgtccctca ctcagggcct gctgaggggc
8941 gggtgctttg gagcagactc aggtttaggg gccaccattg tggggcccaa cctcgaccag
9001 gacacagatt tttctttcct gccctggggc aacacagact ttgggtctgg tgcagggagg
9061 accttctgga aagtcaccaa gcacagagcc ctgactgagg tggtctcagg aagaccccca
9121 ggagggggtt tgtgcccctt cctctcatgt ggaccccatg ccccccaaga taggggcatc
9181 atgcagggca ggtcctccat gcagccacca ctaggcaact ccctggcgcc ggtccccact
9241 gcgcctccat cccggctctg gggatgcagc caccatggcc acaccaggca gcccgggtcc
9301 agcaaccctg cagtgcccaa gcccttggca ggattcccag aggctggagc ccacccctcc
9361 tcatcccccc acacctgcac acacacacct accccctgcc cagtccccct ccaggagggt
9421 tggagccgcc catagggtgg gcgctccagg tctcactcac tcgcttccct tcctgggcaa
9481 aggagcctcg tgccccggtc ccccctgacg gcgctgggca caggtgtggg tactgggccc
9541 cagggctcct ccagccccag ctgccctgct ctccctggga ggcctgggca ccaccagacc
9601 accagtccag ggcacagccc cagggagccg cccactgcca gctcacagga agaagataag
9661 cttcagaccc tcagggccgg gagctgcctt cctgccaccc cttcctgccc cagacctcca
9721 tgccctcccc caaccactta cacacaagcc agggagctgt ttccacacag ttcaacccca
9781 aaccaggacg gcctggcact cgggtcactg ccatttctgt ctgcattcgc tcccagcgcc
9841 cctgtgttcc ctccctcctc cctccttcct ttcttcctgc attgggttc

A method for determining the modified ORF sequences on the basis of the human antibody heavy chain CDR3 sequence may comprise, for example, steps as described below.

In this example, CDR3 is defined as a domain from a C-terminal sequence "CAR"/"CAK" of VH to upstream of a N-terminal sequence "WG" of VH in the amino acid sequence of the human antibody heavy chain (M. Chiu et al., Antibodies 2019; 8 (4): 55; doi.org/10.3390/antib8040055).

Step 1 is of searching for a known sequence of CDR3 having 18 or more amino acids.

Step 2 is of extracting the nucleotide sequence of CDR3.

Step 3 is of introducing synonymous (which does not change an amino acid) substitution mutations into a somatic mutation hotspot sequence as much as possible.

The term "identity" used herein may be determined using a protein or gene search system based on BLAST or FASTA (Zheng Zhang et al., J.Comput. Biol. 2000; 7: 203-214; Altschul, S. F. et al., Journal of Molecular Biology 1990; 215: 403-410; Pearson, W. R. et al., Proc. Natl. Acad. Sci. U.S.A., 1988; 85: 2444-2448) with or without introducing gaps.

### [Method for modifying D-region of human immunoglobulin heavy chain locus]

The D-region of the human immunoglobulin heavy chain locus may be modified by, for example, a method comprising the following steps.

### <Step 1>

A mammalian artificial chromosome vector comprising human immunoglobulin heavy chain and light chain loci ([Ig-NAC]) is provided.

The Ig-NAC has a structure comprising a human immunoglobulin heavy chain locus and a human immunoglobulin light chain κ (or λ) locus from the telomere side toward the centromere side (e.g., JP Patent No. 6868250).

The Ig-NAC is capable of stably replicating and being stably distributed as a chromosome independent from the native chromosome of a host cell. An example of this vector is a rodent artificial chromosome vector (e.g., a mouse or rat artificial chromosome vector). The production of the rodent artificial chromosome vector is described in, for example, JP Patent Nos. 6775224 and 6868250 provided by the present applicant.

A specific production method is as described below.

The mammalian artificial chromosome vector comprises the centromere of the animal, a long-arm fragment proximal to the centromere, and a short-arm fragment, if present in the animal, and the telomere, which may be produced by, for example, the telomere truncation of a mammalian chromosome. For example, a mouse-derived chromosome fragment is a fragment of any of mouse chromosomes 1 to 19 and X and Y chromosomes, preferably a fragment of any of mouse chromosomes 1 to 19 (a long-arm fragment obtained by deleting at least 99.5%, preferably almost 100% of all endogenous genes from the long arm). This fragment comprises a long-arm fragment obtained by deleting a long-arm distal region at a mouse chromosome long-arm site proximal to the centromere. Sequence information of mouse chromosomes is available from DDBJ/EMBL/GenBank, chromosome databases at Santa Cruz Biotechnology, Inc., and other organizations. More specifically, for example, for an artificial chromosome vector derived from a fragment of mouse chromosome 15, the long-arm fragment is composed of a long-arm fragment, from which a more distal region compared to, for example, but not limited to, AC121307 or AC161799, is deleted. Alternatively, for an artificial chromosome vector derived from a fragment of mouse chromosome 16, the long-arm fragment is composed of a long-arm fragment, from which a more distal region compared to, for example, but not limited to, Gm35974, is deleted. Alternatively, the long-arm fragment is composed of a long-arm fragment derived from mouse chromosome 10, from which a long-arm distal region from gene Gm8155, a long-arm site of mouse chromosome 10, is deleted.

Examples of the mouse artificial chromosome vector include mouse artificial chromosomes contained in a deposited cell line DT40 (10MAC) T5-26 (international deposition No. NITE BP-02656) and mouse artificial chromosomes contained in a deposited cell line DT40 (16MAC) T1-14 (international deposition No. NITE BP-02657).

The mammalian artificial chromosome vector of the present invention may be produced by, for example, a method comprising the following steps (a) to (c):
(a) obtaining a cell carrying a mammalian chromosome;
(b) deleting a long-arm distal region of the mouse chromosome so as not to include a majority (i.e., 99.5% to 100%, preferably 100%) of endogenous genes (the number of the genes); and
(c) inserting one or more DNA sequence insertion sites into a long-arm proximal region.

The order of the steps (b) and (c) may be interchangeable.

Hereinafter, each step will be described.

### <Step (1a)>

Firstly, in order to produce the mammalian artificial chromosome vector of the present invention, a cell carrying a mammalian chromosome is produced. For example, a mammalian fibroblast carrying a mammalian chromosome labeled with a drug resistance gene (e.g., blasticidin S resistance gene (BSr)) is subjected to cell fusion to a mouse A9 cell (ATCC VA20110-2209) with a neo gene, a G418 resistance gene, introduced therein. The mouse A9 hybrid cell carrying the mammalian chromosome labeled with a drug resistance gene may be used to transfer the chromosome into a cell having a high homologous recombination frequency, thereby producing the cell carrying a mammalian chromosome. The mammalian fibroblast is available on the basis of procedures described in literatures. For example, the mouse fibroblast may be established from C57B6 mouse commercially available from CLEA Japan, Inc. An example of an available cell having a high homologous recombination frequency is a chicken DT40 cell (Dieken et al., Nature Genetics, 12, 174-182, 1996). Furthermore, the transfer may be carried out using a known chromosome transfer technique, for example, microcell mediated chromosome transfer (Koi et al., Jpn. J. Cancer Res., 80, 413-418, 1973).

### <Step (1b)>

In a cell carrying a single mammalian-derived chromosome, a long-arm distal region of the mammalian chromosome and a short-arm distal region, if present in the animal, are deleted. It is important to delete (or remove or cleave out) a majority of endogenous genes present in the long arm and the short arm to construct an artificial chromosome carrying the mammalian centromere. That is, it is important to determine a cleavage site so as to delete (or remove or cleave out) at least 99.5%, preferably at least 99.7%, more preferably at least 99.8%, most preferably 99.9 to 100%, further most preferably 100% of all endogenous genes (the number of the genes) present in the long arm and the short arm. Thus, cells, tissues, or individuals, into which the artificial chromosome is introduced, are derived from a mammal, preferably derived from a rodent such as a mouse or a rat, stably retain the artificial chromosome at a high retention rate. The endogenous genes may be deleted by, for example, telomere truncation. Specifically, in order to obtain a deletion mutant by telomere truncation, a targeting vector carrying a telomere sequence is constructed and is used to obtain a clone into which an artificial or natural telomere sequence has been inserted at a desirable position on the chromosome by homologous recombination in a cell carrying a mammalian chromosome, . For example, the desirable position (or site) is a cleavage position of a long-arm distal region to be deleted, and the long-arm distal region is deleted by the telomere sequence is inserted or substituted into this position via the homologous recombination. Such a position may be appropriately determined depending on a target sequence design when constructing a targeting vector. For example, a target sequence is designed on the basis of the DNA sequence of the mammalian chromosome long arm, so that the telomere truncation occurs at a position closer to the telomere side than the target sequence. As a result, a mammalian chromosome fragment resulting from deletion of a majority of endogenous genes is obtained. For other chromosomes, the telomere truncation may be carried out in the same manner. The short-arm distal region is cleaved in the same manner.

### <Step (1c)>

A site-directed recombinase recognition site may be preferably inserted as a DNA sequence insertion site. Specifically, the phenomenon, in which a certain enzyme recognizes a specific recognition site and causes DNA recombination specifically at the recognition site, is known. In the mammalian artificial chromosome vector according to the present invention, the sequence of the human immunoglobulin heavy chain or locus and/or the human immunoglobulin light chain gene or locus of interest can be inserted or incorporated using a system having such an enzyme and its recognition site. Examples of such system include a system having bacteriophage P1-derived Cre enzyme and its recognition site, the loxP sequence (a Cre/loxP system; B. Sauer in Methods of Enzymology, 1993, 225, 890-900); a system having budding yeast-derived Flp enzyme and its recognition site, FRT (Flp Recombination Target) sequence (a Flp/FRT system); a system having Streptomyces phagederived φC31 integrase and its recognition site, φC31 attB/attP sequence; a system having R4 integrase and its recognition site, R4 attB/attP sequence; a system having TP901-1 integrase and its recognition site, TP901-1 attB/attP sequence; and a system having Bxb1 integrase and its recognition site, Bxb1 attB/attP sequence; but are not limited to provided that the system can function as a DNA sequence insertion site.

In order to insert such a site-directed recombinase recognition site, known methods, such as homologous recombination, may be employed. The position and the number of insertions may be appropriately determined in a long-arm proximal region and a short-arm proximal region.

A single type of recognition site(s) or different types of recognition sites may be inserted into the mammalian artificial chromosome vector. The setting of a recognition site enables identification of an insertion position for a gene or locus of interest, i.e., the human immunoglobulin heavy chain gene or locus, the human immunoglobulin light chain κ gene or locus, or the human immunoglobulin light chain λ gene or locus. Thus, the insertion position is fixed, and no unexpected positional effects would be exerted.

Preferably, a reporter gene may be inserted, in addition to the sequence of the gene or locus of interest, into the mammalian artificial chromosome vector. Examples of reporter genes include, but are not particularly limited to, fluorescent protein genes (e.g., green fluorescent protein (GFP or EGFP) gene and yellow fluorescent protein (YFP) gene), tag-protein-encoding DNA, β-galactosidase gene, and luciferase gene.

The mammalian artificial chromosome vector may further comprise a selection marker gene. A selection marker is effective when selecting a cell transformed with the vector. As a selection marker gene, either or both of a positive selection marker gene and a negative selection marker gene are exemplified. Examples of positive selection marker genes include drug-resistant genes such as neomycin-resistant gene, ampicillin-resistant gene, blasticidin S (BS)-resistant gene, puromycin-resistant gene, geneticin (G418)-resistant gene, and hygromycin-resistant gene. In addition, examples of negative selection marker genes include herpes simplex thymidine kinase (HSV-TK) gene and diphtheria toxin A fragment (DT-A) gene. In general, HSV-TK is used in combination with ganciclovir or acyclovir.

The "human immunoglobulin gene or locus" used herein refers to a human immunoglobulin heavy chain gene or locus derived from human chromosome 14, a human immunoglobulin light chain κ gene or locus derived from human chromosome 2, and/or a human immunoglobulin light chain λ gene or locus derived from human chromosome 22, unless otherwise specified. Specifically, the human immunoglobulin gene or locus is represented by the base sequence as set forth in, for example, immunoglobulin heavy locus (human) NC_000014.9 ((base numbers 105586437..106879844) or (base numbers 105264221..107043718)) of human chromosome 14, immunoglobulin kappa locus (human) NC_000002.12 ((base numbers 88857361..90235368) or (base numbers 88560086..90265666)) of human chromosome 2, and immunoglobulin lambda locus (human) NC_000022.11 ((base numbers 22026076..22922913) or (base numbers 21620362..23823654)) of human chromosome 22. The human immunoglobulin heavy chain gene or locus has a base length of approximately 1,3 Mb, the human immunoglobulin light chain κ gene or locus has a base length of approximately 1.4 Mb, and the human immunoglobulin light chain λ gene or locus has a base length of approximately 0.9 Mb.

The mouse antibody heavy chain gene or locus is present on mouse chromosome 12, the mouse antibody light chain κ gene or locus is present on mouse chromosome 6, and the mouse antibody light chain λ gene or locus is present on mouse chromosome 16. Specifically, the mouse antibody heavy chain gene or locus is represented by, for example, the base sequence of Chromosome 12, NC_000078.6 (113258768..116009954, complement), the mouse antibody light chain κ gene or locus is represented by the base sequence of Chromosome 6, NC_000072.6 (67555636..70726754), and the mouse antibody light chain λ gene or locus is represented by the base sequence of Chromosome 16, NC_000082.6 (19026858..19260844, complement).

The rat antibody heavy chain gene or locus is present on rat chromosome 6, the rat antibody light chain κ gene or locus is present on rat chromosome 4, and the rat antibody light chain λ gene or locus is present on rat chromosome 11. The base sequences of these genes or loci are available from, for example, U.S. NCBI (e.g., GenBank) and known literature.

### <Step 2>

In the mammalian artificial chromosome vector (Ig-NAC) comprising a human immunoglobulin (heavy chain and/or light chain) locus produced in the step 1, the D-region present between the V-region and the J-region of the human heavy chain locus is deleted from the human immunoglobulin heavy chain locus by a method, for example, genome editing (J.M. Chylinski et al., Science 2012; 337: 816-821) or a site-directed recombination technique (e.g., using site-directed recombinase), to produce "Ig-NAC(ΔDH)".

The term "genome editing" used herein is a technique of performing genomic DNA editing or gene modification using, for example, an artificial cleavage enzyme such as TALEN (TALE nuclease) or a CRISPR-Cas system. In the method of the present invention, any technique of genome editing may be used, and a CRISPR-Cas system is preferably used.

Particularly, a CRISPR/Cas9 system has been found from the adaptive immune system against a bacterial or archaebacterial virus or plasmid and is capable of constructing a vector relatively conveniently and modifying a plurality of genes at the same time (Jinek et al., Science, 17, 337 (6096): 816-821, 2012; and Sander et al., Nature Biotechnology, 32 (4): 347-355, 2014). This system comprises Cas9 protein and guide RNA (gRNA) having a target sequence of approximately 20 bp. By their coexpression in cells, the gRNA recognizes a PAM sequence proximal to the target sequence and specifically binds to target genomic DNA, and the Cas9 protein induces a double-stranded cut upstream (on the 5' side) of the PAM sequence. The Cas9 protein currently used most commonly is the type of SpCas9, which has less limitation for a mutation introduction position because the PAM sequence is NGG (N = C, G, A, or T). In the present invention, the site of the human immunoglobulin heavy chain locus to be targeted by the guide RNA is, for example, and not limited thereto, a 5' upstream site of the human D1-1 fragment. The target sequence of the guide RNA is, for example, 5'-AGATCCTCCATGCGTGCTGTGGG-3' (SEQ ID NO: 4).

The "site-directed recombinase" used herein is an enzyme for causing recombination with the DNA sequence of interest, specifically at its recognition site. Site-directed recombination may be carried out by using such recombinase and its recognition site. Examples of the site-directed recombinase include Cre integrase (also referred to as Cre recombinase), Flp recombinase, φC31 integrase, R4 integrase, TP901-1 integrase, and Bxb1 integrase. Examples of the recognition site for the enzyme include loxP (Cre recombinase recognition site), FRT (Flp recombinase recognition site), φC31 attB and φC31 attP (φC31 recombinase recognition site), R4 attB and R4 attP (R4 recombinase recognition site), TP901-1 attB and TP901-1 attP (TP901-1 recombinase recognition site), and Bxb1 attB and Bxb1 attP (Bxb1 recombinase recognition site).

An acceptor site for introducing a synthetic polynucleotide of the D-region is inserted into the site from which the D-region of the human immunoglobulin heavy chain locus has been deleted. An example of the acceptor site is a cassette comprising a site-directed recombinase recognition site. An exemplary method for inserting the acceptor site is described in <Step 3> below.

In this context, the site-directed recombinase and its recognition site are, for example, a system of Cre recombinase and loxP, a system of Flp recombinase and FRT, a system of φC31 recombinase and φC31 attB and φC31 attP, or the like, as described above. The site-directed recombinase (e.g., Cre) is capable of catalyzing site-directed recombination reaction between recognition site sequences (e.g., between loxP sequences).

### <Step 3>

A synthetic polynucleotide of the D-region is inserted into the acceptor site by a method such as a site-directed recombination technique or a genome editing technique.

The synthetic polynucleotide of the D-region is a modified D-region, as described in the section 1 above, in which a human-derived genomic sequence from D1-1 to D1-26 of a D-region of the human immunoglobulin heavy chain locus is substituted with a modified sequence of the D-region having a combination of the following (1) and (2) or (1) and (3), wherein the modified sequence being a sequence modified so as to comprise:
(1) sequences in which inter-open reading frame (ORF) human-derived genomic sequences from D1-1 to D1-26 of the D-region of the human immunoglobulin heavy chain locus or the inter-ORF human-derived genomic sequences except for the one between D3-9 and D3-10 are truncated to the length of 49 bp or more from the end of each VDJ recombination sequence of an inter-ORF region flanked by VDJ recombination sequences; and
(2) ORF sequences from B1-1 to B9-4 of a D-region of a bovine-derived immunoglobulin heavy chain locus, ORF sequences from 1S5 to 1S35 of a D-region of a monkey-derived immunoglobulin heavy chain locus, or ORF sequences of a D-region of a vertebrate-derived (e.g., sheep-, horse-, rabbit-, bird-, or shark-derived) immunoglobulin heavy chain locus, instead of ORF sequences from D1-1 to D1-26 of the D-region of the human immunoglobulin heavy chain locus; or
(3) 26 types of modified ORF sequences from D1-1 to D1-26 of the D-region of the human immunoglobulin heavy chain locus produced on the basis of a human antibody heavy chain CDR3 sequence of 18 or more amino acids, instead of ORF sequences from D1-1 to D1-26 of the D-region of the human immunoglobulin heavy chain locus.

In one embodiment, a method for substituting a D-region of a human immunoglobulin heavy chain with a modified sequence thereof (which has the same meaning as that of the "synthetic polynucleotide of the D-region") in the production of the mammalian artificial chromosome vector described above may comprise the following steps:
(1') providing a mammalian artificial chromosome vector comprising human immunoglobulin heavy chain and light chain loci;
(2') deleting the D-region of the human immunoglobulin heavy chain from the mammalian artificial chromosome vector of the step (1');
(3') inserting a cassette comprising a first site-directed recombinase recognition site, a promoter, and a second site-directed recombinase recognition site into a deletion site of the D-region in the vector obtained in the step (2');
(4') inserting a cassette comprising the modified sequence of the D-region of the human immunoglobulin heavy chain, the first site-directed recombinase recognition site, a selection marker, and the second site-directed recombinase recognition site into the second site-directed recombinase recognition site of the cassette inserted in the step (3') using the second site-directed recombinase; and
(5') obtaining the artificial chromosome vector in which the cassette comprising the first site-directed recombinase recognition site, the modified sequence of the D-region of the human immunoglobulin heavy chain, and the second site-directed recombinase recognition site is inserted into the deletion site of the D-region by site-directed recombination using the first site-directed recombinase.

The modified sequence of the D-region of the human immunoglobulin heavy chain, the first site-directed recombinase recognition site, the second site-directed recombinase recognition site, the first site-directed recombinase, and the second site-directed recombinase in the steps are not limited, and for example, those described above may be used. The promoter is not limited either, and for example, CAG promoter may be used.

In the step (2'), for example, the technique described above, such as genome editing or site-directed recombination, may be used for deleting the D-region of the human immunoglobulin heavy chain from the mammalian artificial chromosome vector of the step (1').

Specifically, in order to insert the synthetic polynucleotide of the D-region into a cleavage site of the D-region of the human heavy chain in the Ig-NAC(ΔDH), a vector comprising the polynucleotide and a site-directed recombinase recognition site-containing cassette (e.g., the sequence of φC31-BsdR-oriC-R4) at its 5' end is produced, and the synthetic polynucleotide of the D-region may be inserted into the cleavage site of the D-region of the human heavy chain in the Ig-NAC(ΔDH) through site-directed recombination reaction between the vector and the Ig-NAC(ΔDH) (Figures 5, 6, 9, and 10).

Alternatively, in order to insert the synthetic polynucleotide of the D-region into a cleavage site of the D-region of the human heavy chain in the Ig-NAC(ΔDH), a vector comprising a site-directed recombinase recognition site between a partial sequence of the V-region of the human immunoglobulin heavy chain and a partial sequence of the J-region (and optionally, the C-region) of the human immunoglobulin heavy chain is produced, and the site-directed recombinase recognition site is inserted into the cleavage site of the D-region of the human heavy chain in the Ig-NAC(ΔDH) through genome editing (Cas9/gRNA) between this vector and the Ig-NAC(ΔDH). Further, another site-directed recombinase is allowed to act thereon to produce Ig-NAC comprising a site-directed recombinase recognition site in the cleavage site of the D-region of the human heavy chain in the Ig-NAC(ΔDH). Further, in order to insert the synthetic polynucleotide of the D-region into this Ig-NAC, a vector comprising the polynucleotide and a site-directed recombinase recognition site at its 5' end is produced, and the modified Ig-NAC of interest comprising the synthetic polynucleotide of the D-region at the cleavage site of the D-region of the human heavy chain in the Ig-NAC(ΔDH) may be produced by the action of site-directed recombinase (e.g., φC31 recombinase) and further by the action of another site-directed recombinase (e.g. Bxb1 recombinase or R4 recombinase) (Figure 10).

In another aspect, the present invention further provides a mammalian artificial chromosome vector comprising human immunoglobulin heavy chain and light chain loci, wherein a D-region is deleted from the human immunoglobulin heavy chain locus and the human immunoglobulin heavy chain locus comprises a first site-directed recombinase recognition site, a promoter, and a second site-directed recombinase recognition site instead of the deleted D-region.

The vector may further comprise a third site-directed recombinase recognition site.

An example of the mammalian artificial chromosome vector is the vector produced in the step (3') (e.g., Figure 9).

The deleted D-region is a region from D1-1 to D1-26 or a region from D1-1 to D7-27.

In the vector, the first site-directed recombinase recognition site and the second site-directed recombinase recognition site are not limited, and for example, those described above may be used. The promoter is not limited either, and for example, CAG promoter may be used.

### 2. Mammalian cell

In the second aspect, the present invention provides a mammalian cell comprising the mammalian artificial chromosome vector described in the section 1 above.

A human immunoglobulin locus in which a D-region of an antibody heavy chain is modified ("modified Ig-NAC" in the section 1) may be introduced to mammalian-derived cells via the mammalian artificial chromosome vector of the present invention to produce mammalian cells capable of producing a human antibody.

The mammalian artificial chromosome vector of the present invention may be transferred or introduced into any mammalian cell. Examples of the approach therefor include microcell mediated chromosome transfer (micronucleate cell fusion), lipofection, a calcium phosphate method, microinjection, and electroporation, preferably microcell mediated chromosome transfer.

The microcell mediated chromosome transfer technique is a method for transferring the mammalian artificial chromosome vector of the present invention into another desirable cell by micronucleate fusion between a cell (e.g., mouse A9 cell) capable of forming micronuclei and comprising the mammalian artificial chromosome vector and the desirable cell. The cell capable of forming micronucleate cells is treated with a polyploid inducer (e.g., colcemid or colchicine) to form multinucleated micronucleate cells, which may then be treated with cytochalasin to form micronucleate cells, followed by cell fusion of the microcells to the desirable cell.

Examples of the cell into which the above vector may be introduced include mammalian cells such as rodent cells and human cells, for example, germline cells (e.g., oocytes and spermatocytes), stem cells (e.g., embryonic stem (ES) cells, germline stem (GS) cells, somatic stem cells), somatic cells, embryonal cells, adult cells, normal cells, primary cultured cells, subcultured cells, and established cell lines. Examples of the stem cells include embryonic stem (ES) cells, embryonic germline (EG) cells, somatic stem cells (e.g., mesenchymal stem cells), induced pluripotent stem (iPS) cells, and nuclear transfer clone embryo-derived embryonic stem (ntES) cells. The preferred cells are selected from the group consisting of stem cells, precursor cells, somatic cells derived from mammals, for example, rodents (e.g., mice, rats, hamsters, (e.g., Chinese hamsters), and guinea pigs), primates (e.g., humans, monkeys, and chimpanzees), and ungulates (e.g., bovines, pigs, sheep, goats, horses, and camels), and non-human germline cells. When the cell is derived from a rodent, the vector of the present invention is more stably retained in the cell or tissue of the rodent into which the vector of the present invention has been introduced.

Examples of the somatic cells include, but are not limited to, hepatocytes, enterocytes, renal cells, splenocytes, lung cells, cardiac cells, skeletal muscle cells, brain cells, skin cells, bone marrow cells, and fibroblasts.

The ES cells are stem cells having semi-permanent proliferation potency and pluripotency (multipotency), that are established from an inner cell mass of a blastocyst of a fertilized egg derived from a mammal (M. J. Evans and M. H. Kaufman (1981) Nature 292: 154-156; J. A. Thomson et al. (1999) Science 282: 1145-1147; J. A. Thomson et al. (1995) Proc. Natl. Acad. Sci. USA 92: 7844-7848; J. A. Thomson et al. (1996) Biol. Reprod. 55: 254-259; J. A. Thomson and V. S. Marshall (1998) Curr. Top. Dev. Biol. 38: 133-165).

As with the case of mouse ES cells (M. J. Evans and M. H. Kaufman, Nature 1981; 292 (5819): 154-156), rat ES cells are established from the inner cell mass of the rat blastocyst stage embryo or 8-cell-stage embryo, and are pluripotent and self-reproducible cell lines. For example, rat blastocysts with egg zona pellucida dissolved are cultured on mouse embryonic fibroblast (MEFF) feeder using a medium containing leukemia inhibitory factor (LIF), the outgrowth formed from the blastocysts is dispersed 7 to 10 days later and is then transferred to MEF feeder and cultured on the feeder, so that the ES cells appear after 7 days. Preparation of rat ES cells is described in, for example, K. Kawaharada et al., World J. Stem Cells 2015; 7(7): 1054-1063.

An iPS cell colony may be generated in about 3 to 5 weeks by introducing specific reprogramming factors (DNAs or proteins) into a somatic cell (including a somatic stem cell) and subjecting the cell to culture and subculture in an appropriate medium. Examples of known combinations of reprogramming factors include a combination consisting of Oct3/4, Sox2, Klf4, and c-Myc; a combination consisting of Oct3/4, Sox2, and Klf4; a combination consisting of Oct4, Sox2, Nanog, and Lin28; and a combination consisting of Oct3/4, Sox2, Klf4, c-Myc, Nanog, and Lin28 (K. Takahashi and S. Yamanaka, Cell 126: 663-676 (2006); WO2007/069666; M. Nakagawa et al., Nat. Biotechnol. 26: 101-106 (2008); K. Takahashi et al., Cell 131: 861-872 (2007); J. Yu et al., Science 318: 1917-1920 (2007); J. Liao et al., Cell Res. 18, 600-603 (2008)). Examples of the culture include using a mitomycin C-treated mouse embryonic fibroblast cell line (e.g., STO) as a feeder cell, and culturing a somatic cell into which the vector has been introduced (e.g., approximately 10⁴ to 10⁵ cells/ cm²) at the temperature of about 37°C using an ES cell culture medium on the feeder cell layer. The feeder cell is not always necessary (Takahashi, K. et al., Cell 131, 861-872, 2007). Examples of the basic medium include Dulbecco's Modified Eagle's Medium (DMEM), Ham's F-12 medium, and a mixture thereof. As the ES cell culture medium, for example, a mouse ES cell culture medium or a primate ES cell culture medium (Reprocell Inc.) may be used.

### 3. Non-human animal

In the third aspect, the present invention provides a non-human animal comprising human immunoglobulin heavy chain and light chain loci, wherein a human-derived genomic sequence from D1-1 to D1-26 of a D-region of the human immunoglobulin heavy chain locus is substituted with a modified sequence of the D-region having a combination of the following (1) and (2) or (1) and (3), wherein the modified sequence of the D-region comprises:
(1) sequences in which inter-open reading frame (ORF) human-derived genomic sequences from D1-1 to D1-26 of the D-region of the human immunoglobulin heavy chain locus or the inter-ORF human-derived genomic sequences except for the one between D3-9 and D3-10 are truncated to the length of 49 bp or more from the end of each VDJ recombination sequence of an inter-ORF region flanked by VDJ recombination sequences; and
(2) ORF sequences from B1-1 to B9-4 of a D-region of a bovine-derived immunoglobulin heavy chain locus, ORF sequences from 1S5 to 1S35 of a D-region of a monkey-derived immunoglobulin heavy chain locus, or ORF sequences of a D-region of a vertebrate-derived (e.g., sheep-, horse-, rabbit-, bird-, or shark-derived) immunoglobulin heavy chain locus, instead of ORF sequences from D1-1 to D1-26 of the D-region of the human immunoglobulin heavy chain locus; or
(3) 26 types of modified ORF sequences from D1-1 to D1-26 of the D-region of the human immunoglobulin heavy chain locus produced on the basis of a human antibody heavy chain CDR3 sequence of 18 or more amino acids, instead of ORF sequences from D1-1 to D1-26 of the D-region of the human immunoglobulin heavy chain locus; and
wherein endogenous immunoglobulin heavy chain, κ light chain, and λ light chain genes or loci are disrupted.

In another aspect of the non-human animal, the present invention provides a non-human animal comprising the mammalian artificial chromosome vector described in the section 1, wherein endogenous immunoglobulin heavy chain, κ light chain, and λ light chain genes or loci are disrupted.

The term "non-human animal" used herein includes, but is not limited to, a mammal, for example, primates other than humans, such as monkeys and chimpanzees, rodents such as mice, rats, hamsters, and guinea pigs, and ungulates such as bovines, pigs, sheep, goats, horses, and camelids.

### [Production of non-human animal]

A non-human animal capable of producing a human immunoglobulin may be produced, for example, by introducing a human immunoglobulin locus in which a D-region of a human immunoglobulin heavy chain is modified ("modified Ig-NAC") to pluripotent cells (e.g., the ES cells or the iPS cells described above) or totipotent cells (e.g., germ cells, oocytes, and spermatocytes) by an approach such as a technique mediated by a mammalian artificial chromosome vector, a gene targeting technique, a genome editing technique, and/or a microinjection technique.

In such a non-human animal, endogenous loci corresponding to human immunoglobulin heavy chain and light chain κ and λ loci may be disrupted (or knocked out), and optionally, respective foreign DNAs corresponding to a human immunoglobulin heavy chain locus derived from human chromosome 14 in which a D-region of a human immunoglobulin heavy chain is modified, a human immunoglobulin light chain κ locus derived from human chromosome 2, and a human immunoglobulin light chain λ locus derived from human chromosome 22 may be inserted (or knocked in) into the disrupted endogenous loci on the genome of the non-human animal. For example, a technique such as gene targeting or genome editing may be used in a disruption method.

In the gene targeting technique, targeting vectors are constructed in order to disrupt the endogenous loci or in order to further substitute / insert (or knock in) the foreign DNAs instead of the endogenous loci. The vectors for disrupting the endogenous loci and inserting the foreign DNAs comprise a 5' upstream sequence (approximately 2.5 kb or more) and a 3' downstream sequence (approximately 2.5 kb or more) of each endogenous locus and may comprise, for example, a foreign drug resistance gene sequence or the foreign DNA sequence for substituting the endogenous locus to be disrupted between the sequences. The drug resistance gene is, for example, neomycin resistance gene or ampicillin resistance gene.

In the genome editing technique, as described above, a CRISPR/Cas9 system, for example, employs Cas9 protein and guide RNA (gRNA) having a target sequence of approximately 20 bp for their coexpression in cells, the gRNA thereby recognizes a PAM sequence proximal to the target sequence and specifically binds to target genomic DNA, and the Cas9 protein is capable of inducing a double-stranded cut upstream (on the 5' side) of the PAM sequence. By using this technique, non-human animal antibody heavy chain and light chain loci may be cleaved off, or the foreign DNAs may be further inserted (or knocked in) into the cleavage site using vectors comprising the respective foreign DNAs corresponding to a human immunoglobulin heavy chain locus derived from human chromosome 14 in which a D-region of a human immunoglobulin heavy chain is modified, a human immunoglobulin light chain κ locus derived from human chromosome 2, and a human immunoglobulin light chain λ locus derived from human chromosome 22.

The vectors comprising the foreign DNAs of interest as described above may be introduced into non-human animal-derived ES cells or iPS cells, so that the endogenous loci derived from the animal are disrupted and the DNA sequences of interest are inserted into the genome. The inserted foreign DNAs of interest may be confirmed by the identification of amplification products based on PCR using forward and reverse primers produced on the basis of the insert sequences. The ES cells or the iPS cells having the inserted foreign DNA sequences may be microinjected to an early embryo of a surrogate mother non-human animal to produce a chimeric non-human animal.

The non-human animal in which the endogenous loci have been disrupted may be produced, for example, by subjecting the chimeric non-human animal comprising a human immunoglobulin locus in which a D-region of a human immunoglobulin heavy chain is modified or an offspring thereof to crossbreeding with a chimeric animal or offspring thereof in which the entire cluster of corresponding endogenous genes is deleted, and further subjecting the resulting animals, in which the endogenous genes is heterozygously deleted, to crossbreeding.

An example of the non-human animal of the present invention is a non-human animal comprising the human immunoglobulin heavy chain locus derived from human chromosome 14 in which a D-region of a human immunoglobulin heavy chain is modified, and the human immunoglobulin light chain κ locus derived from human chromosome 2, or a non-human animal comprising the artificial chromosome vector described above; a non-human animal comprising the human immunoglobulin heavy chain locus derived from human chromosome 14 in which a D-region of a human immunoglobulin heavy chain is modified, and the human immunoglobulin light chain λ locus derived from human chromosome 22, or a non-human animal comprising the artificial chromosome vector described above; or a non-human animal comprising the human immunoglobulin heavy chain locus derived from human chromosome 14 in which a D-region of a human immunoglobulin heavy chain is modified, the human immunoglobulin light chain κ locus derived from human chromosome 2, and the human immunoglobulin light chain λ locus derived from human chromosome 22, or a non-human animal comprising the artificial chromosome vector described above.

The non-human animal is preferably a rodent (e.g., a mouse and a rat) comprising the mouse artificial chromosome vector.

Hereinafter, examples of the production of the non-human animal using a mouse artificial chromosome (MAC) vector will be described.

Each of an animal cell (e.g. a chicken B cell line DT40) comprising a human chromosome 2-derived human immunoglobulin light chain κ locus modified by the introduction of a site-directed recombinase recognition site (e.g., loxP or FRT) and an animal cell (e.g., DT40) comprising a human chromosome 22-derived human immunoglobulin light chain λ locus modified by the introduction of a site-directed recombinase recognition site (e.g., loxP or FRT) may be transferred into a rodent cell (e.g., a Chinese hamster ovary cell (CHO)) comprising the mouse artificial chromosome (MAC) vector by cell fusion, followed by the action of site-directed recombinase (e.g., Cre or Flp) or the induction of expression of the enzyme to produce a rodent cell comprising the MAC vector comprising the human immunoglobulin light chain κ locus and a rodent cell comprising the MAC vector comprising the human immunoglobulin light chain λ locus.

Meanwhile, a site-directed recombinase recognition site (e.g., loxP or FRT) is introduced to near a human immunoglobulin heavy chain locus on human chromosome 14 carried in an animal cell (e.g., DT40). Then, the animal cell comprising the human immunoglobulin heavy chain locus in which a D-region of a human immunoglobulin heavy chain is modified may be fused with a rodent cell (e.g., CHO) comprising the MAC vector by cell fusion to produce a rodent cell comprising the MAC vector comprising the human immunoglobulin heavy chain locus in which a D-region of a human immunoglobulin heavy chain is modified ("modified Ig-NAC" described above).

Each of the rodent cell comprising the MAC vector comprising the human immunoglobulin heavy chain locus in which a D-region of a human immunoglobulin heavy chain is modified and the human immunoglobulin light chain κ locus, and the rodent cell comprising the MAC vector comprising the human immunoglobulin heavy chain locus in which a D-region of a human immunoglobulin heavy chain is modified and the human chromosome 22-derived human immunoglobulin light chain λ gene or locus may be fused with a non-human animal (e.g., mouse or rat) pluripotent stem cell (e.g., ES cell or iPS cell) by the microcell mediated chromosome transfer technique to produce a non-human animal (e.g., mouse or rat) pluripotent stem cell comprising the MAC vector comprising the human chromosome 14-derived human immunoglobulin heavy chain locus in which a D-region of a human immunoglobulin heavy chain is modified and the human chromosome 2-derived human immunoglobulin light chain κ locus, and a non-human animal (e.g., mouse or rat) pluripotent stem cell comprising the MAC vector comprising the human chromosome 14-derived human immunoglobulin heavy chain locus in which a D-region of a human immunoglobulin heavy chain is modified and the human chromosome 22-derived human immunoglobulin light chain λ locus.

Each of the non-human animal (e.g., mouse or rat) pluripotent stem cell comprising the MAC vector comprising the human chromosome 14-derived human immunoglobulin heavy chain locus in which a D-region of a human immunoglobulin heavy chain is modified and the human chromosome 2-derived human immunoglobulin light chain κ locus, and the non-human animal (e.g., mouse or rat) pluripotent stem cell comprising the MAC vector comprising the human chromosome 14-derived human immunoglobulin heavy chain locus in which a D-region of a human immunoglobulin heavy chain is modified and the human chromosome 22-derived human immunoglobulin light chain λ locus is transferred into an early embryo of a B cell-deficient or -nondeficient non-human animal (e.g., 8-cell-stage embryo or blastocyst stage embryo) to produce chimeric animals comprising each of the MAC vectors. When the chimeric animals carry endogenous B cells, the chimeric animals are further subjected to crossbreeding to allogeneic non-human animals (e.g., mice or rats) deficient in mouse antibody heavy chain, light chain κ, and light chain λ loci (or deficient in B cells). Then, the non-human animal of interest may be selected to produce a non-human animal comprising the MAC vector comprising the human chromosome 14-derived human immunoglobulin heavy chain locus in which a D-region of a human immunoglobulin heavy chain is modified and the human chromosome 2-derived human immunoglobulin light chain κ locus, or the MAC vector comprising the human chromosome 14-derived human immunoglobulin heavy chain locus in which a D-region of a human immunoglobulin heavy chain is modified and the human chromosome 22-derived human immunoglobulin light chain λ locus.

Alternatively, these two types of different offsprings may be further subjected to crossbreeding with each other to produce a non-human animal (e.g., a mouse or a rat) comprising the MAC vector comprising the human immunoglobulin heavy chain locus in which a D-region of a human immunoglobulin heavy chain is modified, the human immunoglobulin light chain κ locus, and the human immunoglobulin light chain λ locus.

In the non-human animal produced by the method described above, it is preferred that all the endogenous antibody genes or loci corresponding to the human immunoglobulin heavy chain gene or locus and the human immunoglobulin light chain κ and λ genes or loci should be disrupted or knocked out. This enables the non-human animal to produce only a human antibody.

### 4. Method for producing human antibody

The present invention further provides a method for producing a human antibody, comprising producing a human antibody using the non-human animal comprising the mammalian artificial chromosome vector comprising human immunoglobulin heavy chain and light chain loci as described in the section 3, and collecting the human antibody.

The "human antibody" used herein may be of any class and subclass of human immunoglobulin (Ig). Examples of such a class include IgG, IgA, IgM, IgD, and IgE, and examples of the subclass include IgG1, IgG2, IgG3, IgG4, IgA1, and IgA2. These classes and subclasses may be classified according to differences in heavy chains, and IgG chains are referred to as γ chains, IgG1 to IgG4 chains are referred to as γ1, γ2, γ3, and γ4 chains, respectively, and IgA, IgM, IgD, and IgE chains are referred to as α chains (α1 and α2), µ chain, δ chain, and ε chain, respectively. It is known that each antibody light chain comprises a κ chain and a λ chain and that, when rearrangement of the κ chain gene is not successfully completed during rearrangement of the immunoglobulin gene, the λ chain gene is rearranged.

The human immunoglobulin heavy chain locus comprises, in the 5' to 3' direction, a V (variable) region gene comprising VH1, VH2..VHm (where m is, for example, 38 to 46); a D (diversity) region gene comprising DH1, DH2..DHn (where n is 23); a J (joining) region gene comprising JH1, JH2..JHr (where r is 6); and a C (constant) region gene comprising Cµ, Cδ, Cγ3, Cγ1, Cα1, Cγ2, Cγ4, Cε, and Cα2. In the present invention, the D-region gene of the human immunoglobulin heavy chain locus comprises substitution with the synthetic nucleotide sequence of the D-region described as the modified sequence having the combination of (1) and (2) or (1) and (3) in the section 1.

In the non-human animal, for example, a human antibody comprising ultralong CDRH3 having 18 to 50 or more amino acids, which is difficult to produce constantly in conventional human antibodies, can be produced as an antibody produced via the rearrangement of the human immunoglobulin genes described above. The human antibody comprising such ultralong CDRH3 has low frequency of appearance in humans and is therefore difficult to produce by usual monoclonal antibody production.

A human antibody molecule has a structure composed of 2 human antibody heavy chains and 2 human antibody light chains, wherein each heavy chain is bound to each light chain by 2 disulfide bonds, and 2 heavy chains are bound to each other by 2 disulfide bonds at constant (C) regions. In each of heavy chain and light chain variable (V) regions of an antibody molecule, there are 3 hypervariable regions, called complementarity-determining regions (CDRs), with particularly high degrees of mutations. The hypervariable regions of the heavy chain variable region are referred to as CDRH1, CDRH2, and CDRH3 from the N terminus, while the hypervariable regions of the light chain variable region are referred to as CDRL1, CDRL2, and CDRL3 from the N terminus. Antibody-antigen binding properties vary depending on differences in sequences of the CDR regions. The present invention expands a human antibody repertoire based on the rearrangement of human immunoglobulin genes.

The human antibody comprising ultralong CDRH3, which is produced by the non-human animal of the present invention, has a structure composed of a long (β-ribbon) stalk and a knob, and the knob has a loop and a plurality of disulfide bonds (J. Dong et al., Frontiers in Immunology, 2019; 10: Article 558; J. K. Haakenson et al., Frontiers in Immunology 2018; 9: Article1 262).

The antibody that may be produced by the method of the present invention is a human antibody comprising ultralong antibody heavy chain CDR3 (CDRH3) such that an amino acid length of CDRH3 is 18 or more, 20 or more, 25 or more, 30 or more, 40 or more, or 50 or more and is therefore considered effective for diseases including infectious diseases caused by, for example, pathogenic viruses such as human immunodeficiency virus (HIV), influenza virus, or coronavirus (e.g., SARS-CoV and MERS-CoV), RS (respiratory syncytial) virus, or other pathogens (e.g., malarial parasite and trypanosoma protozoa). The human antibody permits "key" type interaction through the binding of the protruding CDRH3 region to the recess of a receptor of the virus or the pathogen and is therefore therapeutically useful.

The antibody of the present invention may be used as a cocktail of a plural types of antibodies and may be used for potentiating not only a therapeutic effect but a prophylactic effect.

Specific antibody production encompasses the following methods.

For example, the human antibody-producing non-human animal (e.g., mouse or rat) of the present invention may be immunized with a target antigen such as HIV envelope protein to produce a polyclonal antibody specifically binding to the target antigen.

Alternatively, the animal is immunized with a target antigen, and B lymphocytes of an animal individual having an elevated antigen-specific antibody titer are fused with myeloma cells to produce hybridomas (immortalized B cells). Human monoclonal antibodies in supernatants of the hybridomas may be screened by ELISA analysis for evaluating the ability to bind to the antigen and optionally, by the evaluation of infection with HIV or the like to select a human monoclonal antibody as a broadly neutralizing antibody.

Specifically, in the fourth aspect, the present invention provides a method for producing an antibody, comprising immunizing the non-human animal described in the section 3 with a target antigen, and obtaining an antibody binding to the target antigen from blood of the non-human animal.

In an alternative aspect, the present invention further provides a method for producing an antibody, comprising immunizing the non-human animal described in the section 3 with a target antigen, obtaining splenocytes or lymph node cells from the non-human animal producing an antibody binding to the target antigen, fusing the splenocytes or the lymph node cells with myeloma cells to form hybridomas, and culturing the hybridomas to obtain a monoclonal antibody binding to the target antigen.

In a further alternative aspect, the present invention further provides a method for producing an antibody, comprising immunizing the non-human animal described in the section 3 with a target antigen, obtaining B cells from the non-human animal producing an antibody binding to the target antigen, obtaining nucleic acids encoding proteins of an antibody heavy chain and light chain or their variable regions from the B cells, and producing an antibody (e.g., a recombinant antibody, a monoclonal antibody, and single-chain Fv (scFv)) binding to the target antigen by a DNA recombination technique or a phage display technique using the nucleic acid.

The nucleic acid comprises DNA (including cDNA) or RNA (including mRNA).

The scFv is a recombinant antibody fragment produced by linking a polypeptide of an antibody heavy chain variable region to a polypeptide of an antibody light chain variable region via a linker.

In the phage display technique, human antibody variable regions are expressed as single-chain Fv (scFv) or Fab on phage surface, and a phage that binds to an antigen is selected (Nature Biotechnology, 2005; 23 (9): 1105-1116). The phage selected by binding to the antigen may be analyzed for its genes to determine DNA sequences encoding the variable regions of the human antibody that binds to the antigen. If the DNA sequence of the scFv that binds to the antigen is determined, the human antibody may be obtained by producing an expression vector having the sequence, and introducing the expression vector to an appropriate host so as to express the antibody (WO92/01047, WO92/20791, WO93/06213, WO93/11236, WO93/19172, WO95/01438, WO95/15388, Annu. Rev. Immunol, 1994; 12: 433-455, Nature Biotechnology, 2005; 23(9): 1105-1116).

The antibody produced by any of these methods may be collected and purified by an approach such as affinity chromatography comprising: applying blood (antiserum), cell supernatant, or the like containing the antibody to a column packed with a carrier bound to the antigen substance or an immunoglobulin G binding carrier (e.g., an agarose gel and a silica gel); and subsequently eluting the human antibody bound to the support from the support.

### Examples

The present invention will be described further specifically with reference to the following examples, although the technical scope of the present invention is not limited to these examples.

### [Example 1] Construction of Ig-NAC(ΔDH) by deletion of human IgHD region

In order to construct Ig-NAC(ΔDH) by the deletion of a human IgHD region, gRNAs of CRISPR/Cas9 were designed, evaluated, and selected for 2 sites (upstream and downstream) flanking the DH region. The DH region was removed by cleavage at the 2 sites within CHO cells that retained Ig-NAC. Ig-NAC(ΔDH)-retaining CHO cells were obtained by screening.

### 1. Construction of gRNA expression vector and selection by activity evaluation

Plasmids expressing candidate gRNAs were designed and constructed on the basis of an all-in-one vector eSpCas9(1.1) (Addgene Plasmid #71814) expressing Cas9 and gRNA. The all-in-one vector was introduced to CHO cells that retained Ig-NAC using Lipofectamine LTX (Invitrogen) according to manufacturer's protocol. DNA was extracted from the transfected cells using PureGene kit (Qiagen N.V.). Cleaving activity was confirmed using Cel-1 assay kit (IDT), and vectors of highly active gRNA sequences shown below were selected.
gRNA Up3: 5'-AGATCCTCCATGCGTGCTGT (GGG)-3' (SEQ ID NO: 4)
   (where the gRNA sequence is a 20-base sequence excluding a PAM sequence (GGG).)
gRNA Down4: 5'-CTGCGGCATGAACCCAATGC (AGG)-3' (SEQ ID NO: 5)
   (where the gRNA sequence is a 20-base sequence excluding a PAM sequence (AGG).)

### 2. Obtainment of clone from which DH region was deleted

The DH region was deleted by each combination of gRNA Up3 (in the figure, "5'guide3") and gRNA Down4 (in the figure, "5'guide4") (Figure 1). The all-in-one vector for each gRNA and a CMV-tdTomato expression vector were introduced to Ig-NAC-retaining CHO cells (JP Patent No. 6868250) using Lipofectamine LTX (Invitrogen) according to manufacturer's protocol, and 3 days later, EGFP/tdTomato-copositive cells were obtained by sorting. The signal of EGFP indicates that Ig-NAC was retained, and the signal of tdTomato indicates that lipofection occurred. The copositive cells were further cultured, and 8 days after the first sorting, EGFP-positive and tdTomato-negative cells were collected into a 384-well plate by single-cell sorting. Clones that proliferated were further scaled up and screened as described below.

### 3. Screening of clone from which DH region was deleted

The cells obtained as described above were screened by PCR using the following primer for junction confirmation and primer for DH region deletion confirmation, and using DNA, which was extracted using PureGene kit (Qiagen N.Y.), as a template.
Junction primer F1: 5'-TCCCACGGCCCAAGGAAGACAAGACACA-3' (SEQ ID NO: 6)
Junction primer R1: 5'-TCGAACACGCTCCTAGCATTGCACAGCC-3' (SEQ ID NO: 7)
Deletion confirming primer F1: 5'-ACACCTGTCTCCGGGTTGTG-3' (SEQ ID NO: 8)
Deletion confirming primer R1: 5'-AAGAAACGCAGGACGGTGGA-3' (SEQ ID NO: 9)
Deletion confirming primer F2: 5'-CAGCAGCCACTCTGATCCCA-3' (SEQ ID NO: 10)
Deletion confirming primer R2: 5'-CTGGTGGACTCTACGGCGAA-3' (SEQ ID NO: 11)
Deletion confirming primer F3: 5'-GGGACCCTCAAGGTGTGAAC-3' (SEQ ID NO: 12)
Deletion confirming primer R3: 5'-AGGGTGCTTGGGTCCTGTTA-3' (SEQ ID NO: 13)
Deletion confirming primer F4: 5'-ACAAGCCAGGGAGCTGTTTC-3' (SEQ ID NO: 14)
Deletion confirming primer R4: 5'-TCAAGAAATCCGAGGCGACA-3' (SEQ ID NO: 15)

The enzyme used was KOD FX (Toyobo Co., Ltd.), and reaction was performed for 35 cycles with conditions of 98°C for 15 seconds, 60°C for 30 seconds, and 68°C for 90 seconds. The removal of the D-region was confirmed in 35 clones out of a total of 381 clones obtained for all the combinations. Then, the presence of antibody gene regions other than DH on Ig-NAC was confirmed by PCR according to the method described in JP Patent No. 6868250) to select candidate Ig-NAC(ΔDH) clones.

### 4. Structural confirmation of Ig-NAC(ΔDH)

In order to confirm that Ig-NAC(ΔDH) was independently maintained without being integrated into host chromosomes and that Ig-NAC(ΔDH) retained an expected structure, FISH analysis was conducted according to the method described in JP Patent No. 6868250. As a result, the obtainment of a CHO cell retaining one copy of Ig-NAC(ΔDH) independently of host chromosomes was confirmed (Figure 2), and this cell clone was designated as TF7-B10.

### [Example 2] Insertion of acceptor site for introducing synthetic D-region into Ig-NAC(ΔDH)

An acceptor site is inserted into the deleted DH region in the Ig-NAC(ΔDH) produced in Example 1 by using genome editing and homologous recombination, and thereby permits introduction of a chemically synthesized DH region. For this purpose, a HDR vector in which sequences before and after (V-region side: HRA, J-fragment side: HRB) the deletion site of the DH region in the Ig-NAC(ΔDH), Bxb1 attB, Bxb1 attP, ΦC31 attB, and R4 attB (JP Patent No. 6868250) (their respective sequences were as described below), CAG promoter (pRP[Exp]-CAG>mCherry, synthesized at VectorBuilder Inc.), blasticidin resistance gene (FUJIFII,M Wako Pure Chemical Corp.), and Fcy::fur gene (pSELECT-zeo-Fcy::fur, InvivoGen) were located as shown in Figure 4 was produced by procedures described below.

HRA: (SEQ ID NO: 16) 
GACCTGAGAGCCGCTCCCTGAAGTGTCCCCATTGGGAAGGATGGGGCCTGTGTCT
CCAGGCTCTGGGAGGACAGAATCCTGACCTCAACAGTGGCCGGCACGGACACAA
CTGGCCCCATCCCGGGGACGCTGACCAGCGCTGGGCAACTTTTCCCTTCCCCGAC
GACTGAGCCCCGAGCACCCTCCCTGCTCCCCTACCACCTCCCTTTACAAGGCTGTG
GCCTCTGCACAGATGATAATGGAGCTTGGCTCATTCCCCTAGAGTCGGTAGGGAG
TTAAGGACAAAACTCAGTTTCCTCCACCTGAACTCAAGTCTGCCTATGTTTACCTA
ATCACACCTGGTGGACAGTTTGGACAAACTTGCACACTCAGAGACACAGACACTT
CTAGAAATCATTATCTCCCTGCCCCGGGGACCCCACTCCAGCAGAAGTCTGCTAG
GCACTGGCCTGGGCCCTCCTGCTGTCCTAGGAGGCTGCTGACCTCCTGCCTGGCTC
CTGTCCCCAGGTCCAGAGTCAGAGCAGACTCCAGGGACGCTGCAGGCTAGGAAG
CCGCCCCCTCCAGGCGAGGGTCTAGTGCAGGTGCCCAGGACAAGAAAGATTGTG
AATGCAGGAATGACTGGGCCACACCCCTCCCGTGCACGCCCCCTCCTGCCCTGCA
CCCCACAGCCCAGCCCCCCGTGCTGGATGCCCCCCCACAGCAGAGGTGCTGTTCT
GTGATCCCCTGGGAAAGACGCCCTCAACCTCCACCCTGTCCCACGGCCCAAGGAA
GACAAGACACAGGCCCTCTCCTCACAGTCTCCCCACCTGGCTCCTGCTGGGACCC
TCAAGGTGTGAACAGGGAGGATGGTTGTCTGGGTGGCCCCTAGGAGCCCAGATCT
TCACTCCACAGACCCCAACCCAAGCACCCCCTTCTGCAGGGCCCAGCTCATCCCC
CTCCTCCTCCCTCTGCTCTCCTCT
HRB: (SEQ ID NO: 17) 
CTCACTGAAGGCAGCCTCAGGGTGCCCAGGGGCAGGCAGGGTGGGGGTGAGGCT
TCCAGCTCCAACCGCTCCACTAGCCGAGACTAAGGAAGTGAGAGGCAGCCAGAA
 
ATCCAGACCATTCCATAGCAAATGGATTTCATTAAAGTTACCAGACTTCAGTGTA
AGTAACATGAGCCCCATGCACAACAATCCCTTATGAAGGGGAAGTCAGTGTCGCC
TCGGATTTCTTGAAAAACACAAAAACTTATCAATGCCTGTAAAAGTCTGTTGGAA
AGAAAATATGATTCAAGAATGTTATGCCCAACAAAGCTGGCATATTTTCTACCCG
GACACACTCAGGGAATGTGGTCCCTTGAGTGCTTCTCTCACTGCGTAAATCCTACG
TGGTGTTTAAGCATATTCATAAATGTGTATGTCTATTTTTATGTGTAAGATGGTTC
ATTTTTATTTTATTTATTCAATATGTACAATAAAGAATATTGACAAATAGGCTGGA
CATGGTGGCTCCCACCTGTAATCCCAGCCCTTTGGGAGGCCGAGGCGGGCAGATC
ACCTGAGGTCTGGAGTTCGAGACCAGCCTGGCCAACATGATGAAAACCCATCTCT
ACTAAAAATACAAAGATTAGCCAGGCATGGTGGTGCATGCCTGTAATCCCAGCCA
CTCAGGAGGCTGAGACAGGAGAAATGCGTGAACCCGGAAGGCGGAGGTTGCAGT
GAGCCGAGATCACACCACTGCACTCCAGCCTGGCGACAGAGCAAGATTCCATCTC
AAAAAAAAAAAGACAAAGAAATTTGTTTTTTTGAATAAAGACAAATTTCATCACA
CGAAGATAAAGATGCAAAGCTCCAGACAGGAAGGCACGGACAGCACAGTGAAGC
CCGGAGCGGGCGCTGGGGGGCCAGGGGCATGGCGGGGGTGCCAGCGTCTCTCGG
TGCCTACCATGGCCACTCCAGCCTGTGTTCTCACGAGGATGGCTGTGT
Bxb1 attB: (SEQ ID NO: 18) 
TGGCCGTGGCCGTGCTCGTCCTCGTCGGCCGGCTTGTCGACGACGGCGGTCTCCGT
CGTCAGGATCATCCGGGCCAC
Bxb1 attP: (SEQ ID NO: 19)
ΦC31 attB: (SEQ ID NO: 20) 
TATGGCCGTGATGACCTGTGTCTTCGTGGTTTGTCTGGTCAACCACCGCGGTCTCA
GTGGTGTACGGTACAAACCCA
 
GATGTAGGTCACGGTCTCGAAGCCGCGGTGCGGGTGCCAGGGCGTGCCCTTGGGC
TCCCCGGGCGCGTACTCCACCTCACCCATCTGGTCCATCATGATGAACGGGTCGA
GGTGGCGGTAGTTGATCCCGGCGAACGCGCGGCGCACCGGGAAGCCCTCGCCCTC
 
GAAACCGCTGGGCGCGGTGGTCACGGTGAGCACGGGACGTGCGACGGCGTCGGC
GGGTGCGGATACGCGGGGCAGCGTCAGCGGGTTCTCGACGGTCACGGCGGGCAT
R4 attB: (SEQ ID NO: 21)

GCGCCCAAGTTGCCCATGACCATGCCGAAGCAGTGGTAGAAGGGCACCGGCAGA
CAC

### 1. Insertion of Fcy::fur gene into CAG promoter-containing vector

Negative selection marker Fcy::fur gene was inserted in order to selectively kill cells that underwent insertion by random integration other than the homologous recombination of interest when a HDR vector was inserted into Ig-NAC(ΔDH)-retaining CHO cells. The Fcy::fur gene was amplified by PCR from pSelect-zeo-Fcy::fur plasmid (InvivoGen) DNA using the following primers and conditions.
Fcy-fur-F1: 5'-CATGAATCTAGAAGTGCAGGTGCCAGAACATT-3' (SEQ ID NO: 22)
fur-BspH1-R1: 5'-CATGAATCATGACCCCCTGAACCTGAAACATA-3' (SEQ ID NO: 23)

PCR was performed with 100 ng/µL pSelect-zeo-Fcy::fur plasmid DNA (0.1 µL) as a template using TaqDNA polymerase KODone (Toyobo Co., Ltd.) under reaction conditions of 35 cycles with 98°C for 10 seconds, 60°C for 5 seconds, and 68°C for 13 seconds.

The Fcy::fur gene after PCR was subjected to agarose gel electrophoresis to purify a DNA fragment, and then both ends were cleaved with restriction enzymes BspHI (NEB) and XbaI (NEB). The Fcy::fur gene was inserted into a restriction enzyme AfiIII (NEB) and XbaI (NEB) cleavage site in CAG promoter-containing plasmid DNA pRP[Exp]-CAG>mCherry (synthesized at VectorBuilder Inc.) to obtain a plasmid CAG-Fcy::fur (Figure 3).

### 2. Production of vector plasmid CAG-ΦC31-HRB-Fcy::fur in which ΦC31 attB and HRB were inserted between CAG promoter and Fcy::fur gene

pHRB, plasmid DNA containing ΦC31 attB and HRB, was synthesized at Eurofins Genomics K.K. and used for introducing chemically synthesized DNA to the plasmid CAG-Fcy::fur produced in the above 1. This plasmid was cleaved with a restriction enzyme XbaI. The plasmid CAG-Fcy::fur was cleaved with the restriction enzyme XbaI, and a fragment derived from the pHRB cleaved at both ends with XbaI was inserted between the CAG promoter and the Fcy::fur gene to obtain a plasmid CAG-ΦC31-HRB-Fcy::fur (Figure 3).

### 3. Production of HDR vector plasmid

Plasmid DNA pHRA was synthesized at Eurofins Genomics K.K. and used for introducing HRA, site-directed recombinase sites R4 attB, Bxb1 attB, and Bxb1 attP, and blasticidin resistance gene to the plasmid CAG-ΦC31-HRB-Fcy::fur produced in the above 2. This plasmid was cleaved with restriction enzymes NotI and restriction enzyme SpeI. The plasmid CAG-ΦC31-HRB-Fcy::fur was cleaved with the restriction enzymes NotI and restriction enzyme SpeI, and a 2437-bp fragment derived from the plasmid HRA was inserted upstream side of CAG to obtain an HDR vector (Figure 4).

### 4. Cleavage of Ig-NAC(ΔDH) by CRISPR/Cas9

CRISPR/Cas9 is capable of causing efficient homologous recombination by two double-stranded cuts of DNA at a target site. Guide RNAs, gRNA-A1 and gRNA-B1, were designed for two sites (target) near the deletion site of the DH region on Ig-NAC(ΔDH).
gRNA-A1: GGGCCCAGGCGCCGTTTAAT AGG (SEQ ID NO: 24)
   (where the gRNA sequence is a 20-base sequence excluding a PAM sequence (AGG).)
gRNA-B1: TGCCGCAGAGTGCCAGGTGC AGG (SEQ ID NO: 25)
   (where the gRNA sequence is a 20-base sequence excluding a PAM sequence (AGG).)

NGG recognizable by Cas9 was removed from these gRNAs, and a restriction enzyme BbsI recognition sequence was added thereto to respectively synthesize oligo DNAs (Eurofins Genomics K.K.). These oligo DNAs were further annealed and then cleaved with a restriction enzyme BbsI (NEB), and the resulting plasmid DNAs were each cloned into a Cas9 expression vector (px330, Addgene Plasmid #42230) cleaved with the restriction enzyme BbsI. The obtained *E. coli* colonies were subjected to PCR using primers and conditions described below to confirm the presence of the inserted oligo DNAs. The forward primer used was U6F. The reverse primers used were gRNA-A1R for gRNA-A1 confirmation and gRNA-B1R for gRNA-B 1 confirmation.
U6F: GAGGGCCTATTTCCCATGATTCC (SEQ ID NO: 26)
gRNA-A1F: CACCGGGGCCCAGGCGCCGTTTAAT (SEQ ID NO: 27)
gRNA-A1R: AAACATTAAACGGCGCCTGGGCCCC (SEQ ID NO: 28)
gRNA-B 1F: CACCGTGCCGCAGAGTGCCAGGTGC (SEQ ID NO: 29)
gRNA-B1R: AAACGCACCTGGCACTCTGCGGCAC (SEQ ID NO: 30)

PCR was performed using TaqDNA polymerase (EmeraldAmp PCR Master Mix, Takara Bio Inc.) under reaction conditions of 1 cycle of 98°C for 2 minutes followed by 35 cycles with 98°C for 10 seconds, 60°C for seconds, and 68°C for 13 seconds.

Colonies for which the band of interest was obtained at or around 330 bp by 2% agarose gel electrophoresis after PCR were cultured in liquid LB medium and 100 µL/mL ampicillin, and plasmid DNAs (Cas9-gRNA expression vectors A1 and B1) were obtained using (Nucleospin Plasmid Transfection-grade, Takara Bio Inc.).

### 5. Insertion of HDR vector into Ig-NAC(ΔDH)

The HDR vector was inserted near the deletion site of the DH region in Ig-NAC(ΔDH) in CHO cells by genome editing using Cas9/gRNA (Figure 5). The HDR vector and the Cas9-gRNA expression vectors A1 and B1 were introduced by electroporation to the Ig-NAC(ΔDH)-retaining CHO cell clone TF7B10 produced in Example 1. The CHO cells TF7B 10 were treated with trypsin and suspended at 1 × 10⁷ cells/mL in Opti MEM (Thermo Fisher Scientific Inc.), followed by electroporation at 150 V and 7.5 ms using NEPA21 (Nepa Gene Co., Ltd.) in the presence of 5 µg of the HDR vector, 2.5 µg of gRNA-A1, and 2.5 µg of gRNA-B1. The cells after electroporation were inoculated to three 96-well plates and 48 to 72 hours later, cultured in F12 medium containing 800 µg/mL G418 (FUJIFILM Wako Pure Chemical Corp.), 1600 µg/mL blasticidin (FUJIFILM Wako Pure Chemical Corp.), and 10% FBS (Sigma). 84 to 96 hours after the start of culture, the medium was replaced with F12 medium containing 1600 µg/mL blasticidin, 200 µM 5FC (Tokyo Chemical Industry Co., Ltd.), and 10% FBS to obtain drug-resistant clones.

### 6. PCR analysis

The isolated drug-resistant clones were cultured, and genomic DNA was extracted from the cells using Cellysis (Qiagen N.V.). One clone (TF7B10-74) for which a homologous recombination-specific band was obtained was selected by PCR analysis with the genomic DNA as a template using three homologous recombination-specific primers (described below) and 12 Ig-NAC-specific primers (JP Patent No. 6868250) produced for Ig-NAC. Approximately 0.1 µg of the genomic DNA was used in PCR amplification. The Taq polymerase used was KODone (Toyobo Co., Ltd.), and PCR was performed by 1 cycle of 98°C for 2 minutes followed by 35 cycles with denaturation at 98°C for 10 seconds, annealing at 60°C for 5 seconds, and extension at 68°C for: 5 seconds in the case of an amplification fragment having a length of 500 bp or less; and 100 seconds/kb for a length of 500 bp or more.
HRAprimerF1: 5'-GAACTGACCGTCTGCTCCA-3' (SEQ ID NO: 31)
Bxb1attBF1: 5'-TGCCGAAGCAGTGGTAGAAG-3' (SEQ ID NO: 32)
ΦC31F1: 5'-GGGCAACGTGCTGGTTATTG-3' (SEQ ID NO: 33)
HRAprimerR1: 5'-GTGCCCTTCTACCACTGCTTC-3' (SEQ ID NO: 34)
Bxb1attPR1: 5'-AGAGTGAAGCAGAACGTGGG-3' (SEQ ID NO: 35)
HRBR1: 5'-TGCTTTTTCGCAACCATGGG-3' (SEQ ID NO: 36)

Table 1 shows a clone name in the row and the primers and the extension times used for PCR in the columns. The circle represents positive.

**[Table 1]**

| Primer name | Extension time | Clone 74 |
|---|---|---|
| primer-HRAF1R1 | 11 sec | ○ |
| ΦC31F1-HRBR1 | 19 sec | ○ |
| attBF1-attPR1 | 19 sec | ○ |
| EIF2AK3-F/R | 5 sec | ○ |
| RPIA-F/R | 5 sec | ○ |
| IGKC-F/R | 5 sec | ○ |
| IGKV-F/R | 5 sec | ○ |
| Vk3-2 F/R | 5 sec | ○ |
| ELK2 P2-F/R | 5 sec | ○ |
| VH3-F/R | 5 sec | ○ |
| MTA1-F3/R3 | 5 sec | ○ |
| g1(g2)-F/R | 5 sec | ○ |
| CH3F3/CH4R2 | 5 sec | ○ |
| TRANS L1/R1 | 5 sec | ○ |
| KJneo/PGKr-2 | 5 sec | ○ |

### [Example 3] Removal of blasticidin resistance gene by site-directed recombination using Bxb1 recombinase

When chemically synthesized DNA is introduced to Ig-NAC(ΔDH), in order to confirm whether the DNA is introduced at a site of interest by the presence of blasticidin resistance, it was necessary to remove the inserted blasticidin resistance gene by homologous recombination. As shown in the HDR vector, the blasticidin resistance gene is positioned between Bxb1 attB and Bxb1 attP, and the introduction of Bxb1 recombinase into cells enables removal of the blasticidin resistance gene through site-directed recombination reaction between the Bxb1 attB and the Bxb1 attP (Figure 6).

### 1. Removal of blasticidin resistance gene by introduction of Bxb1 recombinase gene

The method was to introducing Bxb1 recombinase gene expression vector (JP Patent No. 6868250) was introduced by electroporation to the Ig-NAC(ΔDH)-retaining CHO cells TF7B10-74 having the inserted acceptor site obtained in Example 2. The TF7B10-74 cells were treated with trypsin and suspended at 2 × 10⁶ cells/mL in Opti MEM, followed by the addition of 10 µg of the Bxb1 recombinase gene expression vector and electroporation at 150 V and 7.5 ms using NEPA21 (Nepa Gene Co., Ltd.). The cells after electroporation were inoculated to one 10 cm dish. 5 days later, the cells were treated with trypsin and inoculated at 150 cells per 384-well plate. The cells thus inoculated to a 384-well plate were cultured in F12 medium containing 800 µg/mL G418 and 10% FBS for 9 days, and single clones were picked up.

### 2. PCR analysis

The isolated cells were cultured, and genomic DNA was extracted from the cells using Cellysis (Qiagen N.V.). PCR analysis was conducted with the genomic DNA as a template using two Bxb1 site-directed recombination-specific primers and 12 Ig-NAC-specific primers (JP Patent No. 6868250) produced for Ig-NAC. With primerHRAF1-Bxb1attPR2 among the two Bxb1 site-directed recombination-specific primers, a band shifts from (around) 2800 bp to (around) 1500 bp after site-directed recombination. Two clones (clones D1 and J23) were selected for which a Bxb1 site-directed recombination-specific band was obtained. Approximately 0.1 µg of the genomic DNA was used in PCR amplification. The Taq polymerase used was KODone (Toyobo Co., Ltd.), and PCR was performed by 1 cycle of 98°C for 2 minutes followed by 35 cycles with denaturation at 98°C for 10 seconds, annealing at 60°C for 5 seconds, and extension at 68°C for: 5 seconds in the case of an amplification fragment having a length of 500 bp or less; and 100 seconds/kb for a length of 500 bp or more.
HRAprimerF1: 5'-GAACTGACCGTCTGCTCCA-3' (SEQ ID NO: 31)
Bxb1attPR2: 5'-GGGGCGTACTTGGCATATGA-3' (SEQ ID NO: 37)

### 3. Confirmation of blasticidin resistance

The two clones (clones D1 and J23) for which a Bxb1 site-directed recombination-specific band was obtained were cultured in F12 medium containing 800 µg/mL G418, 1600 µg/mL blasticidin, and 10%FBS to confirm the presence of blasticidin resistance. One clone (clone D1: TF7B10-74-D1) out of the two clones was killed in the presence of blasticidin.

### 4. Fluorescence in-situ hybridization (FISH)

FISH analysis was conducted according to the method described in (JP Patent No. 6868250) using a human gene-specific probe, human cot1 (Invitrogen), and a mouse gene-specific probe, mouse cot1 (Invitrogen).

Table 2 shows results of the PCR analysis, the confirmation of blasticidin resistance, and the FISH analysis mentioned above. Table 2 shows clone names in the rows and the primers and the extension times used for PCR in the columns. The circle represents Bxb1 recombination-positive, and the X-mark represents Bxb1 recombination-negative.

**[Table 2]**

| Primer name | Extension time | Clone D1 | Clone J23 |
|---|---|---|---|
| primerHRAF1-attPR2 | 20 sec | ○ | × |
| ΦC31F1-HRBR1 | 19 sec | ○ | ○ |
| EIF2AK3-F/R | 5 sec | ○ | ○ |
| RPIA-F/R | 5 sec | ○ | ○ |
| IGKC-F/R | 5 sec | ○ | ○ |
| IGKV-F/R | 5 sec | ○ | ○ |
| Vk3-2 F/R | 5 sec | ○ | ○ |
| ELK2P2-F/R | 5 sec | ○ | ○ |
| VH3-F/R | 5 sec | ○ | ○ |
| MTA1-F3/R3 | 5 sec | ○ | ○ |
| g1(g2)-F/R | 5 se c | ○ | ○ |
| CH3F3/CH4R2 | 5 sec | ○ | ○ |
| TRANS L1/R1 | 5 sec | ○ | ○ |
| KJneo/PGKr-2 | 5 sec | ○ | ○ |
| blasticidin resistance | | absent | partially present |
| FISH analysis | | tetraploid | not conducted |

### [Example 4] Introduction of Ig-NAC(ΔDH/Bsr-) to CHO cell HPRT(-) strain by micronuclei formation (MMCT)

The TF7B10-74-D1 clone retaining Ig-NAC(ADH/Bsr-), from which the removal of the blasticidin resistance gene by Bxb1 recombinase was confirmed in Example 3, was found to be a tetraploid while independently retaining Ig-NAC by FISH analysis (Table 2). These cells are important as platform cells for introduction of chemically synthesized DNA. Therefore, Ig-NAC(ADH/Bsr-) was transferred to a normal diploid CHO cell HPRT(-) strain by MMCT.

### 1. Transfer of Ig-NAC(ADH/Bsr-) to CHO cell HPRT(-) strain by MMCT

Micronuclei were prepared from approximately 2 × 10⁸ cells of TF7B10-74-D1 using 400 nM paclitaxel and 500 nM reversine. The obtained micronuclei were suspended in 4 mL of 1 × phytohemagglutinin (PHA)-P (FUJIFILM Wako Pure Chemical Corp.). Approximately 2 × 10⁷ cells of the recipient CHO cell HPRT(-) strain were treated with trypsin, then washed three times with DMEM, and after replacement with the micronuclei/pHAP suspension, and left at 37°C for 15 minutes. A supernatant was removed, and 2 mL of a PEG solution (2.5 g of PEG1000 <FUJIFILM Wako Pure Chemical Corp.>, 3 mL of DMEM <FUJIFILM Wako Pure Chemical Corp.>, and 0.5 mL of DMSO <FUJIFILM Wako Pure Chemical Corp.>) was added to the cells, which were then left at room temperature for 1 minute and 30 seconds, followed by gradual addition of 10 mL of DMEM. Then, the cells were washed three times with DMEM, suspended in 10 mL of F12 medium containing 10% FBS warmed at 37°C, and cultured in a 10 cm dish. The cells were passaged 24 hours later, medium was replaced with F12 medium containing 800 µg/mL G418 and 10% FBS in 48 of 96-well plates 48 hours later, and cultured. 24 of the drug-resistant colonies that appeared were picked up approximately 2 weeks later.

### 2. PCR analysis

The isolated cells were cultured, and genomic DNA was extracted from the cells using Cellysis (Qiagen N.V.). PCR was carried out with the genomic DNA as a template using the same primers as in the PCR analysis conducted in Example 2. The band of interest was able to be confirmed in 14 out of the 24 picked-up cell clones.

### 3. FISH analysis

Six clones were arbitrarily selected from the 14 cell clones confirmed to exhibit the band of interest by PCR analysis, and subjected to FISH analysis. The FISH analysis was conducted according to the method described in (JP Patent No. 6868250) using a mouse gene-specific probe, mouse cot1 (Invitrogen). Table 3 shows results of the PCR analysis and the FISH analysis for the six cell clones subjected to the FISH analysis. Table 3 shows clone names in the rows and the primers and the extension times used for PCR in the columns. The circle represents Bxb1 recombination-positive. In the table, Ig-NACx1 represents that one copy of Ig-NAC was detected per cell, and Ig-NACx2 represents that two copies of Ig-NAC were detected per cell.

**[Table 3]**

| Primer name | Extension time | 1-5 | 1-10 | 1-12 | 2-4 | 2-6 | 2-7 |
|---|---|---|---|---|---|---|---|
| primerHRAF1-attPR2 | 20 sec | ○ | ○ | ○ | ○ | ○ | ○ |
| ΦC31F1-HRBR1 | 19 sec | ○ | ○ | ○ | ○ | ○ | ○ |
| EIF2AK3-F/R | 5 sec | ○ | ○ | ○ | ○ | ○ | ○ |
| RPIA-F/R | 5 sec | ○ | ○ | ○ | ○ | ○ | ○ |
| IGKC-F/R | 5 sec | ○ | ○ | ○ | ○ | ○ | ○ |
| IGKV-F/R | 5 sec | ○ | ○ | ○ | ○ | ○ | ○ |
| Vk3-2 F/R | 5 sec | ○ | ○ | ○ | ○ | ○ | ○ |
| ELK2P2-F/R | 5 sec | ○ | ○ | ○ | ○ | ○ | ○ |
| VH3-F/R | 5 sec | ○ | ○ | ○ | ○ | ○ | ○ |
| MTA1-F3/R3 | 5 sec | ○ | ○ | ○ | ○ | ○ | ○ |
| g1(g2)-F/R | 5 sec | ○ | ○ | ○ | ○ | ○ | ○ |
| CH3F3/CH4R2 | 5 sec | ○ | ○ | ○ | ○ | ○ | ○ |
| TRANS L1/R1 | 5 sec | ○ | ○ | ○ | ○ | ○ | ○ |
| KJneo/PGKr-2 | 5 sec | ○ | ○ | ○ | ○ | ○ | ○ |
| FISH analysis | | many Ig-NACx2 | high retention rate of Ig-NACx1 | translocation found | high retention rate of Ig-NACx1 | many Ig-NACx2 | high retention rate of Ig-NACx1 |

### [Example 5] Insertion of chemically synthesized DNA comprising human minimalized IgHD by site-directed recombination using ΦC31 recombinase

A human IgHD region is as large as approximately 40.2 kb, most of which is sequences other than D-fragments encoding an antibody. For example, there is an antibody noncoding region of approximately 2500 bp between the first and second D-fragments. On the other hand, a region between the 9th and 10th D-fragments is as short as approximately 100 bp. In human minimalized IgHD designed here, only 100 bp of the antibody noncoding region remained upstream or downstream of each of the D-fragments 1-1 to 6-26, so that the DNA size was drastically decreased to approximately 7.8 kb. However, when an inter-fragment region is shorter than 100 bp, neither deletion nor addition was carried out. In order to modify only the D-region, synthetic DNA was designed so as to connect the D-region with no space to the deletion site of the D-region deleted in Example 1 without duplication and deletion of bases (Figure 7). In short, sequences from downstream of HRA to the D-fragment 1-1 and from the D-fragment 6-26 to HRB were retained without being minimalized. Further, the production of chemically synthesized DNA carrying ΦC31 attP enables site-directed recombination to occur with ΦC31 attB at the acceptor site introduced in Example 2.

Hereinafter, chemically synthesized DNA recombination sites ΦC31 attP and R4 attP, blasticidin resistance gene for confirming the presence or absence of vector introduction, and the production of a vector carrying human minimalized IgHD will be described. First, the vector sequence was divided into three segments, and the resulting plasmid DNAs, human miniA, human miniB, and human miniC, were each chemically synthesized (Eurofins Genomics K.K.). In this context, the human miniA has R4 attP and from downstream of HRA to a D-fragment 3-10, the human miniB has D-fragments 4-11 to 3-22, and the human miniC has a D-fragment 4-23 to HRB, ΦC31 attP, and blasticidin resistance gene. A human minimalized IgHD vector carrying the human miniA, miniB, and miniC was produced by the following procedures 1. and 2. (Figure 8).

### 1. Insertion of human miniA fragment into human miniB

The human miniA was cleaved with restriction enzymes XhoI and NotI. The miniA was inserted into the human miniB cleaved with the same restriction enzymes XhoI and NotI to obtain R4 attP-HRA/1-1_3-22.

### 2. Insertion of human miniC fragment into R4 attP-HRA/1-1_3-22

The human miniC was cleaved with restriction enzymes EcoRI and Sail. The human miniC fragment was inserted into the R4 attP-HRA/1-1_3-22 cleaved with the same restriction enzymes EcoRI and SalI to construct a human minimalized IgHD vector.

### 3. Introduction of human minimalized IgHD vector using ΦC31 recombinase

Site-directed recombination is capable of occurring between ΦC31 attB in the platform cells for introduction of chemically synthesized DNA constructed in Example 4 and ΦC31 attP on the human minimalized IgHD vector (Figure 9). A ΦC31 recombinase gene expression vector (JP Patent No. 6868250) and the human minimalized IgHD vector were introduced by electroporation to the chemically synthesized DNA introduction platform-carrying and Ig-NAC-retaining CHO cells 1-10 (Example 4). The 1-10 cells were treated with trypsin and suspended at 3 × 10⁶ cells/mL in Opti MEM, followed by the addition of 1 µg of a ΦC31 recombinase gene expression vector ΦC31pEF1 (JP Patent No. 6868250) and 3 µg of the human minimalized IgHD vector and electroporation at 150 V and 7.5 ms using NEPA21 (Nepa Gene Co., Ltd.). The cells thus pulsed were inoculated to two 384-well plates. The cells were medium was replaced with F12 medium containing 800 µg/mL G418, 800 µg/mL blasticidin, and 10% FBS at 48 to 72 hours later and cultured. 24 clones having blasticidin resistance were picked up 11 days later.

### 4. PCR analysis

The isolated cells were cultured, and genomic DNA was extracted from the cells using Cellysis (Qiagen N.V.). PCR analysis was conducted with the genomic DNA as a template using one (DC31 site-directed recombination-specific primer, two primers produced within human minimalized IgHD, and 12 Ig-NAC-specific primers produced for Ig-NAC. Approximately 0.1 µg of the genomic DNA was used in PCR amplification. The Taq polymerase used was KODone (Toyobo Co., Ltd.), and PCR was performed by 1 cycle of 98°C for 2 minutes followed by 35 cycles with denaturation at 98°C for 10 seconds, annealing at 60°C for 5 seconds, and extension at 68°C for: 5 seconds in the case of an amplification fragment having a length of 500 bp or less; and 100 seconds/kb for a length of 500 bp or more.
BsdR1: 5' - ATGCAGATCGAGAAGCACCT - 3' (SEQ ID NO: 38)
2-8to3-9F: 5' - AACGCACCAGACCAGCAGAC - 3' (SEQ ID NO: 39)
4-11to5-12R: 5' -GACGTGGGGCCTAGAGGCT - 3' (SEQ ID NO: 40)
3-22to4-23F: 5' - CAGGCGGGGAAGATTCAGAA- 3' (SEQ ID NO: 41)
4-23to5-24R: 5' - TAGAGAGCCTGGGCCTAGAG- 3' (SEQ ID NO: 42)

### 5. FISH analysis

Three clones confirmed to exhibit the band of interest by PCR analysis were subjected to FISH analysis. The FISH analysis was conducted according to the method described in (JP Patent No. 6868250) using a mouse gene-specific probe, mouse cot1 (Invitrogen). Table 4 shows results of the PCR analysis and the FISH analysis for the three cell clones subjected to the FISH analysis.

Table 4 shows clone names in the rows and the primers and the extension times used for PCR in the columns. The circle represents positive. In the table, Ig-NACx1 represents that one copy of Ig-NAC was detected per cell, and Ig-NACx2 represents that two copies of Ig-NAC were detected per cell.

**[Table 4]**

| Primer name | Extension time | 1-10 pEFI-3 | 1-10 pEFI-8 | 1-10 pEFI-17 |
|---|---|---|---|---|
| phiC31F1-BsdR1 | 5 sec | ○ | ○ | ○ |
| 2-8to3-9/4-11to5-12 | 8 sec | ○ | ○ | ○ |
| 3-22to4-23/4-23to5-24 | 5 sec | ○ | ○ | ○ |
| EIF2AK3-F/R | 5 sec | ○ | ○ | ○ |
| RPIA-F/R | 5 sec | ○ | ○ | ○ |
| IGKC-F/R | 5 sec | ○ | ○ | ○ |
| IGKV-F/R | 5 sec | ○ | ○ | ○ |
| Vk3-2 F/R | 5 sec | ○ | ○ | ○ |
| ELK2P2-F/R | 5 sec | ○ | ○ | ○ |
| VH3-F/R | 5 sec | ○ | ○ | ○ |
| MTA1-F3/R3 | 5 sec | ○ | ○ | ○ |
| g1(g2)-F/R | 5 sec | ○ | ○ | ○ |
| CH3F3/CH4R2 | 5 sec | ○ | ○ | ○ |
| TRANS L1/R1 | 5 sec | ○ | ○ | ○ |
| KJneo/PGKr-2 | 5 sec | ○ | ○ | ○ |
| FISH analysis | | high retention rate of Ig-NACx1 | high retention rate of Ig-NACx1 | many Ig-NACx2 |

### [Example 6] Removal of partial sequence by site-directed recombination using R4 recombinase

Among the sequences introduced in Ig-NAC(ΔDH), the CAG promoter, the blasticidin resistance gene, and the like are sequences unnecessary for antibody gene expression. These sequences are flanked by R4 attB and R4 attP and are capable of being removed through site-directed recombination reaction between R4 attB and R4 attP by introducing R4 recombinase to cells (Figure 10).

### 1. Introduction of R4 recombinase gene expression vector

A R4 recombinase gene expression vector (JP Patent No. 6868250) was introduced by electroporation to the CHO cells 1-10 pEFI-8 which were confirmed to have the inserted human minimalized IgHD vector and had a high retention rate of one copy of Ig-NAC (Example 5). The 1-10 pEFI-8 cells were treated with trypsin and suspended at 3 × 10⁶ cells/mL in Opti MEM, followed by the addition of 10 µg of the R4 recombinase gene expression vector and electroporation at 150 V and 7.5 ms using NEPA21 (Nepa Gene Co., Ltd.). The cells thus pulsed were inoculated to one 10 cm dish. The cells were treated with trypsin 24 to 48 hours later and inoculated at 150 cells per 384-well plate. The cells thus inoculated to a 384-well plate were cultured in F12 medium containing 800 µg/mL G418 and 10% FBS for 2 weeks, and 24 of the single clones were picked up.

### 2. PCR analysis

The isolated cells were cultured, and genomic DNA was extracted from the cells using Cellysis (Qiagen N.V.). PCR analysis was conducted with the genomic DNA as a template using one R4 site-directed recombination-specific primer, two primers produced within human minimalized IgHD, and 12 Ig-NAC-specific primers produced for Ig-NAC. Six clones were selected in which a R4 site-directed recombination-specific band was obtained as a result of PCR using the R4 site-directed recombination-specific primer. Approximately 0.1 µg of the genomic DNA was used in PCR amplification. The Taq polymerase used was KODone (Toyobo Co., Ltd.), and PCR was performed by 1 cycle of 98°C for 2 minutes followed by 35 cycles with denaturation at 98°C for 10 seconds, annealing at 60°C for 5 seconds, and extension at 68°C for: 5 seconds in the case of an amplification fragment having a length of 500 bp or less; and 100 seconds/kb for a length of 500 bp or more.
R4attLF2: 5' -CCCTCAAGGTGTGAACAGGG- 3' (SEQ ID NO: 43)
R4attLR2: 5' -CTGGAGGGAGGCATGTTCTG- 3' (SEQ ID NO: 44)

### 3. FISH analysis

The six cell clones confirmed to exhibit the band of interest by PCR analysis were subjected to FISH analysis. The FISH analysis was conducted according to the method described in (JP Patent No. 6868250) using a mouse gene-specific probe, mouse cot1 (Invitrogen). Table 5 shows results of the PCR analysis and the FISH analysis for the six cell clones subjected to the FISH analysis.

Table 5 shows clone names in the rows and the primers and the extension times used for PCR in the columns. The circle represents positive. In the table, Ig-NACx1 represents that one copy of Ig-NAC was detected per cell.

**[Table 5]**

| Primer name | Extension time | C23 | K5 | L15 | M24 | E8 | I2 |
|---|---|---|---|---|---|---|---|
| R4attLP2 | 5 sec | ○ | ○ | ○ | ○ | ○ | ○ |
| 2-8to3-9/4-1to5-12 | 8 sec | ○ | ○ | ○ | ○ | ○ | ○ |
| 3-22to4-23/4-23to5-24 | 5 sec | ○ | ○ | ○ | ○ | ○ | ○ |
| EIF2AK3-F/R | 5 sec | ○ | ○ | ○ | ○ | ○ | ○ |
| RPIA-F/R | 5 sec | ○ | ○ | ○ | ○ | ○ | ○ |
| IGKC-F/R | 5 sec | ○ | ○ | ○ | ○ | ○ | ○ |
| IGKV-F/R | 5 sec | ○ | ○ | ○ | ○ | ○ | ○ |
| Vk3-2 F/R | 5 sec | ○ | ○ | ○ | ○ | ○ | ○ |
| ELK2 P2-F/R | 5 sec | ○ | ○ | ○ | ○ | ○ | ○ |
| VH3-F/R | 5 sec | ○ | ○ | ○ | ○ | ○ | ○ |
| MTA1-F3/R3 | 5 sec | ○ | ○ | ○ | ○ | ○ | ○ |
| g1(g2)-F/R | 5 sec | ○ | ○ | ○ | ○ | ○ | ○ |
| CH3F3/CH4R2 | 5 sec | ○ | ○ | ○ | ○ | ○ | ○ |
| TRANS L1/R1 | 5 sec | ○ | ○ | ○ | ○ | ○ | ○ |
| KJneo/PGKr-2 | 5 sec | ○ | ○ | ○ | ○ | ○ | ○ |
| FISH analysis | | all six clones exhibited a high retention rate of Ig-NACx1 | | | | | |

### [Example 7] Introduction of human minimalized IgHD-carrying Ig-NAC to mouse ES cell line by micronuclei formation (MMCT)

Two clones, K5 (1-10 pEF1-K5) and M24 (1-10 pEF1-M24), were arbitrarily selected from the six clones confirmed to undergo R4 site-directed recombination by PCR and FISH analysis in Example 6, and used as chromosome donor cells to transfer human minimalized IgHD-carrying Ig-NAC to mouse ES cells by MMCT.

### 1. Transfer of human minimalized IgHD-carrying Ig-NAC to mouse ES cell by MMCT

The chromosome receptor cells used were a mouse ES cell HKD31 6TG-9 strain (XO) (JP Patent No. 4082740) in which both alleles of endogenous immunoglobulin heavy chain and κ chain loci were disrupted. The HKD31 6TG-9 strain was cultured with a method of using a mitomycin C-treated mouse embryonic fibroblast cell line as feeder cells and Dulbecco's modified Eagle medium (DMEM) containing 15% FBS and 1% nucleosides, penicillin-streptomycin, non-essential amino acids, L-glutamine solution, 2-mercaptoethanol, and LIF as a medium for an ES cell and culturing at 37°C on the feeder cell layer. First, 400 nM paclitaxel and 500 nM reversine were added to approximately 4 × 10⁷ cells of 1-10 pEF1-K5 and 1-10 pEF1-M24 to prepare micronuclei . The whole amount of the obtained micronuclei was suspended in 5 mL of DMEM and precipitated by centrifugation. 5 × 10⁶ to 4 × 10⁶ cells (1/2 of the cells in a 10 cm dish was inoculated to one 10 cm dish 2 days before MMCT) of the mouse ES cell HKD31 6TG-9 strain were dispersed in trypsin, then washed three times with DMEM, suspended in 5 mL of DMEM, and then added onto the precipitates of the micronuclei. A supernatant was removed by centrifugation at 1200 rpm for 5 minutes. The precipitates were loosened well by tapping, and 0.5 mL of a PEG solution (2.5 g of PEG1000 <FUJIFILM Wako Pure Chemical Corp.>, 3 mL of DMEM <FUJIFILM Wako Pure Chemical Corp.>, and 0.5 mL of DMSO <FUJIFILM Wako Pure Chemical Corp.>) was added thereto. After shaking in a water bath of 37°C for 90 seconds, 13 mL of DMEM was gradually added thereto. A supernatant was removed by centrifugation at 1200 rpm for 5 minutes, while the precipitates were suspended in 30 mL of a medium for an ES cell and inoculated to three 10 cm dishes inoculated with feeder cells in advance. The medium was replaced with a medium for an ES cell supplemented with 350 µg/mL G418 from 48 hours later, followed by medium replacement according to the same composition once every two days. 14 of the drug-resistant colonies that appeared were picked up 1 week to 10 days later.

### 2. PCR analysis

The isolated cells were cultured, and genomic DNA was extracted from the cells using Cellysis (Qiagen N.V.). PCR was carried out with the genomic DNA as a template using the same primers as in the PCR analysis conducted in Example 5. The band of interest was able to be confirmed in 5 out of the 14 picked-up colonies.

### 3. Karyotypic analysis

PCR positive clones cultured in 6 wells are treated with trypsin 1 hour and 30 minutes after addition of 0.05 µg/mL MAS (Funakoshi Co., Ltd.). The cells were centrifuged at 1500 rpm for 5 minutes, then suspended in 5 mL of 0.074 M potassium chloride, and left for 20 minutes. A preparation was produced by treatment with a Carnoy solution. This preparation was stained with DAPI using 50 µL of a mountant (Slowfade^{™} Gold antifade with DAPI). Then, the absence of karyotypic abnormality in mouse chromosome was confirmed. Table 6 shows results of the PCR analysis and the karyotypic analysis.

Table 6 shows the PCR and karyotypic analysis results on each clone. The table shows clone names in the rows and the primers and the extension times used for PCR in the columns. The circle represents positive.

**[Table 6]**

| Primer name | Extension time | K5-6 | K5-7 | M24-2 | M24-4 | M24-5 |
|---|---|---|---|---|---|---|
| R4attLP2 | 5 sec | ○ | ○ | ○ | ○ | ○ |
| 2-8to3-9/4-11to5-12 | 8 sec | ○ | ○ | ○ | ○ | ○ |
| 3-22to4-23/4-23to5-24 | 5 sec | ○ | ○ | ○ | ○ | ○ |
| EIF2AK3-F/R | 5 sec | ○ | ○ | ○ | ○ | ○ |
| RPIA-F/R | 5 sec | ○ | ○ | ○ | ○ | ○ |
| IGKC-F/R | 5 sec | ○ | ○ | ○ | ○ | ○ |
| IGKV-F/R | 5 sec | ○ | ○ | ○ | ○ | ○ |
| Vk3-2 F/R | 5 sec | ○ | ○ | ○ | ○ | ○ |
| ELK2 P2-F/R | 5 sec | ○ | ○ | ○ | ○ | ○ |
| VH3-F/R | 5 sec | ○ | ○ | ○ | ○ | ○ |
| MTA1-F3/R3 | 5 sec | ○ | ○ | ○ | ○ | ○ |
| g1(g2)-F/R | 5 sec | ○ | ○ | ○ | ○ | ○ |
| CH3F3/CH4R2 | 5 sec | ○ | ○ | ○ | ○ | ○ |
| TRANS L1/R1 | 5 sec | ○ | ○ | ○ | ○ | ○ |
| KJneo/PGKr-2 | 5 sec | ○ | ○ | ○ | ○ | ○ |
| karyotypic analysis percentage of retention of 39 mouse chromosomes and Ig-NAC | | 75% | 100% | 75% | 80% | 89.5% |

### [Example 8] Production of chimeric mouse from mouse ES cell line that retains human minimalized IgHD-containing Ig-NAC

In order to confirm human antibody production, chimeric mice were produced from mouse ES cells that retained human minimalized IgHD-containing Ig-NAC.

Chimeric mice are produced according to the approach described in Gene Targeting, Experimental Medicine, 1995 using the mouse ES cells that retained human minimalized IgHD-containing Ig-NAC, which were obtained in Example 7. The host used were the morula and the 8-cell-stage embryo obtained by sexual crossbreeding of MCH (ICR) (white, purchased from CLEA Japan, Inc.) or HKLD mice having deficiency (Igh/Igκ KO) or low expression (Igl1 low) of an antibody gene. Whether the newborn mice obtained though transplantation of the injected embryo into a surrogate mother are chimeric mice, can be determined based on the coat color. The transplantation of the embryos into surrogate mothers produces chimeric mice (a dark brown color area is observed in coat color).

Four clones of the mouse ES cell line that retained human minimalized -containing Ig-NAC were injected to a total of 211 ICR embryos, which were then transplanted to six surrogate mothers. As a result, a total of five GFP-positive chimeric mice were obtained. As a result of determining a chimeric rate based on coat color, the chimeric rate was 10 to 40% as to the five chimeric mice. Two clones of the mouse ES cell line were injected to 465 HKLD embryos, which were then transplanted to 27 surrogate mothers. As a result, 14 GFP-positive individuals were obtained. As a result of determining a chimeric rate based on coat color, the chimeric rate was 5 to 50% as to the 14 chimeric mice.

### [Example 9] Insertion of chemically synthesized DNA comprising bovinized human IgHD by site-directed recombination using ΦC31 recombinase

In bovines, an antibody having CDRH3 as very long as 40 to 70 amino acids is observed at frequency of approximately 10% (Wang et al., Cell, 153: 1379-93, 2013). Long CDRH3 takes a form protruding from the surface of an antibody molecule and is capable of binding to a site, such as an epitope hidden by protein folding, to which the binding of a usual antibody is difficult. In order to adopt this feature to a human antibody, the sequences of human D-fragments serving as an antibody coding region in human minimalized DH were substituted with bovine D-fragment sequences. This substitution was carried out such that a bovine D-fragment 8-2 corresponded to the position of a human D-fragment 3-10. However, the order of the bovine D-fragments was not changed. Unreplaced three human D-fragments were deleted together with 100 bp upstream and downstream thereof because the number of bovine D-fragments is fewer than that of human ones. In this way, bovinized human IgHD was designed (Figure 11).

This bovinized human IgHD was divided into three segments in accordance with Example 5, and these three segments of the plasmid DNA, bovine miniA (comprising HRA and bovine D-fragments B1-1 to B8-2 corresponding to a region from human D-fragments 1-1 to 3-10), bovine miniB (comprising bovine D-fragments B6-2 to B5-4 corresponding to a region from human D-fragments 4-11 to 3-22), and bovine miniC (comprising bovine D-fragments B6-4 to B9-4 corresponding to a region from human D-fragments 4-23 to 1-26, HRB, ΦC31 attP, and blasticidin resistance gene), were chemically synthesized (Eurofins Genomics K.K.). A bovinized human minimalized IgHD vector carrying the bovine miniA, miniB, and miniC was produced by the following procedures 1. and 2. (Figure 12).

### 1. Insertion of bovine miniA fragment into bovine miniB

The bovine miniA was cleaved with restriction enzymes XhoI and NotI. The miniA was inserted into the bovine miniB cleaved with the same restriction enzymes XhoI and NotI to obtain R4 attP-HRA/1-1_3-22 (comprising HRA and bovine D-fragments B1-1 to B5-4).

### 2. Insertion of bovine miniC fragment into R4 attP-HRA/1-1_3-22

The human miniC was cleaved with restriction enzymes EcoRI and Sail. The human miniC fragment was inserted into the R4 attP-HRA/1-1_3-22 cleaved with the same restriction enzymes EcoRI and SalI to construct a bovinized human IgHD vector.

### 3. Introduction of bovinized human IgHD vector by ΦC31 recombinase

The same method as in Example 5 was carried out. The 1-10 cells (Example 4) were treated with trypsin and suspended at 3 × 10⁶ cells/mL in Opti MEM, followed by the addition of 1 µg of a ΦC31 recombinase gene expression vector ΦC31pEF1 (JP Patent No. 6868250) and 3 µg of the bovinized human IgHD vector and electroporation at 150 V and 7.5 ms using NEPA21 (Nepa Gene Co., Ltd.). The cells thus pulsed were inoculated to two 384-well plates. For the cells, medium was replaced with F12 medium containing 800 µg/mL G418, 800 µg/mL blasticidin, and 10% FBS at 48 to 72 hours later and cultured. 16 clones having blasticidin resistance were picked up 13 days later.

### 4. PCR analysis

The isolated cells were cultured, and genomic DNA was extracted from the cells using Cellysis (Qiagen N.V.). PCR analysis was conducted with the genomic DNA as a template using the same primers as in Example 5.

### 5. FISH analysis

Four clones were arbitrarily selected from 16 cell clones confirmed to exhibit the band of interest by PCR analysis, and subjected to FISH analysis. The FISH analysis was conducted according to the method described in (JP Patent No. 6868250) using a mouse gene-specific probe, mouse cot1 (Invitrogen).

Table 7 shows results of the PCR analysis and the FISH analysis for the four cell clones subjected to the FISH analysis.

Table 7 shows clone names in the rows and the primers and the extension times used for PCR in the columns. The circle represents positive. In the table, Ig-NACx1 represents that one copy of Ig-NAC was detected per cell, and Ig-NACx2 represents that two copies of Ig-NAC were detected per cell.

**[Table 7]**

| Primer name | Extension time | 1-10 C1 | 1-10 C2 | 1-10 C3 | 1-10 C4 |
|---|---|---|---|---|---|
| phiC31F1-BsdR1 | 5 sec | ○ | ○ | ○ | ○ |
| 2-8to3-9/4-11to5-12 | 8 sec | ○ | ○ | ○ | ○ |
| 3-22to4-23/4-23to5-24 | 5 sec | ○ | ○ | ○ | ○ |
| EIF2AK3-F/R | 5 sec | ○ | ○ | ○ | ○ |
| RPIA-F/R | 5 sec | ○ | ○ | ○ | ○ |
| IGKC-F/R | 5 sec | ○ | ○ | ○ | ○ |
| IGKV-F/R | 5 sec | ○ | ○ | ○ | ○ |
| Vk3-2 F/R | 5 sec | ○ | ○ | ○ | ○ |
| ELK2 P2-F/R | 5 sec | ○ | ○ | ○ | ○ |
| VH3-F/R | 5 sec | ○ | ○ | ○ | ○ |
| MTA1-F3/R3 | 5 sec | ○ | ○ | ○ | ○ |
| g1(g2)-F/R | 5 sec | ○ | ○ | ○ | ○ |
| CH3F3/CH4R2 | 5 sec | ○ | ○ | ○ | ○ |
| TRANS L1/R1 | 5 sec | ○ | ○ | ○ | ○ |
| KJneo/PGKr-2 | 5 sec | ○ | ○ | ○ | ○ |
| FISH analysis | | high retention rate of Ig-NACx1 | many Ig-NACx2 | high retention rate of Ig-NACx0 | high retention rate of Ig-NACx1 |

### [Example 10] Removal of partial sequence by site-directed recombination using R4 recombinase

### 1. Introduction of R4 recombinase gene expression vector

The same method as in Example 6 was carried out. The 1-10 C4 cells confirmed to be introduced with the bovinized human IgHD vector (Example 9) were treated with trypsin and suspended at 3 × 10⁶ cells/mL in Opti MEM, followed by the addition of 10 µg of the R4 recombinase gene expression vector and electroporation at 150 V and 7.5 ms using NEPA21 (Nepa Gene Co., Ltd.). The cells thus pulsed were inoculated to one 10 cm dish. The cells were treated with trypsin 24 to 48 hours later and inoculated at 150 cells per 384-well plate. The cells thus inoculated to a 384-well plate were cultured in F12 medium containing 800 µg/mL G418 and 10% FBS for 17 days, and 22 of single clones were picked up.

### 2. PCR analysis

The isolated cells were cultured, and genomic DNA was extracted from the cells using Cellysis (Qiagen N.V.). PCR analysis was conducted with the genomic DNA as a template using one R4 site-directed recombination-specific primer, two primers produced within human minimalized IgHD, and 12 Ig-NAC-specific primers produced for Ig-NAC in accordance with Example 6. Three clones were selected in which a R4 site-directed recombination-specific band was obtained as a result of PCR using the R4 site-directed recombination-specific primer.

### 3. FISH analysis

The three clones confirmed to exhibit the band of interest by PCR analysis were subjected to FISH analysis. The FISH analysis was conducted in accordance with the method described in (JP Patent No. 6868250) using a mouse gene-specific probe, mouse cot1 (Invitrogen). Table 8 shows results of the PCR analysis and the FISH analysis for the three cell clones subjected to the FISH analysis. Table 8 shows clone names in the rows and the primers and the extension times used for PCR in the columns. The circle represents positive. In the table, Ig-NACx1 represents that one copy of Ig-NAC was detected per cell.

**[Table 8]**

| Primer name | Extension time | D4 | G11 | L2 |
|---|---|---|---|---|
| R4attLP2 | 5 sec | ○ | ○ | ○ |
| 2-8to3-9/4-11to5-12 | 8 sec | ○ | ○ | ○ |
| 3-22to4-23/4-23to5-24 | 5 sec | ○ | ○ | ○ |
| EIF2AK3-F/R | 5 sec | ○ | ○ | ○ |
| RPIA-F/R | 5 sec | ○ | ○ | ○ |
| IGKC-F/R | 5 sec | ○ | ○ | ○ |
| IGKV-F/R | 5 sec | ○ | ○ | ○ |
| Vk3-2 F/R | 5 sec | ○ | ○ | ○ |
| ELK2P2-F/R | 5 sec | ○ | ○ | ○ |
| VH3-F/R | 5 sec | ○ | ○ | ○ |
| MTA1-F3/R3 | 5 sec | ○ | ○ | ○ |
| g1(g2)-F/R | 5 sec | ○ | ○ | ○ |
| CH3F3/CH4R2 | 5 sec | ○ | ○ | ○ |
| TRANS L1/R1 | 5 sec | ○ | ○ | ○ |
| KJneo/PGKr-2 | 5 sec | ○ | ○ | ○ |
| FISH analysis | | all three clones had a high retention rate of Ig-NACx1 | | |

### [Example 11] Introduction of bovine minimalized IgHD (bovinized human minimalized IgHD)-containing Ig-NAC to mouse ES cell line by micronuclei formation (MMCT)

### 1. Transfer of bovine minimalized IgHD-carrying Ig-NAC to mouse ES cell by MMCT

The chromosome donor cells used were two clones, G11 (1-10 C4-G11) and L2 (1-10 C4-L2), arbitrarily selected from the three clones confirmed to undergo R4 site-directed recombination by PCR and FISH analysis in Example 10. The chromosome receptor cells used were a mouse ES cell HKD31 6TG-9 strain (XO) (JP Patent No. 4082740) in which both alleles of endogenous immunoglobulin heavy chain and κ chain loci were disrupted. Micronuclei were formed by the same method as in Example 7 and fused with the mouse ES cell HKD31 6TG-9 strain (XO), and the cells were inoculated to three 10 cm dishes. The medium was replaced with a medium for an ES cell supplemented with 350 µg/mL G418 from 48 hours later, followed by medium replacement according to the same composition once every two days. 23 of the drug-resistant colonies that appeared were picked up 1 week to 10 days later.

### 2. PCR analysis

The isolated cells were cultured, and genomic DNA was extracted from the cells using Cellysis (Qiagen N.V.). PCR was carried out with the genomic DNA as a template using the same primers as in the PCR analysis conducted in Example 6. The band of interest was able to be confirmed in 14 out of the 23 picked-up cell clones.

### 3. Karyotypic analysis

The absence of karyotypic abnormality in mouse chromosome was confirmed by the same method as in Example 7. Table 9 shows results of the PCR analysis and the karyotypic analysis. Among the karyotypically analyzed clones, nine clones had 39 mouse chromosomes and a high retention rate of Ig-NACx1 without translocation. Table 9 shows the PCR and karyotypic analysis results on a clone basis. The table shows clone names in the rows and the primers and the extension times used for PCR in the columns. The circle represents positive. In the table, "translocated" represents that Robertsonian translocation was observed. In the table, Ig-NACx1 represents that one copy of Ig-NAC was detected per cell.

**[Table 9]**

| Primer name | Extension time | G11-1 | G11-2 | G11-3 | G11-5 | G11-9 | L2-3 | L2-4 | L2-5 | L2-6 | L2-7 | L2-8 | L2-9 | L2-10 | L2-12 |
|---|---|---|---|---|---|---|---|---|---|---|---|---|---|---|---|
| R4attLP2 | 5 sec | ○ | ○ | ○ | ○ | ○ | ○ | ○ | ○ | ○ | ○ | ○ | ○ | ○ | ○ |
| 2-8to3-9/4-11to5-12 | 8 sec | ○ | ○ | ○ | ○ | ○ | ○ | ○ | ○ | ○ | ○ | ○ | ○ | ○ | ○ |
| 3-22to4-23/4-23to5-24 | 5 sec | ○ | ○ | ○ | ○ | ○ | ○ | ○ | ○ | ○ | ○ | ○ | ○ | ○ | ○ |
| EIF2AK3-F/R | 5 sec | ○ | ○ | ○ | ○ | ○ | ○ | ○ | ○ | ○ | ○ | ○ | ○ | ○ | ○ |
| RPIA-F/R | 5 sec | ○ | ○ | ○ | ○ | ○ | ○ | ○ | ○ | ○ | ○ | ○ | ○ | ○ | ○ |
| IGKC-F/R | 5 sec | ○ | ○ | ○ | ○ | ○ | ○ | ○ | ○ | ○ | ○ | ○ | ○ | ○ | ○ |
| IGKV-F/R | 5 sec | ○ | ○ | ○ | ○ | ○ | ○ | ○ | ○ | ○ | ○ | ○ | ○ | ○ | ○ |
| Vk3-2 F/R | 5 sec | ○ | ○ | ○ | ○ | ○ | ○ | ○ | ○ | ○ | ○ | ○ | ○ | ○ | ○ |
| ELK2P2-F/R | 5 sec | ○ | ○ | ○ | ○ | ○ | ○ | ○ | ○ | ○ | ○ | ○ | ○ | ○ | ○ |
| VH3-F/R | 5 sec | ○ | ○ | ○ | ○ | ○ | ○ | ○ | ○ | ○ | ○ | ○ | ○ | ○ | ○ |
| MTA1-F3/R3 | 5 sec | ○ | ○ | ○ | ○ | ○ | ○ | ○ | ○ | ○ | ○ | ○ | ○ | ○ | ○ |
| g1(g2)-F/R | 5 sec | ○ | ○ | ○ | ○ | ○ | ○ | ○ | ○ | ○ | ○ | ○ | ○ | ○ | ○ |
| CH3F3/CH4R2 | 5 sec | ○ | ○ | ○ | ○ | ○ | ○ | ○ | ○ | ○ | ○ | ○ | ○ | ○ | ○ |
| TRANS L1/R1 | 5 sec | ○ | ○ | ○ | ○ | ○ | ○ | ○ | ○ | ○ | ○ | ○ | ○ | ○ | ○ |
| KJneo/PGKr-2 | 5 sec | ○ | ○ | ○ | ○ | ○ | ○ | ○ | ○ | ○ | ○ | ○ | ○ | ○ | ○ |
| karyotypic analysis (percentage of retention of 39 mouse chromosomes and Ig-NACx1) | | 80% | 80% translocation | 80% translocation | 95% | 85% | 80% | 0% | 90% | 85% | 75% | 85% translocation | 94% | 0% | 15% |

### [Example 12] Production of chimeric mouse from mouse ES cell line that retains bovine minimalized IgHD-containing Ig-NAC

In order to confirm human antibody production, chimeric mice were produced from mouse ES cells that retained bovine minimalized IgHD-containing Ig-NAC.

Chimeric mice are produced according to the approach described in Gene Targeting, Experimental Medicine, 1995 using the obtained mouse ES cells that retained bovine minimalized IgHD-containing Ig-NAC. The host used were the morula and the 8-cell-stage embryo obtained by sexual crossbreeding of MCH (ICR) (white, purchased from CLEA Japan, Inc.) or HKLD mice having deficiency (Igh/Igκ KO) or low expression (Igl1 low) of an antibody gene. Whether the newborn mice obtained though transplantation of the injected embryo into a surrogate mother are chimeric mice, can be determined based on the coat color. The transplantation of the embryos into surrogate mothers produces chimeric mice (a dark brown color area is observed in coat color).

Eight clones of the mouse ES cells that retained bovine minimalized IgHD-containing Ig-NAC were injected to a total of 591 ICR embryos, which were then transplanted to 32 surrogate mothers. As a result, 18 GFP-positive individuals were obtained. As a result of determining a chimeric rate based on coat color, the chimeric rate was 5 to 60% as to the 18 chimeric mice. Five clones were injected to a total of 380 HKLD embryos, which were then transplanted to 21 surrogate mothers. As a result, 13 GFP-positive individuals were obtained. As a result of determining a chimeric rate based on coat color, the chimeric rate was 5 to 80% as to the 13 chimeric mice.

### [Example 13] Analysis of human minimalized chimeric mouse (B cell analysis, ELISA, and cDNA preparation)

B cell differentiation analysis, ELISA analysis for measurement of an antibody concentration in plasma, and gene sequence analysis by cDNA collection from the spleen may be conducted to evaluate whether a human antibody is produced from a human minimalized chimeric mouse.

### 1. B cell differentiation analysis

If cells copositive to EGFP and a B cell marker B220 can be detected, this indicates B cell differentiation based on contribution to antibody production and serves as indirect evaluation of functional expression of an antibody. Peripheral blood obtained from each chimeric mouse was used as a sample to analyze B220 by flow cytometry in accordance with the method described in JP Patent No. 6868250. As a result, not only a GFP-positive cell population but a GFP- and B220-copositive population was confirmed, indicating that a functional antibody was expressed. A GFP-positive rate on each individual was up to 28.12%, and a GFP- and B220-copositive rate on each individual was up to 2.30%.

### 2. Antibody expression analysis by ELISA

The measurement of the plasma concentrations of a mouse antibody (my, mµ, mκ, and mλ) and a human antibody (hy, hµ, hκ, hλ, hγ1, hy2, hy3, hy4, hα, hε, and hδ) are measured, as well as the confirmation of the presence of antibody expression in mice, are performed by the method described in JP Patent No. 6868250 using plasma obtained from each chimeric mouse.

### 3. Expression analysis and sequence identification of human antibody

cDNA is synthesized from RNA derived from the spleen of each human antibody-producing mouse, and variable regions of a human antibody gene are cloned and sequenced for base sequence. Analysis and evaluation were conducted in accordance with JP Patent No. 6868250. The spleen was collected, then preserved overnight at 4°C in RNAlater (Ambion, Inc.), then taken out of the solution, and stored at -80°C. 1 mL of ISOGEN (Nippon Gene Co., Ltd.) is added to a pressure-resistant tube containing zirconia beads, and 50 mg of tissues is added thereto. The tissues were homogenized in an apparatus (approximately 30 to 45 seconds). After confirmation that the tissues were homogenized, the homogenates were spun down. Then, 1 mL of a supernatant was separated, and 200 µL of chloroform was added thereto, followed by RNA extraction using RNeasy Mini kit according to manufacturer's protocol.

### [Example 14] Analysis of bovine minimalized chimeric mouse (B cell analysis, ELISA, and cDNA preparation)

B cell differentiation analysis, ELISA analysis for measurement of an antibody concentration in plasma, and gene sequence analysis by cDNA collection from the spleen may be conducted to evaluate whether a human antibody is produced from a bovine minimalized chimeric mouse.

### 1. B cell differentiation analysis

If cells copositive to EGFP and a B cell marker B220 can be detected, this indicates B cell differentiation based on contribution to antibody production and serves as indirect evaluation of functional expression of an antibody. Peripheral blood obtained from each chimeric mouse was used as a sample to analyze B220 by flow cytometry in accordance with the method described in JP Patent No. 6868250. As a result, not only a GFP-positive cell population but a GFP- and B220-copositive population was confirmed, indicating that a functional antibody was expressed. A GFP-positive rate on each individual was up to 40.38%, and a GFP- and B220-copositive rate on each individual was up to 3.23%.

### 2. Antibody expression analysis by ELISA

The measurement of the plasma concentrations of a mouse antibody (my, mµ, mκ, and mλ) and a human antibody (hy, hµ, hκ, hλ, hγ1, hy2, hy3, hy4, hα, hε, and hδ), as well as the confirmation of the presence of antibody expression in mice, are performed by the method described in JP Patent No. 6868250 using plasma obtained from each chimeric mouse.

### 3. Expression analysis and sequence identification of human antibody

cDNA is synthesized from RNA derived from the spleen of each human antibody-producing mouse, and variable regions of a human antibody gene are cloned and sequenced for base sequence. Analysis and evaluation were conducted in accordance with JP Patent No. 6868250. The spleen was collected, then preserved overnight at 4°C in RNAlater (Ambion, Inc.), then taken out of the solution, and stored at -80°C. 1 mL of ISOGEN (Nippon Gene Co., Ltd.) is added to a pressure-resistant tube containing zirconia beads, and 50 mg of tissues is added thereto. The tissues were homogenized in an apparatus (approximately 30 to 45 seconds). After confirmation that the tissues were homogenized, the homogenates were spun down. Then, 1 mL of a supernatant was separated, and 200 µL of chloroform was added thereto, followed by RNA extraction using RNeasy Mini kit according to manufacturer's protocol.

### [Example 15] Repertoire analysis of human minimalized (ΔIgH/ΔIgκ-ES) chimeric mouse

### 1. Cloning and sequencing for base sequence of heavy chain region of human antibody gene from cDNA derived from spleen of human minimalized (ΔIgH/ΔIgκ-ES) chimeric mouse

A heavy chain variable region of a human antibody gene was amplified by PCR using primers shown below as to cDNA derived from the spleen of each chimeric mouse retaining human minimalized IgHD. Spleen-derived cDNA obtained from a nonchimeric mouse ICR was used as a negative control.
For constant region:
   HIGMEX1-2: 5' - CCAAGCTTCAGGAGAAAGTGATGGAGTC - 3' (SEQ ID NO: 45)
For variable region:
   VH1/5BACK: 5' - CAGGTGCAGCTGCAGCAGTCTGG - 3' (SEQ ID NO: 46)
   VH4BACK: 5' - CAGGTGCAGCTGCAGGAGTCGGG - 3' (SEQ ID NO: 47)
   VH3BACK: 5' - GAGGTGCAGCTGCAGGAGTCTGG - 3' (SEQ ID NO: 48)

PCR was carried out using sets of the primer for the constant region × primer for the variable region (3 types) under conditions of 35 cycles with 98°C for 10 seconds, 59°C for 5 seconds, and 68°C for 5 seconds. All amplification products were detected by 1.5% agarose gel electrophoresis followed by ethidium bromide staining. As a result, amplification products were detected at or around expected 470 bp in all the combinations. On the other hand, no specific amplification product was detected at the same position in all the combinations for the negative control. Each of these amplification products was extracted from agarose gel and then treated with restriction enzymes HindIII and PstI. The resultant was cloned into a HindIII-PstI site of a pUC119 (Takara Bio Inc.) vector. Among plasmids having the inserted amplification product, VH1 family-derived clones, VH4 family-derived clones, and VH3 family-derived clones were each sequenced to determine the base sequences of the amplification products.

### 2. Analysis of base sequence of heavy chain variable region of human antibody gene from cDNA derived from spleen of human minimalized (ΔIgH/ΔIgκ-ES) chimeric mouse

A known germline V gene (referred to as a "V-fragment"), D gene (referred to as a "D-fragment"), and J gene (referred to as a "J-fragment") used in each clone were identified as to the base sequences determined in the above 1. Table 10 shows results about 27 clones whose D-fragment of origin was able to be identified. 15 clones were subjected to the same analysis using spleen-derived cDNA obtained from a human antibody-producing mouse retaining wild-type Ig-NAC (JP Patent No. 6868250). The results are shown in Table 11.

As a result, regarding the use of the D-fragment in chimeric mouse spleen Ig µ-cDNA, 13 D-fragments were used in the 27 human minimalized clones, and, by contrast, 6 D-fragments were used in the 15 wild-type human antibody-producing mouse clones, among which the bias that the D-fragment 3-10 was used in 7 clones was observed (Figure 13).

**[Table 10]**

| | V | D | J |
|---|---|---|---|
| 1 | V1-8 | D3-22 | J4 |
| 2 | V1-8 | D3-3 | J6 |
| 3 | V1-8 | D4-23 | J4 |
| 4 | V1-8 | D3-17 | J6 |
| 5 | V1-8 | D6-6 | J6 |
| 6 | V1-3 | D6-6 | J6 |
| 7 | V1-8 | D4-4 or D4-11 | J4 |
| 8 | V1-8 | D4-23 | J4 |
| 9 | V1-8 | D4-17 | J4 |
| 10 | V1-8 | D7-27 | J4 |
| 11 | V1-8 | D5-12 | J4 |
| 12 | V1-8 | D3-22 | J4 |
| 13 | V1-8 | D3-3 | J6 |
| 14 | V4-4 | D7-27 | J3 |
| 15 | 4-39 | D2-21 | J6 |
| 16 | 4-39 | D2-21 | J6 |
| 17 | 4-39 | D1-1 | J3 |
| 18 | V4-34 | D3-10 | J5 |
| 19 | V4-39 | D7-27 | J6 |
| 20 | V3-33 | D4-17 | J4 |
| 21 | V3-33 | D6-19 | J4 |
| 22 | V3-23 | D7-27 | J4 |
| 23 | V3-48 | D6-13 | J4 |
| 24 | V3-9 | D6-6 | J2 |
| 25 | V3-33 | D6-6 | J2 |
| 26 | V3-33 | D4-23 | J6 |
| 27 | V3-21 | D1-1 | J4 |

**[Table 11]**

| | V | D | J |
|---|---|---|---|
| 1 | V1-8 | D3-9 | J6 |
| 2 | V1-8 | D5-12 | J4 |
| 3 | V1-3 | D3-10 | J6 |
| 4 | V1-8 | D2-2 | J6 |
| 5 | V1-8 | D5-12 | J6 |
| 6 | V1-8 | D3-10 | J5 |
| 7 | V3-33 | D3-9 | J4 |
| 8 | V3-33 | D6-19 | J4 |
| 9 | V3-11 | D3-10 | J6 |
| 10 | V3-7 | D1-26 | J6 |
| 11 | V3-48 | D3-10 | J4 |
| 12 | V3-33 | D3-10 | J4 |
| 13 | V3-48 | D3-9 | J4 |
| 14 | V3-48 | D3-10 | J4 |
| 15 | V4-59 | D3-10 | J4 |

### [Example 16] Repertoire analysis of bovine minimalized (ΔIgH/ΔIgκ-ES) chimeric mouse

A heavy chain variable region of a human antibody gene was amplified by PCR with the method described in Example 15 using cDNA derived from the spleen of each bovine minimalized (ΔIgH/ΔIgκ-ES) chimeric mouse. Spleen-derived cDNA obtained from a nonchimeric mouse ICR was used as a negative control. As a result, use of a bovine D-fragment in chimeric mouse spleen Ig µ-cDNA is shown.

In order to confirm use of a bovine D-fragment, bovine D-fragment-specific primers shown below were designed and used. Spleen-derived cDNA obtained from a nonchimeric mouse ICR was used as a negative control.
cowF1: 5'-ATTATCTGCAGAAGTCTGACCCGCACACAGG - 3' (SEQ ID NO: 49)
cowF2: 5'-ATTATCTGCAGTGTGGAGCTGGCCAATGCAT - 3' (SEQ ID NO: 50)
cowF3: 5'-ATTATCTGCAGATGGTTATGGTTATGGTTATGG - 3' (SEQ ID NO: 51)
cowR1: 5'-GTCGGAAGCTTATAACCACAACCATAACCAT - 3' (SEQ ID NO: 52)

PCR was carried out under conditions of 35 cycles with 98°C for 10 seconds, 59°C for 5 seconds, and 68°C for 5 seconds using sets of each forward primer (cowF1, cowF2, and cowF3) produced within the fragment and HIGMEX1-2 (primer for the constant region) used in Example 15, and sets of the reverse primer (cowR1) produced within the fragment and each primer for the variable region (VH1/5BACK, VH4BACK, and VH3BACK) used in Example 15. All amplification products were detected by 1.5% agarose gel electrophoresis followed by ethidium bromide staining. As a result, amplification products were detected with expected lengths (the forward primer produced within the fragment and HIGMEX1-2: at or around 250 bp; the reverse primer produced within the fragment and the primer for the variable region used in Example 15: at or around 400 bp). On the other hand, no specific amplification product was detected at the same position in all the combinations for the negative control. Each of these amplification products was extracted from agarose gel and then treated with restriction enzymes HindIII and PstI. The resultant was cloned into a HindIII-PstI site of a pUC119 (Takara Bio Inc.) vector. Plasmids having the inserted amplification product were each sequenced to determine the base sequences.

A known germline D-fragment and V-fragment or J-fragment used in each clone were identified as to the base sequences determined as described above. The base sequences of four clones whose bovine D-fragment of origin was able to be identified are shown below.

Clone 1
   D-fragment (bovine D1-3):
      TGTGGAGCTGGCCA (SEQ ID NO: 53)
   J-fragment (human J3):
      ATGCTTTTGATATCTGGGGCCAAGGGACAATGGTCACCGTCTCTTCAG (SEQ ID NO: 54)
Clone 2
   D-fragment (bovine 6-3 or 6-4):
      ATGGTTATGGTTATGGTTATGGTTGTGGTTATGGTTATGGTTATAC (SEQ ID NO: 55)
   J-fragment (human 11):
      CTTCCAGCACTGGGGCCAGGGCACCCTGGTCACCGTCTCCTCAG (SEQ ID NO: 56)
Clone 3
   V-fragment (human V3-23): 
GGGGGAGGCTTGGTACAGCCTGGGGGGTCCCTGAGACTCTCCTGTGCAGCCTCTG
GATTCACCTTTAGCAGCTATGCCATGAGCTGGGTCCGCCAGGCTCCAGGGAAGGG
GCTGGAGTGGGTCTCAGCTATTAGTGGTAGTGGTGGTAGCACATACTACGCAGAC
TCCGTGAAGGGCCGGTTCACCATCTCCAGAGACAATTCCAAGAACACGCTGTATC
TGCAAATGAACAGCCTGAGAGCCGAGGACACGGCCGTATATTACTGTGCGAAAG
A (SEQ ID NO: 57)
   D-fragment (bovine 6-3 or 6-4):
      TAGTTGTTATAGTGGTTATGGTTATGGTTATGGTTGTGGTTAT (SEQ ID NO: 58)
Clone 4
   V-fragment (human V2-5): 
AGGAGTCTGGTCCTACGCTGGTGAAACCCACACAGACCCTCACGCTGACCTGCAC
CTTCTCTGGGTTCTCACTCAGCACTAGTGGAGTGGGTGTGGGCTGGATCCGTCAGC
CCCCAGGAAAGGCCCTGGAGTGGCTTGCATTCATTTATTGGGATGATGATAAGCG
CTACAGCCCATCTCTGAAGAGCAGGCTCACCATCACCAAGGACACCTCCAAAAAC
CAGGTGGTCCTTACAATGACCAACATGGACCCTGTGGACACAGCCACATATTACT
GTGCACACAG (SEQ ID NO: 59)
   D-fragment (bovine 6-3 or 6-4):
      TTGTTATAGTGGTTATGGTTATGGTTATGGTTGTGGTTAT (SEQ ID NO: 60)

As a result of these procedures, cDNA in which a bovine D-fragment was linked to a human V-fragment or J-fragment was detected in the chimeric mouse spleen. For example, the amino acid sequence derived from the cDNA sequence of the clone 2 is the following sequence:
GYGYGYGCGYGYGYTFQHWGQGTLVTVSS (SEQ ID NO: 61)
CDRH3 was confirmed to be long CDR3 having a length of 18 or more amino acids (the number of amino acids underlined).

### [Example 17] Immune response of human minimalized (ΔIgH/ΔIgκ-ES) chimeric mouse

Whether an immune response and an antigen-specific antibody are induced can be evaluated by administering an antigen protein to a human minimalized chimeric mouse, and performing enzyme-linked immunosorbent assay (hereinafter, referred to as ELISA) using a serum or plasma sample collected after administration.

### 1. Administration of antigen

A 1 mg/mg antigen protein solution is prepared with PBS containing 0.4 M arginine and mixed at 1:1 with Freund's Complete adjuvant (Sigma F5881) for initial immunization and with Sigma Adjuvant System^{™} (SAS^{™}) (Sigma S6322) for boosters to provide antigen dosing solutions. The antigen dosing solutions are administered to a 6-week-old chimeric mouse at doses of 200 µL (100 µg of the antigen, intraperitoneal injection) for initial immunization, 100 µL (50 µg of the antigen, intraperitoneal injection, every two weeks) for boosters, and 100 µL (50 µg of the antigen, 1 mg/mg antigen protein solution, intravenous injection) for final immunization.

### 2. Antiserum ELISA

Plasma is prepared from peripheral blood 3 days after each immunization shot. In order to evaluate the titer of antigen-specific IgG in the plasma, antiserum ELISA is carried out using an antigen-immobilized 96-well plate and an anti-human IgG Fc antibody.

### [Example 18] Immune response of bovine minimalized (ΔIgH/ΔIgκ-ES) chimeric mouse

Whether an immune response and an antigen-specific antibody were induced was evaluated by administering a receptor binding domain (hereinafter, referred to as RBD) of SARS-CoV2 spike protein S1 as an antigen to a bovine minimalized chimeric mouse, and performing ELISA using a serum or plasma sample collected after administration.

### 1. Administration of antigen

A 1 mg/mg antigen protein solution is prepared with PBS containing 0.4 M arginine and mixed at 1:1 with Freund's Complete adjuvant (Sigma F5881) for initial immunization and with Sigma Adjuvant System^{™} (SAS^{™}) (Sigma S6322) for boosters to provide antigen dosing solutions. The antigen dosing solutions were administered to a 6-week-old chimeric mouse at doses of 200 µL (100 µg of the antigen, intraperitoneal injection) for initial immunization and 100 µL (50 µg of the antigen, intraperitoneal injection, every two weeks, five times) for boosters.

### 2. Antiserum ELISA

Plasma was prepared from peripheral blood 3 days after each immunization shot. In order to evaluate the titer of antigen-specific IgG in the plasma, antiserum ELISA was carried out using an antigen-immobilized 96-well plate and an anti-human IgG Fc antibody. It was consequently confirmed that continuous boosters caused an immune response and elevated an antibody titer against RBD (Figure 14).

### [Example 19] Insertion of chemically synthesized DNA comprising monkeynized IgHD (Synplogen Co., Ltd.) by site-directed recombination using ΦC31 recombinase

Monkeynized human IgHD of approximately 40 kbp that maintained the gene sequence of the whole human antibody D-region except for D-fragments was designed by substituting the human D-fragments with D-fragments of a cynomolgus macaque which is evolutionarily related to humans and whose antibody gene sequence has been elucidated (the base sequence of each D-fragment is described in http://www.imgt.org/) (Figure 15).

### 1. Production of chemically synthesized DNA comprising monkeynized IgHD

The number of human D-fragments to be substituted is 26, whereas the number of cynomolgus macaque D-fragments is 40. Therefore, mutually similar sequences between the cynomolgus macaque D-fragments were excluded, and largely changed fragments compared to human IgHD were prioritized to narrow the cynomolgus macaque D-fragments to 26 fragments shown in Table 12 below. Mutations in VDJ recombination sequences present upstream and downstream of four human D-fragments (D4-11, D1-14, D4-23, and D5-24) were converted to normal type.

**[Table 12]**

| Human D-fragment before substitution | Cynomolgus macaque D-fragment after substitution |
|---|---|
| D1-1 | 1S5 |
| D2-2 | 2S11 |
| D3-3 | 3S6 |
| D4-4 | 4S24 |
| D5-5 | 5S8 |
| D6-6 | 6S4 |
| D1-7 | 1S10 |
| D2-8 | 2S17 |
| D3-9 | 3S18 |
| D3-10 | 2S34 |
| D4-11 | 5S14 |
| D5-12 | 5S31 |
| D6-13 | 6S3 |
| D1-14 | 1S27 |
| D2-15 | 2S22 |
| D3-16 | 4S36 |
| D4-17 | 4S30 |
| D5-18 | 5S37 |
| D6-19 | 6S20 |
| D1-20 | 1S33 |
| D2-21 | 2S28 |
| D3-22 | 6S32 |
| D4-23 | 3S12 |
| D5-24 | 6S38 |
| D6-25 | 6S26 |
| D1-26 | 1S39 |

A vector having, in addition to monkeynized IgHD carrying these 26 cynomolgus macaque D-fragments, chemically synthesized DNA recombination sites ΦC31 attP and R4 attP, and blasticidin resistance gene for confirming the presence of vector introduction was chemically synthesized by OGAB (Ordered Gene Assembly in Bacillus subtilis) using *Bacillus subtilis* from Synplogen Co., Ltd.

### 2. Introduction of monkeynized IgHD vector by ΦC31 recombinase

The same method as in Example 5 was carried out. The 1-10 cells were treated with trypsin and suspended at 3 × 10⁶ cells/mL in Opti MEM, followed by the addition of 1 µg of a ΦC31 recombinase gene expression vector ΦC31pEF1 (JP Patent No. 6868250) and 3 µg of the monkeynized IgHD vector and electroporation at 150 V and 7.5 ms using NEPA21 (Nepa Gene Co., Ltd.). The cells thus pulsed were inoculated to two 384-well plates. For the cells, medium was replaced with F12 medium containing 800 µg/mL G418, 800 µg/mL blasticidin, and 10% FBS at 48 hours later and cultured. 17 clones having blasticidin resistance were picked up 12 days later.

### 3. PCR analysis

The isolated cells were cultured, and genomic DNA was extracted from the cells using Cellysis (Qiagen N.V.). PCR analysis was conducted with the genomic DNA as a template using one ΦC31 site-directed recombination-specific primer as in Example 5 and 12 Ig-NAC-specific primers produced for Ig-NAC, and one monkeynized IgHD-specific primer shown below. Approximately 0.1 µg of the genomic DNA was used in PCR amplification. The Taq polymerase used was KODone (Toyobo Co., Ltd.), and PCR was performed by 1 cycle of 98°C for 2 minutes followed by 35 cycles with denaturation at 98°C for 10 seconds, annealing at 60°C for 5 seconds, and extension at 68°C for: 5 seconds in the case of an amplification fragment having a length of 500 bp or less; and 100 seconds/kb for a length of 500 bp or more.
3-10monkeyF: 5'-CACCCACAGTGTCACAGAGT- 3' (SEQ ID NO: 62)
4-11 monkeyr: 5'-TCACTGTGGGTGGCAAAACT- 3' (SEQ ID NO: 63)

In order to further confirm whether the whole monkeynized IgHD was retained in a clone, five primers shown below were produced and used to perform long PCR. Approximately 0.1 µg of the genomic DNA was used in PCR amplification. The Taq polymerase used was PrimeSTAR^{™} GXI, Premix Fast (Takara Bio Inc.), and PCR was carried out as follows by the stepdown method.

1 cycle of 98°C for 2 minutes -> 5 cycles with denaturation at 98°C for 10 seconds, and annealing and extension at 72°C for: 5 seconds/length (kb) in the case of an amplification fragment having a length of 10 kbp or less; and 10 seconds/kb for a length of more than 10 kb -> 5 cycles with denaturation at 98°C for 10 seconds, and annealing and extension at 70°C for: 5 seconds/length (kb) in the case of an amplification fragment having a length of 10 kbp or less; and 10 seconds/kb for a length of more than 10 kb -> 30 cycles with denaturation at 98°C for 10 seconds, and annealing and extension at 68°C for: 5 seconds/length (kb) in the case of an amplification fragment having a length of 10 kbp or less; and 10 seconds/kb for a length of more than 10 kb.
PACF1: 5'- CGGTGTGCGGTTGTATGCCTGCTGT - 3' (SEQ ID NO: 64)
3-3to4-4R: 5'- GGCCAGGCAGGATGATGGACTCCCA - 3' (SEQ ID NO: 65)
3-3F: 5'- CGGTTACTATTACACCCACAGCGTC - 3' (SEQ ID NO: 66)
3-10F2: 5'- TTGTAGTGGTGGTGTCTGCTACACC - 3' (SEQ ID NO: 67)
3-16R: 5'- GTAGTTACTGTATTCACACAGTGAC - 3' (SEQ ID NO: 68)
F37: 5'- CTGCAGGCCCTGTCCTCTTC - 3' (SEQ ID NO: 69)
4-23R: 5'- ATAGTAATACCACTGTGGGAGGGCC - 3' (SEQ ID NO: 70)

### 4. FISH analysis

One cell clone confirmed to exhibit the band of interest by PCR analysis was subjected to FISH analysis. The FISH analysis was conducted in accordance with the method described in (JP Patent No. 6868250) using a mouse gene-specific probe, mouse cot1 (Invitrogen).

Table X shows results of the PCR analysis and the FISH analysis for the one cell clone subjected to the FISH analysis.

Table 13 shows a clone name in the row and the primers and the extension times used for PCR in the columns. The circle represents positive. In the table, Ig-NACx1 represents that one copy of Ig-NAC was detected per cell.

**[Table 13]**

| Primer name | Extension time | 1-10 m9 |
|---|---|---|
| phiC31 F1/BsdR1 | 5 sec | ○ |
| 3-10monkeyF/4-11monkeyR | 9 sec | ○ |
| PACF/3-3to4-4 | 40 sec | ○ |
| 3-3F/4-11monkeyR | 100 sec | ○ |
| 3-10F2/3-16R | 45 sec | ○ |
| F37/4-23R | 140 sec | ○ |
| 3-10monkeyF/HRBR1 | 40 sec | ○ |
| EIF2AK3-F/R | 5 sec | ○ |
| RPIA-F/R | 5 sec | ○ |
| IGKC-F/R | 5 sec | ○ |
| IGKV-F/R | 5 sec | ○ |
| Vk3-2 F/R | 5 sec | ○ |
| ELK2 P2-F/R | 5 sec | ○ |
| VH3-F/R | 5 sec | ○ |
| MTA1-F3/R3 | 5 sec | ○ |
| g1(g2)-F/R | 5 sec | ○ |
| CH3F3/CH4R2 | 5 sec | ○ |
| TRANS L1/R1 | 5 sec | ○ |
| KJneo/PGKr-2 | 5 sec | ○ |
| FISH analysis | | retention rate of Ig-NACx1: 94% |

### [Example 20] Removal of partial sequence by site-directed recombination using R4 recombinase (monkeynization)

### 1. Introduction of R4 recombinase gene expression vector

The same method as in Example 6 was carried out. The cell clone confirmed to be introduced with the monkeynized human IgHD vector (Example 19) was treated with trypsin and suspended at 3 × 10⁶ cells/mL in Opti MEM, followed by the addition of 10 µg of the R4 recombinase gene expression vector and electroporation at 150 V and 7.5 ms using NEPA21 (Nepa Gene Co., Ltd.). The cells thus pulsed were inoculated to one 10 cm dish. The cells were treated with trypsin 24 to 48 hours later and inoculated at 150 cells per 384-well plate. For the cells, medium was replaced with F12 medium containing 800 µg/mL G418 and 10% FBS 17 days after inoculation to a 384-well plate and cultured, and single clones were picked up.

### 2. PCR analysis

The isolated cells were cultured, and genomic DNA was extracted from the cells using Cellysis (Qiagen N.V.). PCR analysis was conducted with the genomic DNA as a template using one R4 site-directed recombination-specific primer in accordance with Example 6, two primers produced within monkeynized IgHD, and 12 Ig-NAC-specific primers produced for Ig-NAC. A clone (1-10 M9-I9) was selected in which a R4 site-directed recombination-specific band was obtained by PCR using the R4 site-directed recombination-specific primer.

### 3. FISH analysis

The clone (1-10 M9-I9) confirmed to exhibit the band of interest by PCR analysis was subjected to FISH analysis. The FISH analysis was conducted according to the method described in (JP Patent No. 6868250) using a mouse gene-specific probe, mouse cot1 (Invitrogen).

### [Example 21] Introduction of monkeynized IgHD-carrying Ig-NAC to mouse ES cell line by micronuclei formation (MMCT)

### 1. Transfer of monkeynized IgHD-carrying Ig-NAC to mouse ES cell by MMCT

The chromosome donor cells used are the clone confirmed to undergo R4 site-directed recombination by PCR and FISH analysis in Example 10. The chromosome receptor cells used are a mouse ES cell HKD31 6TG-9 strain (XO) (JP Patent No. 4082740) in which both alleles of endogenous immunoglobulin heavy chain and κ chain loci are disrupted. Micronuclei are formed by the same method as in Example 7 and fused with the mouse ES cell HKD31 6TG-9 strain (XO), and the cells are inoculated to three 10 cm dishes. The medium is replaced with a medium for an ES cell supplemented with 350 µg/mL G418 from 48 hours later, followed by medium replacement according to the same composition once every two days. Drug-resistant colonies that appear are picked up 1 week to 10 days later.

### 2. PCR analysis

The isolated cells are cultured, and genomic DNA is extracted from the cells using Cellysis (Qiagen N.V.). PCR is carried out with the genomic DNA as a template using the same primers as in the PCR analysis conducted in Example 6.

### 3. Karyotypic analysis

The absence of karyotypic abnormality in mouse chromosome is confirmed by the same method as in Example 7. Among the karyotypically analyzed clones, clones that have 39 mouse chromosomes and a high retention rate of Ig-NACx1 without translocation are selected.

### [Example 22] Production of chimeric mouse from mouse ES cell line that retains monkeynized IgHD-containing Ig-NAC

In order to confirm human antibody production, chimeric mice are produced from mouse ES cells that retain monkeynized IgHD-containing Ig-NAC. Chimeric mice are produced according to the approach described in Gene Targeting, Experimental Medicine, 1995 using the obtained mouse ES cells that retain monkeynized IgHD-containing Ig-NAC. The host cells used are the morula and the 8-cell-stage embryo obtained by sexual crossbreeding of MCH (ICR) (white, purchased from CLEA Japan, Inc.) or HKLD mice having deficiency (Igh/Igκ KO) or low expression (Igl1 low) of an antibody gene. Whether the newborn mice obtained though transplantation of the injected embryo into a surrogate mother are chimeric mice, can be determined based on the coat color. The transplantation of the embryos into surrogate mothers produces chimeric mice (a dark brown color area is observed in coat color).

### [Example 23] Analysis of monkeynized IgHD chimeric mouse (B cell analysis, ELISA, and cDNA preparation)

B cell differentiation analysis, ELISA analysis for measurement of an antibody concentration in plasma, and gene sequence analysis by cDNA collection from the spleen can be conducted to evaluate whether a monkeynized IgHD human antibody is produced from a monkeynized IgHD chimeric mouse.

### 1. B cell differentiation analysis

If cells copositive to EGFP and a B cell marker B220 can be detected, this indicates B cell differentiation based on contribution to antibody production and serves as indirect evaluation of functional expression of an antibody. Peripheral blood obtained from each chimeric mouse is used as a sample to analyze B220 by flow cytometry in accordance with the method described in JP Patent No. 6868250. As a result, not only a GFP-positive cell population but a GFP- and B220-copositive population is confirmed, indicating that a functional antibody is expressed.

### 2. Antibody expression analysis by ELISA

The measurement of the plasma concentrations of a mouse antibody (mγ, mµ, mκ, and mλ) and a human antibody (hγ, hµ, hκ, hλ, hγ1, hγ2, hγ3, hγ4, hα, hε, and hδ), as well as the confirmation of the presence of antibody expression in mice, are performed by the method described in JP Patent No. 6868250 using plasma obtained from each chimeric mouse.

### 3. Expression analysis and sequence identification of human antibody

cDNA is synthesized from RNA derived from the spleen of each human antibody-producing mouse, and variable regions of a human antibody gene are cloned and sequenced for base sequence. Analysis and evaluation were conducted in accordance with JP Patent No. 6868250. The spleen was collected, then preserved overnight at 4°C in RNAlater (Ambion, Inc.), then taken out of the solution, and stored at -80°C. 1 mL of ISOGEN (Nippon Gene Co., Ltd.) is added to a pressure-resistant tube containing zirconia beads, and 50 mg of tissues is added thereto. The tissues are homogenized in an apparatus (approximately 30 to 45 seconds). After confirmation that the tissues are homogenized, the homogenates are spun down. Then, 1 mL of a supernatant is separated, and 200 µL of chloroform is added thereto, followed by RNA extraction using RNeasy Mini kit in accordance with manufacturer's protocol.

### [Example 24] Repertoire analysis of monkeynized (ΔIgH/ΔIgκ-ES) chimeric mouse

### 1. Cloning and sequencing for base sequence of heavy chain region of human antibody gene from cDNA derived from spleen of monkeynized (ΔIgH/ΔIgκ-ES) chimeric mouse

A heavy chain variable region of a human antibody gene is amplified by PCR using primers given below as to cDNA derived from the spleen of each chimeric mouse retaining monkeynized IgHD. Spleen-derived cDNA obtained from a nonchimeric mouse ICR is used as a negative control.
For constant region:
   HIGMEX1-2: 5'-CCAAGCTTCAGGAGAAAGTGATGGAGTC-3' (SEQ ID NO: 71)
For variable region:
   VH1/5BACK: 5'-CAGGTGCAGCTGCAGCAGTCTGG-3' (SEQ ID NO: 72)
   VH4BACK: 5'-CAGGTGCAGCTGCAGGAGTCGGG-3' (SEQ ID NO: 73)
   VH3BACK: 5'-GAGGTGCAGCTGCAGGAGTCTGG-3' (SEQ ID NO: 74)

PCR is carried out using sets of the primer for the constant region × primer for the variable region (3 types) under conditions of 35 cycles with 98°C for 10 seconds, 59°C for 5 seconds, and 68°C for 5 seconds. All amplification products are detected by 1.5% agarose gel electrophoresis followed by ethidium bromide staining. Each of the amplification products is extracted from agarose gel and then treated with restriction enzymes HindIII and PstI. The resultant is cloned into a HindIII-PstI site of a pUC119 (Takara Bio Inc.) vector. Plasmids having the inserted amplification product are sequenced to determine the base sequences of the amplification products.

### 2. Analysis of base sequence of heavy chain variable region of human antibody gene from cDNA derived from spleen of monkeynized (ΔIgH/ΔIgκ-ES) chimeric mouse

A known germline V gene fragment, D-fragment, and J-fragment used in each clone are identified as to the base sequences determined in the above 1. As a result, the presence of heavy chain variable region cDNA of a human antibody gene comprising a monkey DH sequence is confirmed by VDJ recombination.

### [Example 25] Immune response of monkeynized (ΔIgH/ΔIgκ-ES) chimeric mouse

Whether an immune response and an antigen-specific antibody are induced can be evaluated by administering an antigen protein to a monkeynized (ΔIgH/ΔIgκ-ES) chimeric mouse, and performing enzyme-linked immunosorbent assay (hereinafter, referred to as ELISA) using a serum or plasma sample.

### 1. Administration of antigen

A 1 mg/mg antigen protein solution is prepared with PBS containing 0.4 M arginine and mixed at 1:1 with Freund's Complete adjuvant (Sigma F5881) for initial immunization and with Sigma Adjuvant System^{™} (SAS^{™}) (Sigma S6322) for boosters to provide antigen dosing solutions. The antigen dosing solutions are administered to a 6-week-old chimeric mouse at doses of 200 µL (100 µg of the antigen, intraperitoneal injection) for initial immunization, 100 µL (50 µg of the antigen, intraperitoneal injection, every two weeks) for boosters, and 100 µL (50 µg of the antigen, 1 mg/mg antigen protein solution, intravenous injection) for final immunization.

### 2. Antiserum ELISA

Plasma is prepared from peripheral blood 3 days after each immunization shot. In order to evaluate the titer of antigen-specific IgG in the plasma, antiserum ELISA is carried out using an antigen-immobilized 96-well plate and an anti-human IgG Fc antibody.

### [Example 26] Establishment of mouse lineage that retains human minimalized IgHD-carrying Ig-NAC while endogenous immunoglobulin heavy chain and κ chain loci are disrupted

A chimeric mouse was produced as shown in Example 8 using mouse ES cells that retained human minimalized IgHD-carrying Ig-NAC, which were produced with mouse ES cells TT2F (XO) as receptor cells in Example 7, and this chimeric mouse was subjected to crossbreeding to a mouse lineage in which an endogenous immunoglobulin heavy chain and κ chain were disrupted, in order to establish a mouse lineage that retained human minimalized IgHD-carrying Ig-NAC while endogenous immunoglobulin heavy chain and κ chain loci were disrupted. The obtained mouse individual (#200) that retained human minimalized IgHD-carrying Ig-NAC while endogenous immunoglobulin heavy chain and κ chain loci were disrupted was analyzed by flow cytometry with the method described in Example 13. As a result, peripheral blood mononuclear cells had a GFP-positive rate of 96.97% and had a GFP- and B220-copositive rate of 18.78%. This value was equivalent to a mouse individual that retained wild-type Ig-NAC instead of the human minimalized IgHD while endogenous immunoglobulin heavy chain and κ chain loci were disrupted (JP Patent No. 6868250).

### [Example 27] Establishment of mouse lineage that retains bovine minimalized IgHD-carrying Ig-NAC while endogenous immunoglobulin heavy chain and κ chain loci are disrupted

A chimeric mouse was produced as shown in Example 12 using mouse ES cells that retained bovine minimalized IgHD-carrying Ig-NAC, which were produced with mouse ES cells TT2F (XO) as receptor cells in Example 11, and this chimeric mouse was subjected to crossbreeding to a wild-type mouse or a mouse lineage in which an endogenous immunoglobulin heavy chain and κ chain were disrupted, in order to establish a mouse lineage that retained bovine minimalized IgHD-carrying Ig-NAC while endogenous immunoglobulin heavy chain and κ chain loci were disrupted. The obtained mouse individual (#241) that retained bovine minimalized IgHD-carrying Ig-NAC while endogenous immunoglobulin heavy chain and κ chain loci were disrupted was analyzed by flow cytometry with the method described in Example 13. As a result, peripheral blood mononuclear cells had a GFP-positive rate of 99.09% and had a GFP- and B220-copositive rate of 6.26%. This value was equivalent to a mouse individual that retained wild-type Ig-NAC instead of the bovine minimalized IgHD while endogenous immunoglobulin heavy chain and κ chain loci were disrupted (JP Patent No. 6868250).

### [Example 28] Establishment of mouse lineage that retains monkeynized IgHD-carrying Ig-NAC while endogenous immunoglobulin heavy chain and κ chain loci are disrupted

A chimeric mouse produced in Example 22 or a chimeric mouse produced as shown in Example 22 with mouse ES cells TT2F (XO) as receptor cells in Example 21, can be subjected to crossbreeding to a wild-type mouse or a mouse lineage in which an endogenous immunoglobulin heavy chain and κ chain are disrupted, to establish a mouse lineage that retains monkeynized IgHD-carrying Ig-NAC while endogenous immunoglobulin heavy chain and κ chain loci are disrupted.

### [Example 29] Insertion of chemically synthesized DNA comprising human long minimalized IgHD by site-directed recombination using ΦC31 recombinase

A human long minimalized IgHD vector was produced in accordance with the procedures 1. and 2. as in Example 5 by substituting D-fragments 1-1 to 1-26 of the human minimalized IgHD designed in Example 5 with 26 modified long DH sequences shown in Figure 16. The human long DH sequences were designed on the basis of a long (18 or more amino acids) CDR3 sequence found in the cDNA sequence of a human antibody registered in NCBI. Each vector used for human long miniA, human long miniB, and human long miniC was synthesized at Eurofins Genomics K.K. in accordance with Example 5.

### 1. Insertion of human long miniA fragment into human long miniB

The human long miniA was cleaved with restriction enzymes XhoI and NotI. The long miniA was inserted into the human long miniB cleaved with the same restriction enzymes XhoI and NotI to construct a human long miniA/B vector.

### 2. Insertion of human long miniC fragment into human long miniA/B vector

The human long miniC was cleaved with restriction enzymes EcoRI and Sail. The human long miniC fragment was inserted into the human long miniA/B vector cleaved with the same restriction enzymes EcoRI and SalI to construct a human long minimalized IgHD vector.

### 3. Introduction of human long minimalized IgHD vector by ΦC31 recombinase

A ΦC31 recombinase gene expression vector (JP Patent No. 6868250) and the human long minimalized IgHD vector were introduced by electroporation to the chemically synthesized DNA introduction platform-carrying and Ig-NAC-retaining CHO cells 1-10 (Example 4) with the same method as in Example 5. The 1-10 cells were treated with trypsin and suspended at 3 × 10⁶ cells/mL in Opti MEM, followed by the addition of 1 µg of a ΦC31 recombinase gene expression vector ΦC31pEF1 (JP Patent No. 6868250) and 3 µg of the human long minimalized IgHD vector and electroporation at 150 V and 7.5 ms using NEPA21 (Nepa Gene Co., Ltd.). The cells thus pulsed were inoculated to two 384-well plates. For the cells, medium was replaced with F12 medium containing 800 µg/mL G418, 800 µg/mL blasticidin, and 10% FBS at 48 to 72 hours later and cultured. Clones having blasticidin resistance were picked up 11 days later.

### 4. PCR analysis

The clones obtained in 3. were analyzed by PCR using one ΦC31 site-directed recombination-specific primer, two primers produced within human long minimalized IgHD, and 12 Ig-NAC-specific primers produced for Ig-NAC, by the same method as in Example 5. Approximately 0.1 µg of genomic DNA was used in PCR amplification. The Taq polymerase used was KODone (Toyobo Co., Ltd.), and PCR was performed by 1 cycle of 98°C for 2 minutes followed by 35 cycles with denaturation at 98°C for 10 seconds, annealing at 60°C for 5 seconds, and extension at 68°C for: 5 seconds in the case of an amplification fragment having a length of 500 bp or less; and 100 seconds/length (bp) for a length of 500 bp or more.

### 5. FISH analysis

The clone (1-10 HL15) confirmed to exhibit the band of interest by PCR analysis was subjected to FISH analysis. The FISH analysis was conducted according to the method described in (JP Patent No. 6868250) using a mouse gene-specific probe, mouse cot1 (Invitrogen).

### [Example 30] Removal of partial sequence by site-directed recombination using R4 recombinase

Among the sequences introduced in Ig-NAC(ΔDH), the CAG promoter, the blasticidin resistance gene, and the like are sequences unnecessary for antibody gene expression. These sequences are flanked by R4 attB and R4 attP and are capable of being removed through site-directed recombination reaction between R4 attB and R4 attP by introducing R4 recombinase to cells.

### 1. Introduction of R4 recombinase gene expression vector

A R4 recombinase gene expression vector (JP Patent No. 6868250) was introduced by electroporation to the CHO cell clone (1-10 HL15) which was confirmed to have the inserted human long minimalized IgHD vector and had a high retention rate of one copy of Ig-NAC in accordance with Example 6. The resulting G418-resistant clones were picked up.

### 2. PCR analysis

PCR analysis was conducted with genomic DNA of each G418-resistant clone obtained in 1. as a template in accordance with Example 6, and clones (1-10 HL15-28 and 1-10 HL15-43) were selected in which a R4 site-directed recombination-specific band was obtained.

### 3. FISH analysis

The cell clones (1-10 HL15-28 and 1-10 HL15-43) confirmed to exhibit the band of interest by PCR analysis were subjected to FISH analysis. The FISH analysis was conducted according to the method described in (JP Patent No. 6868250) using a mouse gene-specific probe, mouse cot1 (Invitrogen).

### [Example 31] Introduction of human long minimalized IgHD-carrying Ig-NAC to mouse ES cell line by micronuclei formation (MMCT)

The clones confirmed to undergo R4 site-directed recombination by PCR and FISH analysis in Example 30 are used as chromosome donor cells to transfer human long minimalized IgHD-containing Ig-NAC to mouse ES cells by MMCT.

### 1. Transfer of human long minimalized IgHD-containing Ig-NAC to mouse ES cell by MMCT

A mouse ES cell HKD31 6TG-9 strain (XO) (JP Patent No. 4082740) in which both alleles of endogenous immunoglobulin heavy chain and κ chain loci are disrupted is used as chromosome receptor cells to introduce human long minimalized IgHD-containing Ig-NAC by micronuclei formation (MMCT) in accordance with Example 7. Drug-resistant colonies that appear are picked up.

### 2. PCR analysis

In accordance with Example 7, the isolated cells are cultured, and genomic DNA is extracted from the cells using Cellysis (Qiagen N.V.). PCR is carried out with the genomic DNA as a template, and clones are selected in which the band of interest is detected.

### 3. Karyotypic analysis

A chromosome preparation was produced in accordance with Example 7. This preparation is stained with DAPI, and clones having no karyotypic abnormality in mouse chromosome are selected.

### [Example 32] Production of chimeric mouse from mouse ES cell line that retains human long minimalized IgHD-containing Ig-NAC

In order to confirm human antibody production, chimeric mice are produced in accordance with Example 8 from the mouse ES cells that have human minimalized IgHD-containing Ig-NAC, which are obtained in Example 31.

The mouse ES cell line clone that retains human long minimalized HD-containing Ig-NAC is injected to ICR embryos, which are then transplanted to surrogate mothers. As a result, GFP-positive chimeric mice are obtained.

The obtained chimeric mice are analyzed by the methods described in, for example, Examples 13, 14, and 15. As a result, use of long human D-fragments in chimeric mouse spleen Ig µ-cDNA is shown.

### [Example 33] Construction of Ig-NAC retaining acceptor introduced with synthetic D-region from which human IgHD 7-27 has been deleted

In Ig-NAC(ΔDH) from which the human IgHD region has been deleted, a total of 26 fragments from human D-fragments 1-1 to 1-26 are deleted while 7-27 which is located distant from the D-region and near the J-region is not deleted. In order to eliminate the influence of the remaining D-fragment 7-27, gRNAs of CRISPR/Cas9 are designed, evaluated, and selected for two sites upstream and downstream of the D-fragment 7-27. Then, the chemically synthesized DNA introduction platform-carrying and Ig-NAC-retaining CHO cells 1-10 (Example 4) are subjected to cleavage at the two sites by genome editing using the gRNAs and Cas9 to obtain Ig-NAC retaining an acceptor introduced with the synthetic D-region from which human IgHD 7-27 has been deleted.

### Industrial Applicability

The present invention expands a human antibody repertoire. The present invention enables a human antibody comprising CDRH3 having preferably 18 to 50 or more amino acids to be produced.

### Free Text of Sequence Listing

SEQ ID NO: 1: nucleotide of a human minimalized D-region
SEQ ID NO: 2: nucleotide sequence of a human minimalized D-region substituted with bovine ORF
   737..767: B1-1 to replace D2-2
   1024..1039: B2-1 to replace D3-3
   1296..1331: B3-1 to replace D4-4
   1588..1630: B4-1 to replace D5-5
   1887..1900: B9-1 to replace D6-6
   2157..2187: B1-2 to replace D1-7
   2444..2459: B2-2 to replace D2-8
   2715..2756: B5-2 to replace D3-9
   2910..3057: B8-2 to replace D3-10
   3374..3431: B6-2 to replace D4-11
   3688..3806: B1-3 to replace D5-12
   4063..4078: B2-3 to replace D6-13
   4338..4373: B3-3 to replace D1-14
   4630..4696: B7-3 to replace D2-15
   4953..4994: B5-3 to replace D3-16
   5251..5308: B6-3 to replace D4-17
   5565..5595: B1-4 to replace D5-18
   5852..5867: B2-4 to replace D6-19
   6124..6159: B3-4 to replace D1-20
   6416..6488: B7-4 to replace D1-21
   6745..6785: B5-4 to replace D3-22
   7103..7160: B6-4 to replace D4-23
   7417..7430: B9-4 to replace D5-24
SEQ ID NO: 3: nucleotide sequence of a human minimalized D-region substituted with modified ORF
   647..690: modified D1-1
   947..990: modified D2-2
   1247..1290: modified D3-3
   1547..1590: modified D4-4
   1847..1890: modified D5-5
   2147..2190: modified D6-6
   2447..2508: modified D1-7
   2765..2808: modified DL2-8
   3064..3149: modified D3-9
   3303..3385: modified D3-10
   3702..3760: modified D4-11
   4017..4078: modified D5-12
   4335..4384: modified D6-13
   4644..4711: modified D1-14
   4968..5035: modified D2-15
   5292..5365: modified D3-16
   5622..5695: modified D4-17
   5952..6016: modified D5-18
   6273..6334: modified D6-19
   6591..6652: modified D1-20
   6909..6961: modified D2-21
   7218..7264: modified D3-22
   7581..7642: modified D4-23
   7899..7960: modified D5-24
   8217..8263: modified D6-25
   8520..8578: modified D1-26
SEQ ID NO: 4: target nucleotide sequence of guide RNA gRNA Up3
   21..23: PAM sequence
SEQ ID NO: 5: target nucleotide sequence of guide RNA gRNA Down4
   21..23: PAM sequence
SEQ ID NOs: 6 to 15: primers
SEQ ID NO: 18: nucleotide sequence of Bxb1 attB
SEQ ID NO: 19: nucleotide sequence of Bxb1 attP
SEQ ID NO: 20: nucleotide sequence of ΦC31 attB
SEQ ID NO: 21: nucleotide sequence of R4 attB
SEQ ID NOs: 22 and 23: primers
SEQ ID NO: 24: target nucleotide sequence of guide RNA gRNA-A1
   21..23: PAM sequence
SEQ ID NO: 25: target nucleotide sequence of guide RNA gRNA-B1
   21..23: PAM sequence
SEQ ID NOs: 26 to 52: primers
SEQ ID NO: 61: amino acid sequence of a peptide comprising long CDRH3
SEQ ID NOs: 62 to 74: primers
SEQ ID NO: 75: modified long DH sequence by which the nucleotide sequence of human DH No. 1-1 is substituted
SEQ ID NO: 76: modified long DH sequence by which the nucleotide sequence of human DH No. 2-2 is substituted
SEQ ID NO: 77: modified long DH sequence by which the nucleotide sequence of human DH No. 3-3 is substituted
SEQ ID NO: 78: modified long DH sequence by which the nucleotide sequence of human DH No. 4-4 is substituted
SEQ ID NO: 79: modified long DH sequence by which the nucleotide sequence of human DH No. 5-5 is substituted
SEQ ID NO: 80: modified long DH sequence by which the nucleotide sequence of human DH No. 6-6 is substituted
SEQ ID NO: 81: modified long DH sequence by which the nucleotide sequence of human DH No. 1-7 is substituted
SEQ ID NO: 82: modified long DH sequence by which the nucleotide sequence of human DH No. 2-8 is substituted
SEQ ID NO: 83: modified long DH sequence by which the nucleotide sequence of human DH No. 3-9 is substituted
SEQ ID NO: 84: modified long DH sequence by which the nucleotide sequence of human DH No. 3-10 is substituted
SEQ ID NO: 85: modified long DH sequence by which the nucleotide sequence of human DH No. 4-11 is substituted
SEQ ID NO: 86: modified long DH sequence by which the nucleotide sequence of human DH No. 5-12 is substituted
SEQ ID NO: 87: modified long DH sequence by which the nucleotide sequence of human DH No. 6-13 is substituted
SEQ ID NO: 88: modified long DH sequence by which the nucleotide sequence of human DH No. 1-14 is substituted
SEQ ID NO: 89: modified long DH sequence by which the nucleotide sequence of human DH No. 2-15 is substituted
SEQ ID NO: 90: modified long DH sequence by which the nucleotide sequence of human DH No. 3-16 is substituted
SEQ ID NO: 91: modified long DH sequence by which the nucleotide sequence of human DH No. 4-17 is substituted
SEQ ID NO: 92: modified long DH sequence by which the nucleotide sequence of human DH No. 5-18 is substituted
SEQ ID NO: 93: modified long DH sequence by which the nucleotide sequence of human DH No. 6-19 is substituted
SEQ ID NO: 94: modified long DH sequence by which the nucleotide sequence of human DH No. 1-20 is substituted
SEQ ID NO: 95: modified long DH sequence by which the nucleotide sequence of human DH No. 2-21 is substituted
SEQ ID NO: 96: modified long DH sequence by which the nucleotide sequence of human DH No. 3-22 is substituted
SEQ ID NO: 97: modified long DH sequence by which the nucleotide sequence of human DH No. 4-23 is substituted
SEQ ID NO: 98: modified long DH sequence by which the nucleotide sequence of human DH No. 5-24 is substituted
SEQ ID NO: 99: modified long DH sequence by which the nucleotide sequence of human DH No. 6-25 is substituted
SEQ ID NO: 100: modified long DH sequence by which the nucleotide sequence of human DH No. 1-26 is substituted

All publications, patents, and patent applications cited herein are incorporated herein by reference in their entirety.

## Claims

1. A mammalian artificial chromosome vector comprising human immunoglobulin heavy chain and light chain loci, wherein a human-derived genomic sequence from D1-1 to D1-26 of a D-region of the human immunoglobulin heavy chain locus is substituted with a modified sequence of the D-region having a combination of the following (1) and (2) or (1) and (3), wherein the modified sequence of the D-region comprises:
(1) sequences in which inter-open reading frame (ORF) human-derived genomic sequences from D1-1 to D1-26 of the D-region of the human immunoglobulin heavy chain locus or the inter-ORF human-derived genomic sequences except for the one between D3-9 and D3-10 are truncated to the length of 49 bp or more from the end of each VDJ recombination sequence of an inter-ORF region flanked by VDJ recombination sequences; and
(2) ORF sequences from B1-1 to B9-4 of a D-region of a bovine-derived immunoglobulin heavy chain locus, ORF sequences from 1S5 to 1S35 of a D-region of a monkey-derived immunoglobulin heavy chain locus, or ORF sequences of a D-region of a sheep-, horse-, rabbit-, bird-, or shark-derived immunoglobulin heavy chain locus, instead of ORF sequences from D1-1 to D1-26 of the D-region of the human immunoglobulin heavy chain locus; or
(3) 26 types of modified ORF sequences from D1-1 to D1-26 of the D-region of the human immunoglobulin heavy chain locus produced on the basis of a human antibody heavy chain CDR3 sequence of 18 or more amino acids, instead of ORF sequences from D1-1 to D1-26 of the D-region of the human immunoglobulin heavy chain locus.

2. The mammalian artificial chromosome vector according to claim 1, wherein the inter-ORF human-derived genomic sequences of the D-region of the human animal immunoglobulin heavy chain locus in (1) comprise sequences truncated to the length of 100 to 500 bp.

3. The mammalian artificial chromosome vector according to claim 1 or 2, wherein the ORF sequences from B1-1 to B9-4 of a D-region of a bovine-derived immunoglobulin heavy chain locus comprise the nucleotide sequences of SEQ ID NOs: 127 to 149 or nucleotide sequences having 90% or higher identity to the nucleotide sequences.

4. The mammalian artificial chromosome vector according to claim 1 or 2, wherein the ORF sequences from 1S5 to 1S35 of a D-region of a monkey-derived immunoglobulin heavy chain locus comprise the nucleotide sequences of SEQ ID NOs: 150 to 175 or nucleotide sequences having 90% or higher identity to the nucleotide sequences.

5. The mammalian artificial chromosome vector according to claim 1 or 2, wherein the 26 types of modified ORF sequences in (3) comprise the nucleotide sequences of SEQ ID NOs: 75 to 100 or nucleotide sequences having 90% or higher identity to the nucleotide sequences.

6. The mammalian artificial chromosome vector according to any one of claims 1 to 5, wherein the modified sequence of the D-region comprises the nucleotide sequence of SEQ ID NO: 2 or SEQ ID NO: 3 or a nucleotide sequence having 90% or higher identity to the nucleotide sequence.

7. The mammalian artificial chromosome vector according to any one of claims 1 to 6, wherein the D-region of the human immunoglobulin heavy chain locus further lacks D7-27.

8. The mammalian artificial chromosome vector according to any one of claims 1 to 7, wherein the vector is a rodent artificial chromosome vector.

9. The mammalian artificial chromosome vector according to claim 8, wherein the rodent artificial chromosome vector is a mouse artificial chromosome vector.

10. The mammalian artificial chromosome vector according to any one of claims 1 to 9, wherein the light chain locus is a κ light chain locus and/or a λ light chain locus.

11. A mammalian cell comprising a mammalian artificial chromosome vector according to any one of claims 1 to 10.

12. The mammalian cell according to claim 11, wherein the cell is a rodent cell or a human cell.

13. The mammalian cell according to claim 11, wherein the cell is a pluripotent stem cell selected from the group consisting of a mammalian-derived iPS cell and ES cell.

14. A non-human mammal comprising a mammalian artificial chromosome vector according to any one of claims 1 to 10, wherein endogenous immunoglobulin heavy chain, κ light chain, and λ light chain genes or loci are disrupted.

15. A non-human animal comprising human immunoglobulin heavy chain and light chain loci, wherein a human-derived genomic sequence from D1-1 to D1-26 of a D-region of the human immunoglobulin heavy chain locus is substituted with a modified sequence of the D-region having a combination of the following (1) and (2) or (1) and (3),
wherein the modified sequence of the D-region comprises:
(1) sequences in which inter-open reading frame (ORF) human-derived genomic sequences from D1-1 to D1-26 of the D-region of the human immunoglobulin heavy chain locus or the inter-ORF human-derived genomic sequences except for the one between D3-9 and D3-10 are truncated to the length of 49 bp or more from the end of each VDJ recombination sequence of an inter-ORF region flanked by VDJ recombination sequences; and
(2) ORF sequences from B1-1 to B9-4 of a D-region of a bovine-derived immunoglobulin heavy chain locus, ORF sequences from 1S5 to 1S35 of a D-region of a monkey-derived immunoglobulin heavy chain locus, or ORF sequences of a D-region of a sheep-, horse-, rabbit-, bird-, or shark-derived immunoglobulin heavy chain locus, instead of ORF sequences from D1-1 to D1-26 of the D-region of the human immunoglobulin heavy chain locus; or
(3) 26 types of modified ORF sequences from D1-1 to D1-26 of the D-region of the human immunoglobulin heavy chain locus produced on the basis of a human antibody heavy chain CDR3 sequence of 18 or more amino acids, instead of ORF sequences from D1-1 to D1-26 of the D-region of the human immunoglobulin heavy chain locus; and
wherein endogenous immunoglobulin heavy chain, κ light chain, and λ light chain genes or loci are disrupted.

16. The non-human animal according to claim 14 or 15, wherein the animal is a rodent.

17. The non-human animal according to claim 16, wherein the rodent is a mouse or a rat.

18. A method for producing an antibody, comprising immunizing a non-human animal according to any one of claims 14 to 17 with a target antigen, and obtaining an antibody binding to the target antigen from blood of the non-human animal.

19. A method for producing an antibody, comprising immunizing a non-human animal according to any one of claims 14 to 17 with a target antigen, obtaining splenocytes, lymph node cells, or B cells from the non-human animal producing an antibody binding to the target antigen, fusing the splenocytes, the lymph node cells, or the B cells with myeloma cells to form hybridomas, and culturing the hybridomas to obtain a monoclonal antibody binding to the target antigen.

20. A method for producing an antibody, comprising immunizing a non-human animal according to any one of claims 14 to 17 with a target antigen, obtaining B cells from the non-human animal producing an antibody binding to the target antigen, obtaining nucleic acids encoding proteins composed of an antibody heavy chain and light chain or their variable regions from the B cells, and producing an antibody binding to the target antigen by a DNA recombination technique or a phage display technique using the nucleic acid.

21. A method for substituting a D-region of a human immunoglobulin heavy chain with a modified sequence thereof in the production of a mammalian artificial chromosome vector according to any one of claims 1 to 10, comprising:
(1') providing a mammalian artificial chromosome vector comprising human immunoglobulin heavy chain and light chain loci;
(2') deleting the D-region of the human immunoglobulin heavy chain from the mammalian artificial chromosome vector of the step (1');
(3') inserting a cassette comprising a first site-directed recombinase recognition site, a promoter, and a second site-directed recombinase recognition site into a deletion site of the D-region in the vector obtained in the step (2');
(4') inserting a cassette comprising the modified sequence of the D-region of the human immunoglobulin heavy chain, the first site-directed recombinase recognition site, a selection marker, and the second site-directed recombinase recognition site into the second site-directed recombinase recognition site of the cassette inserted in the step (3') using the second site-directed recombinase; and
(5') obtaining the artificial chromosome vector in which the cassette comprising the first site-directed recombinase recognition site, the modified sequence of the D-region of the human immunoglobulin heavy chain, and the second site-directed recombinase recognition site is inserted into the deletion site of the D-region by site-directed recombination using the first site-directed recombinase.

22. The method according to claim 21, wherein the D-region of the human immunoglobulin heavy chain is deleted by genome editing.

23. A mammalian artificial chromosome vector comprising human immunoglobulin heavy chain and light chain loci, wherein a D-region is deleted from the human immunoglobulin heavy chain locus and the human immunoglobulin heavy chain locus comprises a first site-directed recombinase recognition site, a promoter, and a second site-directed recombinase recognition site instead of the deleted D-region.

24. The mammalian artificial chromosome vector according to claim 23, wherein the deleted D-region is a region from D1-1 to D1-26.

25. The mammalian artificial chromosome vector according to claim 23, wherein the deleted D-region is a region from D1-1 to D7-27.
